# EUROPÄISCHE PATENTSCHRIFT

(11) **EP 0 961 774 B1**
(45) Veröffentlichungstag und Bekanntmachung des Hinweises auf die Patenterteilung: **12.12.2001**
(21) Anmeldenummer: 96930126.6
(22) Anmeldetag: 29.08.1996
(51) Int. Cl.: C07D 409/06, A01N 43/56, C07D 411/06, C07D 417/06

(54) **PYRAZOL-4-YL-BENZOYLDERIVATE UND IHRE VERWENDUNG ALS HERBIZIDE**
PYRAZOL-4-YL-BENZOYL DERIVATIVES AND THEIR USE AS HERBICIDES
DERIVES DE PYRAZOL-4-YL-BENZOYLE ET LEUR UTILISATION COMME HERBICIDES

(30) Priorität: 01.09.1995 DE 19532312
(43) Veröffentlichungstag der Anmeldung: 08.12.1999
(73) Patentinhaber: BASF AKTIENGESELLSCHAFT, 67056 Ludwigshafen (DE)
(72) Erfinder: OTTEN, Martina, D-67069 Ludwigshafen (DE); VON DEYN, Wolfgang, D-67434 Neustadt (DE); ENGEL, Stefan, D-65510 Idstein (DE); HILL, Regina, Luise, D-67346 Speyer (DE); KARDORFF, Uwe, D-68159 Mannheim (DE); VOSSEN, Marcus, D-68199 Mannheim (DE); PLATH, Peter, D-67227 Frankenthal (DE); GÖTZ, Norbert, D-67547 Worms (DE); WALTER, Helmut, D-67283 Obrigheim (DE); WESTPHALEN, Karl-Otto, D-67346 Speyer (DE); MISSLITZ, Ulf, D-67433 Neustadt (DE)
(86) Internationale Anmeldenummer: EP9603794
(87) Internationale Veröffentlichungsnummer: WO9709327

(56) Entgegenhaltungen:
- EP-A- 0 352 543
- WO-A-93/15060
- WO-A-93/18031
- WO-A-94/01431

## Beschreibung

Die vorliegende Erfindung betrifft neue Pyrazol-4-yl-benzoyl-derivate mit herbizider Wirkung, Verfahren zur Herstellung der Pyrazol-4-yl-benzoylderivate, Mittel welche diese enthalten sowie die Verwendung dieser Derivate oder diese enthaltende Mittel zur Unkrautbekämpfung.

Aus der Literatur sind herbizidwirksame Pyrazolbenzoylderivate bekannt, beispielsweise aus EP 352543, Wo 93/15060, Wo 94/01431, WO 93/18031 und EP 712 853.

Die herbiziden Eigenschaften der bekannten Verbindungen sowie die Verträglichkeit gegenüber Kulturpflanzen können jedoch nur bedingt befriedigen.

Die Aufgabe bestand darin, neue Pyrazolbenzoylderivate mit verbesserten Eigenschaften zu finden.

Es wurden Pyrazol-4-yl-benzoylderivate der Formel I gefunden 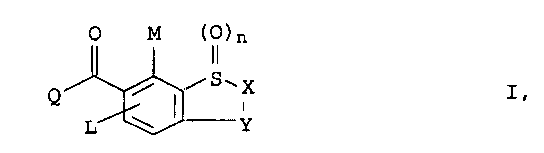 in der die Substituenten folgende Bedeutung haben:
- L,M: Wasserstoff, C₁-C₆-Alkyl, C₂-C₆-Alkenyl. C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro, eine Gruppe -(A)ₘ-S(O)ₙR¹ oder eine Gruppe -(A)ₘ-CO-R²;
- Y: eine Gruppe bestehend aus C=O, C=N-R³, CR⁷-NR⁵R⁶, CR⁷-OR⁸, CR¹⁰R¹¹, CR⁷-SR⁸; ein Heteroatom ausgewählt aus der Gruppe Sauerstoff, Schwefel und Stickstoff;
- X: eine Kette (-CR¹²R¹³-), (-CR¹²R¹³-CR²¹R²²-), (-CR¹²=CR¹³-) (-CR¹²R¹³-CR¹²=CR¹³-) ; NR²³;
die Bindung zwischen X und Y kann gesättigt oder ungesättigt sein;
- A: Sauerstoff oder NR¹⁴;
- m: null oder eins;
- n: null, eins oder zwei;
- R¹: C₁-C₄-Alkyl, C₁-C₄-Haloalkyl oder NR¹⁴;
- R²: C₁-C₄ Alkyl, C₁-C₄-Haloalkyl, C₁-C₄-Alkoxy oder NR¹⁴;
- R³: Wasserstoff, -NR⁹R⁴; C₁-C₆-Alkyl, C₁-C₆-Haloalkyl, C₁-C₆-Alkoxy, C₁-C₆-Haloalkoxy, C₂-C₆-Alkenyl, C₂-C₆-Haloalkenyl, C₂-C₆ Alkinyl;
einfach bis mehrfach substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro; ein- bis mehrfach substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
ein bis mehrfach substituiertes Benzyloxy wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R⁴: Wasserstoff, C₁-C₆-Alkyl, C₁-C₆-Haloalkyl, C₂-C₆-Alkenyl, C₂-C₆-Alkinyl, C=O-NR¹⁴;
einfach bis mehrfach substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro; ein- bis mehrfach substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R⁹: Wasserstoff, C₁-C₆-Alkyl, C₁-C₆-Haloalkyl, C₂-C₆-Alkenyl, C₂-C₆-Alkinyl, C=O-NR¹⁴;
einfach bis mehrfach substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro; ein- bis mehrfach substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R⁵, R⁶: unabhängig voneinander Wasserstoff, C₁-C₆-Alkyl, C₂-C₆-Alkenyl, C₁-C₄ Haloalkyl, C₂-C₆ Haloalkenyl, C₁-C₆-Alkoxy, C₁-C₆-Haloalkoxy;
einfach bis mehrfach substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro; ein- bis mehrfach substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R⁷: Wasserstoff, C₁-C₆-Alkyl, C₁-C₄-Alkoxy, C₁-C₄-Haloalkyl, C₁-C₄-Haloalkoxy; gegebenenfalls substitutiertes Phenyl, wobei die Substituenten bestehen können aus ein bis drei Halogenen, C₁-C₄ Alkyl, C₁-C₄-Alkoxy, C₁-C₄-Haloalkoxy, Nitro; R⁷ und R²¹ oder R⁷ und R²³ oder R⁷ und R¹² können eine Bindung bilden;
- R⁸: Wasserstoff, C₁-C₆ Alkyl,C₁-C₄-Halogenalkyl, substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro; substituiertes Benzyl wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R¹⁰, R¹¹: unabhängig voneinander Wasserstoff, C₁-C₆-Alkyl; Phenyl gegebenenfalls substituiert mit ein bis drei Halogenen, C₁-C₄-Alkyl, C₁-C₄-Alkoxy, C₁-C₄-Haloalkoxy, Nitro;
R¹⁰ und R¹² oder R¹⁰ und R²³ oder R¹⁰ und R²¹ können eine Bindung bilden;
oder R¹⁰ und R¹¹ können gemeinsam mit dem Kohlenstoff, an den sie gebunden sind, 1,3-Dioxanyl oder 1,3-Dioxolanyl, das mit Wasserstoff der C₁-C₄-Alkyl substituiert ist, bilden;
- R¹²,R¹³: unabhängig voneinander Wasserstoff, C₁-C₆-Alkyl, C₁-C₆-Haloalkyl, C₁-C₆-Alkoxy, C₁-C₆-Haloalkoxy; gegebenenfalls substituiertes Phenyl wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R¹⁴: C₁-C₄-Alkyl;
- R²¹: Wasserstoff, C₁-C₆-Alkyl, C₁-C₆-Haloalkyl, C₁-C₆-Alkoxy, C₁-C₆-Haloalkoxy; gegebenenfalls substituiertes Phenyl wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R²²: Wasserstoff, C₁-C₆-Alkyl, C₁-C₆-Haloalkyl, C₁-C₆-Alkoxy, C₁-C₆-Haloalkoxy; gegebenenfalls substituiertes Phenyl wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
- R²³: Wasserstoff, C₁-C₆-Alkyl, C₂-C₆-Alkenyl, C₁-C₆ Alkoxy; gegebenenfalls mit C₁-C₄-Alkyl, C₁-C₄-Alkoxy, C₁-C₄-Haloalkoxy, C₁-C₄-Haloalkyl, Halogen, Cyano, Nitro substituiertes Phenyl oder Benzyl;
- Q: ein in Stellung 4 verknüpfter Pyrazolring der Formel II 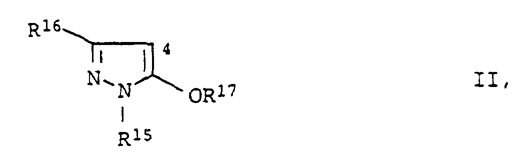 in welcher
- R¹⁵: C₁-C₄-Alkyl,
- R¹⁶: Wasserstoff, C₁-C₄-Alkyl oder C₁-C₄-Halogenalkyl und
- R¹⁷: Wasserstoff, C₁-C₄-Alkylsulfonyl, Phenylsulfonyl oder Alkylphenylsulfonyl bedeuten,
wobei für den Fall, daß Y = C=O ist, X ungleich NR²³ ist,
sowie landwirtschaftlich brauchbare Salze der Verbindung I.

Verbindungen der Formel I erhält man dadurch, daß man 5-Hydroxypyrazole der Formel IIa mit einem Benzoesäurederivat der Formel III umsetzt und zu Pyrazol-4-yl-benzoylderivaten der Formel I umlagert:

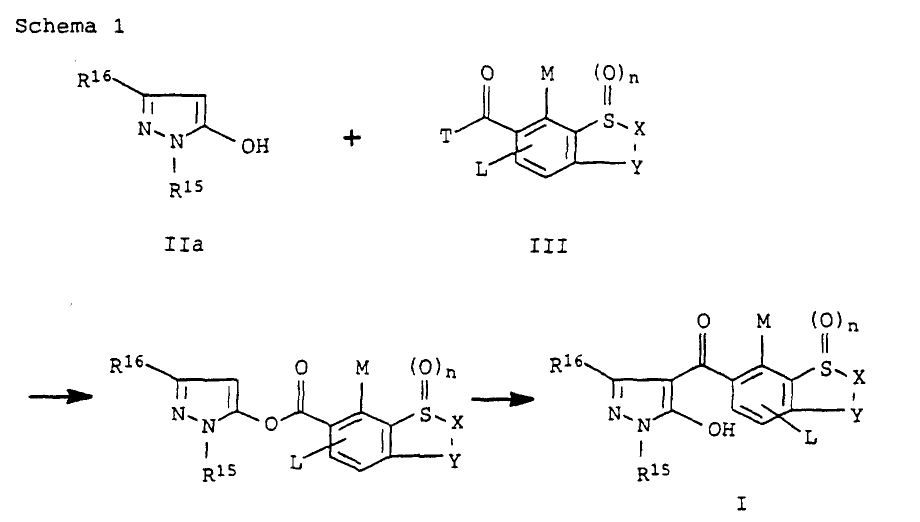

In dem obigen Schema 1 hat T in den genannten Formeln die Bedeutung Halogen oder OH und R¹⁵, R¹⁶, L, M, X, Y und n die oben angegebene Bedeutung.

Der erste Schritt der Reaktionsabfolge, die Acylierung, erfolgt in allgemein bekannter Weise z.B. durch Zugabe eines Säurechlorids der Formel III (T=C1) oder einer z.B. mit DCC (Dicyclocarbodiimide) oder ähnlichen literaturbekannten Mitteln z.B. Triphenylphosphin/DEAD = Diethylazodicarboxylat, 2-Pyridindisulfid/ Triphenylphosphin aktivierten Carbonsäuren III (T=OH) zur Lösung oder Suspension eines Cyclohexandions II gegebenenfalls in Gegenwart einer Hilfsbase. Die Reaktanden und die Hilfsbase werden dabei zweckmäßig in äquimolaren Mengen eingesetzt. Ein geringer Überschuß, z.B. 1,2 bis 1,5 Moläquivalente, bezogen auf II, der Hilfsbase kann u.U. vorteilhaft sein.

Als Hilfsbase eignen sich tertiäre Alkylamine, Pyridin oder Alkalicarbonate. Als Lösungsmittel können z.B. Methylenchlorid, Dioxan, Diethylether, Toluol, Acetonitril oder Essigsäureethylester verwendet werden.

Während der Zugabe des Säurechlorids wird die Reaktionsmischung vorzugsweise auf 0 bis 10°C gekühlt, danach wird bei einer Temperatur von 20 bis 100°C, insbesondere 25 bis 50°C gerührt, bis die Umsetzung beendet ist. Die Aufarbeitung erfolgt in üblicher Weise, z.B. wird das Reaktionsgemisch in Wasser gegossen und das Wertprodukt extrahiert, z.B. mit Methylenchlorid. Nach Trocknen der organischen Phase und Entfernung des Lösungsmittels kann der rohe Enolester ohne weitere Reinigung zur Umlagerung eingesetzt werden. Herstellungsbeispiele für Benzoesäureester von 5-Hydroxypyrazolen findet man z.B. EP-A-282 944 oder US 4,643,757.

Die Umlagerung der 5-Hydroxypyrazoylbenzoesäureester zu den Verbindungen der Formel I erfolgt zweckmäßig bei Temperaturen von 20 bis 40°C in einem Lösungsmittel und in Gegenwart einer Hilfsbase sowie mit eventueller Hilfe einer Cyanoverbindung als Katalysator.

Als Lösungsmittel kann z.B. Acetonitril, Methylenchlorid, tert.-Amylalkohol, Dioxan, 1,2-Dichlorethan, Essigsäureethylester oder Toluol verwendet werden. Bevorzugte Lösungsmittel sind Acetonitril und Dioxan. Als Hilfsbase eignen sich tertiäre Alkylamine, Pyridin oder Alkalicarbonate, die vorzugsweise in äquimolarer Menge oder bis zu einem vierfachen Überschuß, bezogen auf den 5-Hydroxypyrazolbenzoesäureester, eingesetzt werden. Bevorzugte Hilfsbasen sind Triethylamin und Alkalicarbonat in doppelter Menge.

Als Katalysator eignen sich Kaliumcyanid, Acetoncyanhydrin und Trimethylsilylcyanid, vorzugsweise in einer Menge von 1 bis 50 Molprozent, bezogen auf den Enolester. Bevorzugt setzt man Acetoncyanhydrin zu, z.B. in der Menge von 5 bis 15, insbesondere 10 Molprozent.

Beispiele zur Umlagerung von Benzoesäureestern von 5-Hydroxypyrazolen findet man z. B. in EP-A 282 944 oder US 4,643,757, jedoch wird dort lediglich Kaliumcarbonat oder Natriumcarbonat in Dioxan als Katalysator verwendet. Die Verwendung von Kaliumcyanid oder Acetoncyanhydrin ist zwar im Zusammenhang mit der analogen Umlagerung von Enolestern von Cyclohexan-1,3-dionen bekannt (US 4,695,673), jedoch sind aus der Literatur keine Beispiele bekannt, daß sich Cyanidverbindungen besonders gut zur Fries-Umlagerung von O-Acylderivaten des 5-Hydroxypyrazols eignen.

Die Aufarbeitung erfolgt in an sich bekannter Weise z. B. wird das Reaktionsgemisch mit verdünnten Mineralsäuren wie 5%iger Salzsäure oder Schwefelsäure angesäuert und mit einem organischen Lösungsmittel wie Methylenchlorid oder Essigsäureethylester extrahiert. Zur Reinigung wird der Extrakt mit kalter 5 bis 10%iger Alkalicarbonatlösung extrahiert, wobei das Endprodukt in die wäßrige Phase übergeht. Durch Ansäuern der wäßrigen Lösung wird das Produkt der Formel Ia-Ie ausgefällt oder erneut mit Methylenchlorid oder Essigsäureethylester extrahiert, getrocknet und anschließend vom Lösungsmittel befreit.

Die als Ausgangsmaterial verwendeten 5-Hydroxypyrazole der Formel II sind bekannt und können nach an sich bekannten Verfahren hergestellt werden (vgl. EP-A 240 001 und J. Prakt. Chem. 315, 382 (1973)). 1,3-Dimethyl-5-hydroxypyrazol ist eine käufliche Verbindung.

Benzoesäuren der Formel III lassen sich folgendermaßen herstellen:

Benzoylhalogenide wie beispielsweise Benzoylchloride der Formel III (T = Cl) werden in an sich bekannter Weise durch Umsetzung der Benzoesäuren der Formel III (T = OH) mit Thionylchlorid hergestellt.

Die Benzoesäuren der Formel III (T = OH) können in bekannter Weise durch saure oder basische Hydrolyse aus den entsprechenden Estern der Formel III (T = C₁-C₄-Alkoxy) hergestellt werden.

Die Zwischenprodukte der Formel III sind teilweise bekannt oder lassen sich nach literaturbekannten Verfahren darstellen.

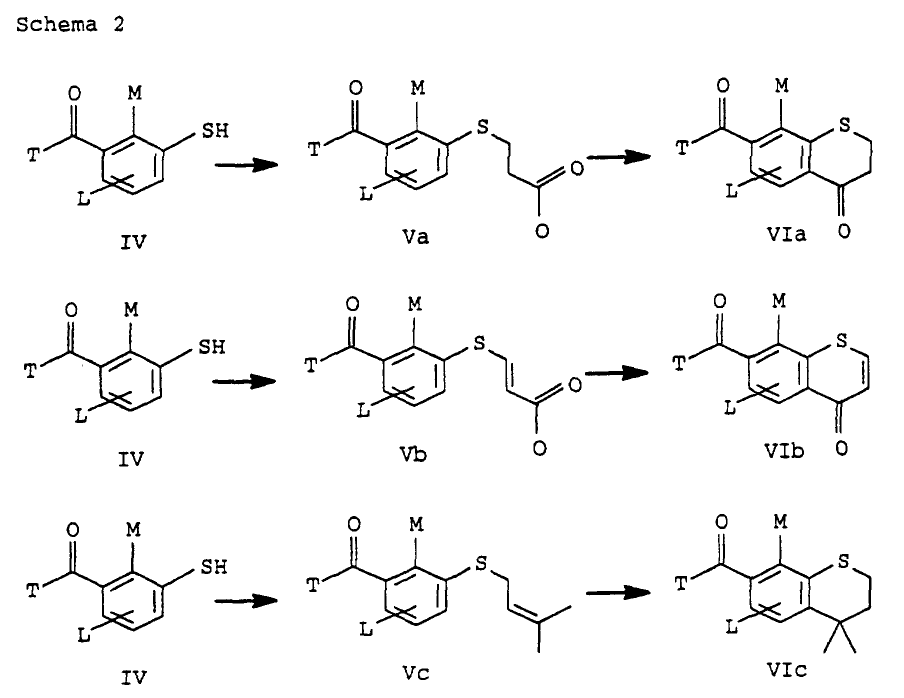

Danach lassen sich z.B. wie in Schema 2 dargestellt Arylthioverbindungen IV mit substituierten Haloalkenyl gemäß J. Med. Chem. 1984, 27, 1516, substituierten Alkinylcarbonsäuren gemäß J. Org.

Chem. 1980, 45, 4611 oder J. Am. Chem. Soc. 1983, 105, 883, substituierten Haloalkylcarbonsäuren gemäß Chem. Ber. 1925, 58, 1612 in Gegenwart einer Base wie Alkalihydroxid, Alkalihydrid oder Alkalicarbonat umsetzen. Die entstehenden Verbindungen V werden unter Friedel-Crafts Bedingungen unter Zusatz einer Lewissäure oder einer Protonensäure zu VI cyclisiert. Als Lewissäuren werden AlCl₃ oder SnCl₄ und als Protonensäure Polyphoshorsäure und Schwefelsäure bevorzugt gemäß Can. J. Chem. 1981, 59, 199; Chem. Ber. 1925, 58, 1625; Chem. Ber. 1926, 59, 1074; Phosp. and Sulf. 1984, 19, 31.

Thiochromenonsäuren lassen sich weiterhin durch z.B. Abspaltung von Halogenwasserstoff von 3-Halothiochromanonsäuren oder z.B. durch Umsetzung der substituierten Thiophenolsäuren mit substituierten α-Alkylacetessigestern in Gegenwart von Phosphorpentoxid gemäß Ann. Chem. 1964, 680, 40 darstellen.

Die Arylthioverbindungen IV können beispielsweise durch Sandmeyer-Reaktion aus entsprechenden Anilinen erhalten werden, die ihrerseits durch Reduktion von geeigneten Nitroverbindungen gemäß Organikum, 19. Auflage 1992, 552ff synthetisiert werden.

Für den Fall, daß z.B. X gleich (-CR¹²R¹³-) bzw. (-CR¹²R¹³CR²¹R²²-), Y gleich C=O und T gleich C₁-C₄-Alkoxy ist, läßt sich der Thiochromanonester bzw. Dihydrobenzothiophenester wie in Schema 2 beschrieben durch Alkylierung der Arylthioverbindung IV in Gegenwart einer der obengenannten Basen in Lösungsmittel oder Wasser mit Halogenpropionsäure bzw. Halogenessigsäure herstellen und cyclisieren zu VI.

Die Reaktanden und die Base werden dabei zweckmäßig in äquimolaren Mengen eingesetzt. Die Reaktionsmischung wird vorzugsweise bei 20-100°C, insbesondere bei 20-40°C, gerührt. Die Aufarbeitung erfolgt beispielsweise so, daß das Reaktionsgemisch auf Wasser gegossen wird, die wäßrige Phase mit Mineralsäuren wie Salzsäure oder Schwefelsäure sauer gestellt und das Wertprodukt abgesaugt wird oder durch Extraktion mit Methylenchlorid oder Essigsäureethylester extrahiert, getrocknet und vom Lösungsmittel befreit wird. Der Ester kann ohne weitere Reinigung umgesetzt werden.

Durch Rühren von V in z.B. Polyphosphorsäure bei 40-140°C, insbesondere bei 70-100°C, oder durch Aktivierung der Carbonsäure durch Überführung ins Säurechlorid und Rühren mit 2-6, insbesondere 3.5 bis 4.5, Moläquivalenten einer Lewissäure wie z.B. AlCl₃ oder SnCl₄ in einem Lösungsmittel oder durch Rühren mit oder in Schwefelsäure, erhält man nach an sich bekannter Aufarbeitung, d.h. Zugabe von Eiswasser und Absaugen des Wertproduktes oder Extraktion der wäßrigen Phase mit Essigsäureethylester oder Methylenchlorid, Trocknen und Entfernen des Lösungsmittel eine Zwischenstufe der Formel III.

Für den Fall, daß z.B. X gleich einer Ethylengruppierung (-CR¹²=CR¹³-), Y gleich C=O und T gleich C₁-C₄-Alkoxy läßt sich der Thiochromenonester durch z.B. Umsetzung von einer Arylthioverbindung mit einem Acetylencarbonsäurederivat in Wasser oder Lösungsmittel bei einer Temperatur von 0-140°C umsetzen. Die Aufarbeitung erfolgt nach an sich bekannter Weise durch Zugabe von Wasser und verdünnter Mineralsäure wie Salzsäure. Das Wertprodukt wird entweder abgesaugt oder durch Extraktion mit Methylenchlorid oder Essigsäureethylester, anschließendem Trocknen und Entfernen des Lösungsmittel erhalten.

Die Zwischenprodukte der Formel III können durch literaturbekannte Reaktionen wie Reduktion gemäß Jerry March " Advanced Organic Chemistry, Fourth Ed., z.B. S.910ff, Oximierung gemäß Jerry March " Advanced Organic Chemistry, Fourth Ed., z.B. S.934, 935, 1039, 1226, 405ff, Überführung in Imine und Amine gemäß Jerry March " Advanced Organic Chemistry, Fourth Ed., Ketalisierung, Alkylierung, Halogenierung, Eliminierung und Oxidation gemäß Jerry March " Advanced Organic Chemistry, Fourth Ed. weiter funktionalisiert werden.

Die Säuren der 3-Alkoxy-1,2-benzisothiazol-1,1-dioxide oder 3-Alkoxy-1,2-benzisothiazole können ausgehend von entsprechenden Saccharinderivaten oder 1,2-Benzisothiazolen durch z.B. Umsetzung mit PCl₅, POCl₃ oder Chlor und Alkohol gegebenenfalls in Gegenwart einer Hilfsbase wie z.B. Triethylamin erhalten werden, was beispielsweise beschrieben ist in US 4,571,429, Arch. Pharm. 1984, 317, 807, US 4,461,901, US 450,916, J. Med. Chem. 1986, 29, 359. Saccharincarbonsäuren können durch Literatur bekannte Verfahren gemäß Ann. Chem. 427, 231, 1922, Chem. Ber. 13, 1554, 1980, Chem. Ber. 25, 1740, 1892, DE-OS 3607343, deutsche Patentanmeldung P 44 27 995.7 erhalten werden.

Die Derivate der Benzo-1,4-oxathiinsäuren sind teilweise bekannt, z.B. aus J. Org. Chem. 1968, 33, 456 oder lassen sich z.B. durch Reaktion aus den entsprechenden Phenolderivaten gemäß Chem. Comm., 1975, 451, J. Org. Chem. 1974, 39, 1811, J. Am. Chem. Soc. 1954, 76, 1068 oder durch Kombination von z.B. Substitutionsreaktion an Halogen-substituierten Thiophenolderivaten und weiterführende Reaktionen wie z.B Oxidation, Reduktion oder Addition gemäß J. Het. Chem. 1983, 20, 867 aufbauen.

Die Benzoesäuren der Formel III können auch erhalten werden, indem man die entsprechende brom- oder iodsubstituierte Verbindung der Formel VII 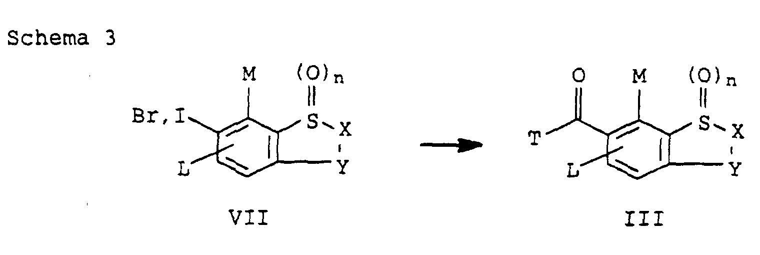
- T: OH, C₁-C₄-Alkoxy
- Y,L,M,X,: die oben beschriebene Bedeutung haben
in Gegenwart eines Palladium-, Nickel-, Cobalt- oder Rhodium-Übergangsmetallkatalysators und einer Base mit Kohlenmonoxid und Wasser unter erhöhtem Druck umsetzt.

Die Katalysatoren Nickel, Cobalt, Rhodium und insbesondere Palladium können metallisch oder in Form üblicher Salze wie in Form von Halogenverbindungen, z.B. PdCl₂, RhCl₃·H₂O, Acetaten, z.B. Pd(OAc)₂, Cyaniden usw. in den bekannten Wertigkeitsstufen vorliegen. Ferner können Metallkomplexe mit tertiären Phosphinen, Metallalkylcarbonyle, Metallcarbonyle, z.B. CO₂(CO)₈, Ni(CO)₄, Metallcarbonyl-Komplexe mit tertiären Phosphinen, z.B. [P(Ph)₃]₂Ni(CO)₂, oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 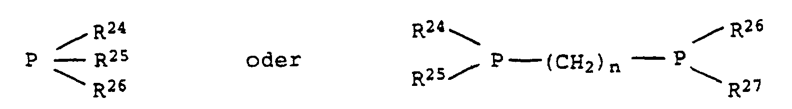 wobei n die Zahlen 1, 2, 3 oder 4 bedeutet und die Reste R²⁴ bis R²⁶ für niedermolekulares Alkyl, z.B. C₁-C₆-Alkyl, Aryl, C₁-C₄-Alkylaryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise, z.B. wie in den eingangs genannten Dokumenten beschrieben, erfolgen. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)₂, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Moläquivalente, besonders bevorzugt 1 bis 5 Moläquivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff II bzw. III verwenden.

Zur Herstellung der Benzoesäuren III (T = OH) führt man die Umsetzung mit Kohlenmonoxid und mindestens äquimolaren Mengen an Wasser, bezogen auf die Ausgangsstoffe VI durch. Der Reaktionspartner Wasser kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether z.B. Methyl-tert.butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base, im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie tert.-Alkylamine, z.B. Trialkylamine wie Triethylamin, cyclische Amine wie N-Methylpiperidin oder N,N'-Dimethylpiperazin, Pyridin, Alkali- oder -hydrogencarbonate, oder tetraalkylsubstituierte Harnstoffderivate wie Tetra-C₁-C₄-alkylharnstoff, z.B. Tetramethylharnstoff, zu nennen.

Die Menge an Base ist nicht kritisch; üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf VI vorliegt. Vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Die als Ausgangsverbindungen benutzten Arylhalogenverbindungen VII sind bekannt oder können leicht durch geeignete Kombination bekannter Synthesen und nach oben beschriebenen Reaktionsfolgen hergestellt werden.

Im Hinblick auf die bestimmungsgemäße Verwendung der Pyrazol-4-yl-benzoylderivate der allgemeinen Formel I kommen als Substituenten folgende Reste in Betracht:
L,M Wasserstoff,
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-l-methylpropyl oder l-Ethyl-2-methyl-propyl,
insbesondere Methyl, Ethyl, 1-Methylethyl, 1-Methylpropyl, 2-Methylpropyl, 1,1-Dimethylethyl und 1,1-Dimethylpropyl;
C₂-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3 butenyl, 1,1-Dimethyl-2-propenyl,1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 5 3-Methyl-2-pentenyl, 4-Methyl-2-pentenyl, 1-Methyl-3 pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2, 3-Dimethyl-2-butenyl, 2,3-Dimethyl-3-butenyl, 1-Ethyl-2-butenyl, 1-Ethyl-3 butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
insbesondere 1-Methyl-2-propenyl, 1-Methyl-2-butenyl, 1,1-Dimethyl-2-propenyl und 1,1-Dimethyl-2-butenyl;
C₂-C₆-Alkinyl wie Propargyl, 2-Butinyl, 3-Butenyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2 propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-l-methyl-2-propinyl;
C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy,
insbesondere C₁-C₃-Alkoxy wie Methoxy, Ethoxy, i-Propoxy,
wobei diese Gruppen gegebenenfalls durch ein bis fünf Halogenatome wie Fluor, Chlor, Brom und Iod, vorzugsweise Fluor und Chlor oder C₁-C₄-Alkoxy wie vorstehend genannt substituiert sein können.

Die vorstehend definierte Gruppe -(A)ₘ-S(O)ₙR¹ steht beispielsweise für
C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio;
C₁-C₄-Alkylsulfinyl wie Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl und 1,1-Dimethylethylsulfinyl, insbesondere Methylsulfinyl;
C₁-C₄-Alkylsulfonyl wie Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl und 1,1-Dimethylethylsulfonyl, insbesondere Methylsulfonyl;
C₁-C₄-Alkoxysulfonyl wie Methoxysulfonyl, Ethoxysulfonyl, n-Propoxysulfonyl, 1-Methylethoxysulfonyl, n-Butoxysulfonyl, 1-Methylpropoxysulfonyl, 2-Methylpropoxysulfonyl und 1,1-Dimethylethoxysulfonyl, insbesondere Methoxysulfonyl;
N-C₁-C₄-Alkylsulfamoyl wie N-Methylsulfamoyl, N-Ethylsulfamoyl, N-n-Propylsulfamoyl, N-1-Methylethylsulfamoyl, N-n-Butylsulfamoyl, N-1-Methylpropylsulfamoyl, N-2-Methylpropylsulfamoyl und N-1,1-Dimethylethylsulfamoyl, insbesondere N-Methylsulfamoyl;
N-C₁-C₄-Alkylsulfinamoyl wie N-Methylsulfinamoyl, N-Ethylsulfinamoyl, N-n-Propylsulfinamoyl, N-1-Methylethylsulfinamoyl, N-n-Butylsulfinamoyl, N-1-Methylpropylsulfinamoyl, N-2-Methylpropylsulfinamoyl und N-1,1-Dimethylethylsulfinamoyl, insbesondere N-Methylsulfinamoyl;
Di-C₁-C₄-Alkylsulfamoyl wie Dimethylsulfamoyl, Diethylsulfamoyl, Dipropylsulfamoyl, Dibutylsulfamoyl, N-Methyl-N-ethylsulfamoyl, N-Methyl-N-propylsulfamoyl, N-Methyl-N-1-methylethylsulfamoyl, N-Methyl-N-1,1-Dimethylethylsulfamoyl, Di-1-Methylethylsulfamoyl, N-Ethyl-N-1-Methylethylsulfamoyl und N-Ethyl-N-1,1-dimethyl ethylsulfamoyl; insbesondere Dimethylsulfamoyl;
Di-C₁-C₄-Alkylsulfinamoyl wie Dimethylsulfinamoyl, Diethylsulfinamoyl, Dipropylsulfinamoyl, Dibutylsulfinamoyl, N-Methyl-N-ethylsulfinamoyl, N-Methyl-N-propylsulfinamoyl, N-Methyl-N-1-methylethylsulfinamoyl, N-Methyl-N-1,1-Dimethylethylsulfinamoyl, Di-1-Methylethylsulfinamoyl, N-Ethyl-N-1-Methylethylsulfinamoyl und N-Ethyl-N-1,1-dimethylethylsulfinamoyl; insbesondere Dimethylsulfinamoyl,
C₁-C₄-Alkylsulfinyloxy wie Methylsulfinyloxy, Ethylsulfinyloxy, n-Propylsulfinyloxy, 1-Methylethylsulfinyloxy, n-Butylsulfinyloxy, l-Methylpropylsulfinyloxy, 2-Methylpropylsulfinyloxy und 1,1-Dimethylethylsulfinyloxy, insbesondere Methylsulfinyloxy; C₁-C₄-Alkylsulfonyloxy wie Methylsulfonyloxy, Ethylsulfonyloxy, n-Propylsulfonyloxy, 1-Methylethylsulfonyloxy, n-Butylsulfonyloxy, 1-Methylpropylsulfonyloxy, 2-Methylpropylsulfonyloxy und 1,1-Dimethylethylsulfonyloxy, insbesondere Methylsulfonyloxy;
C₁-C₄-Alkylsulfinylamino wie Methylsulfinylamino, Ethylsulfinylamino, n-Propylsulfinylamino, 1-Methylethylsulfinylamino, n-Butylsulfinylamino, 1-Methylpropylsulfinylamino, 2-Methylpropylsulfinylamino und 1,1-Dimethylethylsulfinylamino, insbesondere Methylsulfinylamino;
C₁-C₄-Alkylsulfonylamino wie Methylsulfonylamino, Ethylsulfonylamino, n-Propylsulfonylamino, 1-Methylethylsulfonylamino, n-Butylsulfonylamino, 1-Methylpropylsulfonylamino, 2-Methylpropylsulfonylamino und 1,1-Dimethylethylsulfonylamino, insbesondere Methylsulfonylamino;
N-C₁-C₄-Alkylsulfinyl-N-methyl-amino wie N-Methylsulfinyl-N-methyl-amino, N-Ethylsulfinyl-N-methyl-amino, N-n-Propylsulfinyl-N-methyl-amino, N-1-Methylethylsulfinyl-N-methyl-amino, N-n-Butylsulfinyl-N-methyl-amino, N-1-Methylpropylsulfinyl-N-methyl-amino, N-2-Methylpropylsulfinyl-N-methyl-amino und N-1,1-Dimethylethylsulfinyl-N-methyl-amino, insbesondere N-Methylsulfinyl-N-methyl-amino;
N-C₁-C₄-Alkylsulfinyl-N-ethyl-amino wie N-Methylsulfinyl-N-ethylamino, N-Ethylsulfinyl-N-ethyl-amino, N-n-Propylsulfinyl-N-ethylamino, N-1-Methylethylsulfinyl-N-ethyl-amino, N-n-Butylsulfinyl-N-ethyl-amino, N-1-Methylpropylsulfinyl-N-ethyl-amino, N-2-Methylpropylsulfinyl-N-ethyl-amino und N-1,1-Dimethylethylsulfinyl-N-ethyl-amino, insbesondere N-Methylsulfinyl-N-ethylamino;
N-C₁-C₄-Alkylsulfonyl-N-methyl-amino wie N-Methylsulfonyl-N-methyl-amino, N-Ethylsulfonyl-N-methyl-amino, N-n-Propylsulfonyl-N-methyl-amino, N-1-Methylethylsulfonyl-N-methyl-amino, N-n-Butylsulfonyl-N-methyl-amino, N-1-Methylpropylsulfonyl-N-methyl-amino, N-2-Methylpropylsulfonyl-N-methyl-amino und N-1,1-Dimethylethylsulfonyl-N-methyl-amino, insbesondere N-Methylsulfonyl-N-methyl-amino;
N-C₁-C₄-Alkylsulfonyl-N-ethyl-amino wie N-Methylsulfonyl-N-ethylamino, N-Ethylsulfonyl-N-ethyl-amino, N-n-Propylsulfonyl-N-ethylamino, N-1-Methylethylsulfonyl-N-ethyl-amino, N-n-Butylsulfonyl-N-ethyl-amino, N-1-Methylpropylsulfonyl-N-ethyl-amino, N-2-Methylpropylsulfonyl-N-ethyl-amino und N-1,1-Dimethylethylsulfonyl-N-ethyl-amino, insbesondere N-Methylsulfonyl-N-ethylamino;
C₁-C₄-Halogenalkylthio wie Chlormethylthio, Dichlormethylthio, Trichlormethylthio, Fluormethylthio, Difluormethylthio, Trifluormethylthio, Chlorfluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio.

Die vorstehend definierte Gruppe -(A)ₘ-CO-R² steht beispielsweise für
C₁-C₄-Alkylcarbonyl wie Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl und 1,1-Dimethylethylcarbonyl, insbesondere Methylcarbonyl;
C₁-C₄-Alkoxycarbonyl wie Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl und 1,1-Dimethylethoxycarbonyl, insbesondere Methoxycarbonyl;
N-C₁-C₄-Alkylcarbamoyl wie N-Methylcarbamoyl, N-Ethylcarbamoyl, N-n-Propylcarbamoyl, N-1-Methylethylcarbamoyl, N-n-Butylcarbamoyl, N-1-Methylpropylcarbamoyl, N-2-Methylpropylcarbamoyl und N-1,1-Dimethylethylcarbamoyl, insbesondere N-Methylcarbamoyl;
Di-C₁-C₄-Alkylcarbamoyl wie Dimethylcarbamoyl, Diethylcarbamoyl, Dipropylcarbamoyl, Dibutylcarbamoyl, N-Methyl-N-ethylcarbamoyl, N-Methyl-N-propylcarbamoyl, N-Methyl-N-1-methylethylcarbamoyl, N-Methyl-N-1,1-Dimethylethylcarbamoyl, Di-1-Methylethylcarbamoyl, N-Ethyl-N-1-Methylethylcarbamoyl und N-Ethyl-N-1,1-dimethylethylcarbamoyl; insbesondere Dimethylcarbamoyl;
C₁-C₄-Alkylcarbonyloxy wie Methylcarbonyloxy, Ethylcarbonyloxy, n-Propylcarbonyloxy, 1-Methylethylcarbonyloxy, n-Butylcarbonyloxy, l-Methylpropylcarbonyloxy, 2-Methylpropylcarbonyloxy und 1,1-Dimethylethylcarbonyloxy, insbesondere Methylcarbonyloxy; C₁-C₄-Alkylcarbonylamino wie Methylcarbonylamino, Ethylcarbonylamino, n-Propylcarbonylamino, l-Methylethylcarbonylamino, n-Butylcarbonylamino, 1-Methylpropylcarbonylamino, 2-Methylpropylcarbonylamino und 1,1-Dimethylethylcarbonylamino, insbesondere Methylcarbonylamino;
N-C₁-C₄-Alkylcarbonyl-N-methyl-amino wie N-Methylcarbonyl-N-methyl-amino, N-Ethylcarbonyl-N-methyl-amino, N-n-Propylcarbonyl-N-methyl-amino, N-1-Methylethylcarbonyl-N-methyl-amino, N-n-Butylcarbonyl-N-methyl-amino, N-1-Methylpropylcarbonyl-N-methyl-amino, N-2-Methylpropylcarbonyl-N-methyl-amino und N-1,1-Dimethylethylcarbonyl-N-methyl-amino, insbesondere N-Methylcarbonyl-N-methyl-amino.
   X steht beispielsweise für:
      CH₂, CH(CH₃), C((CH₃)₂), CH(C₂H₅), C((C₂H₅)₂), CH(C₆H₅), CH₂-CH₂, CH₂-CH(CH₃), CH₂-C((CH₃)₂), CH(CH₃)-CH(CH₃), CH(CH₃)-C((CH₃)₂), C( (CH₃)₂)-C((CH₃)₂),
      CH₂-CH(C₂R₅), CH₂-C((C₂H₅)₂), CH(C₂H₅)-CH(C₂H₅), CH(C₂H₅)-C((C₂H₅)₂), C((C₂H₅)₂)-C((C₂H₅)₂), CH₂-CH(C₃H₇), CH₂-CH(iC₃H₇), CH₂-CH(C₄H₉), CH₂-CH(iC₄H₉),
      CH₂-CH(Br), CH₂-C((Br)₂), CH(Br)-CH(Br), C((Br)₂)-C((Br)₂),
      CH₂-CH(Cl), CH₂-C((Cl)₂), CH(Cl)-C((Cl₂), C((Cl)₂)-C((Cl)₂),
      CH₂-CH(C₆H₅), CH(C₆H₅)-CH(C₆H₅), CH₂-CH(p-NO₂C₆H₅),
      CH=CH, C(CH₃)=CH, C(CH₃)=CCH₃, CH=CBr, CH=CCl, CBr=CBr, CCl=CCl, CH=C(OCH₃), CH=C(C₆H₅), C(C₆H₅)=C(C₆H₅), C(C₂H₅)=CH,
      C(C₂H₅)=C(C₂H₅), CH=C(C₃H₅), CH=C(C₄H₇), CH₂-CH=CH, CH(CH₃)-CH=CH, C ((CH₃)₂)-CH=CH, CH₂-CH=C(CH₃), CH₂-C(CH₃)=CH, CH₂-C(CH₃)=C(CH₃), CH(CH₃)-C(CH₃)=C(CH₃), C((CH₃)₂)-C(CH₃)=C(CH₃), N-H, N-CH₃, N-C₂H₅, N-C₃H₇, N-C₄Hg, N-iCH₃H₇, N-OCH₃, N-OC₂H₅, N-CH₂C₆H₅, N-C₆H₅;
   Y steht beispielsweise für:
      C=O, CH-OH, CH-OCH₃, CH-OC₂H₅, CH-OC₃H₇, CH-OiPr, CH-OC₄H₉, CH-OiBu, CH-OC₅H₁₁, CH-OC₆H₁₃, CH-OC₆H₅, C(CH₃)-OCH₃, C (CH₃)-OC₂H₅. C(CH₃)-OC₃H₇, C(CH₃)-OC₄H₉, C(CH₃)-OiPr, C(CH₃)-OiBu, C(CH₃)-OtBu, C(CH₃)-OPh, CH₂, CH(CH₃), C((CH₃)₂), C=N-CH_{3,} C=N-C₂H₅, C=N-C₃H₇, C=N-C₄H₉, C=N-iC₄H₉, C=N-tC₄H₉, C=N-iPr, C=N-OCH₃, C=N-OC₂H₅, C=N-OC₃H₇, C=N-OC₄H₉, C=N-OiC₄H₉, C=N-OtC₄H₉, C=N-OCH₂CH=CH₂, C=N-OCH(CH₃₎ CH=CH₂, C=N-OCH₂CH=CHCH₃, C=N-OCH₂CH=C(CH₃)₂, C=N-OCH₂CH=CHBr, C=N-OCH₂CH=CHCl, C=N-OCH₂CH=CHC₂H₅, C=N-OCH₂C≡CH, C=N-OCH₂C≡CCH3, C=N-OCH₂C₆H₅, CH-NH(OCH₃), CH-NH(OC₂H₅), CH-NH(OiPr), CH-NH(OnPr), CH-NH(OC₆H₅), CH-NCH₃(OCH₃), CH-NCH₃(OC₂H₅), CH-NCH₃(OiPr), CH-NCH₃(OnPr), CH-NCH₃(OC₆H₅), CH-NH(CH₃), CH-NH(C₂H₅), CH-NH(C₃H₇), CH-NH(C₄H₉), CH-NH(iPr), CH-NH(iBu), CH-NH(tBu), CH-NH(C₆H₅), CH-N(CH₃)₂. CH-NCH₃(C₂H₅), CH-NCH₃(C₃H₇), CH-NCH₃(C₄H₉), CH-NCH₃(iPr), CH-NCH₃(iBu), C=N-NH₂, C=N-NHCH₃, C=N-N((CH₃)₂), C=N-NH(C₂H₅), C=N-NCH₃(C₂H₅), C=N-N((C₂H₅)₂), CH-SCH₃, CH-SC₂H₅, CH-SC₃H₇, CH-SC₄H₉, CH-SPr, CH-SiBu, CH-SH, C(CH₃)-SCH₃, C(CH₃)-SC₂H₅, C(CH₃)-SC₃H₇, 1,3-Dioxanyl, 1,3-Dioxolanyl, 5,5-Dimethyl-1,3-dioxanyl.

Bevorzugt sind Pyrazol-4-yl-benzoylderivate der Formel Ia 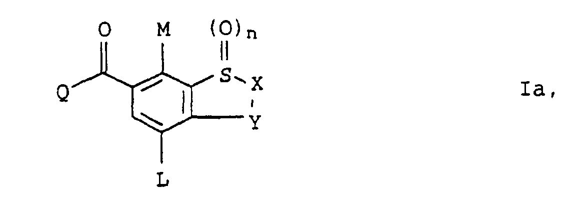 in der L für Wasserstoff, C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkoxy, C₁-C₄-Halogenalkylthio, C₁-C₄-Alkylsulfonyl, Halogen, Nitro oder Cyano steht und Q, X, n und Y die oben angegebenen Bedeutungen haben, wobei für den Fall, daß Y = C=O ist, X ungleich NR²³ ist.

Weiterhin bevorzugt sind Pyrazol-4-yl-benzoylderivate der Formel Ib 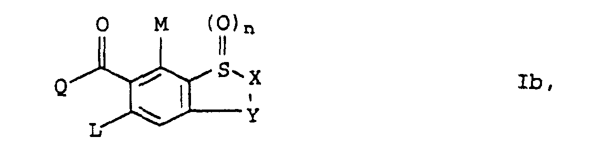 in der L für C₁-C₆-Alkyl, C₂-C₆-Alkenyl, C₂-C₆-Alkinyl, C₁-C₄-Alkoxyl C₁-C₄-Halogenalkyl, C₁-C₄-Halogenalkoxy, Halogen, Nitro oder Cyano und M für Wasserstoff C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und Q, X, n und Y die oben angegebenen Bedeutungen haben, wobei für den Fall, daß Y = C=O ist, X ungleich NR²³ ist.

Bevorzugt sind auch Pyrazol-4-yl-benzoylderivate der Formel I, in der die Reste L bzw. M für Wasserstoff, Methyl, Methoxy, Chlor, Cyano, Nitro, Trifluormethyl stehen.

Bevorzugt sind Pyrazol-4-yl-benzoylderivate der Formel Ic 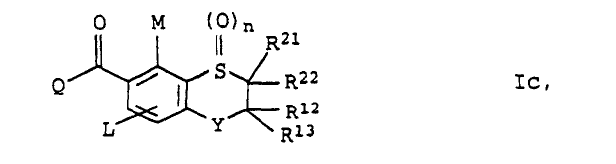 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und Q, n, Y sowie R²², R²¹, R¹² und R¹³ die oben angegebenen Bedeutungen haben.

Ebenfalls bevorzugt sind Pyrazol-4-yl-benzoylderivate der Formel Id 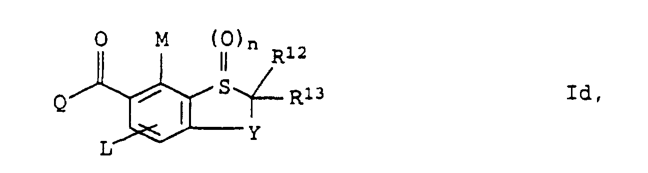 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und Q, n, Y sowie R¹² und R¹³ die oben angegebenen Bedeutungen haben.

Auch bevorzugt sind Pyrazol-4-yl-benzoylderivate der Formel Ie 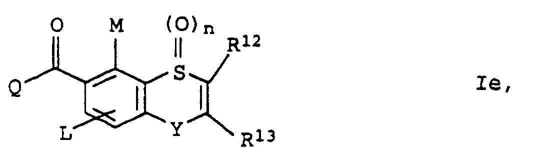 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und Q, n, Y sowie R¹² und R¹³ die oben angegebenen Bedeutungen haben.

Bevorzugt sind ebenfalls Pyrazol-4-yl-benzoylderivate der Formel I, in der n für eins oder zwei steht und Y für CR⁷-OR⁸, wobei R⁷ und R⁸ die oben angegebene Bedeutung haben.

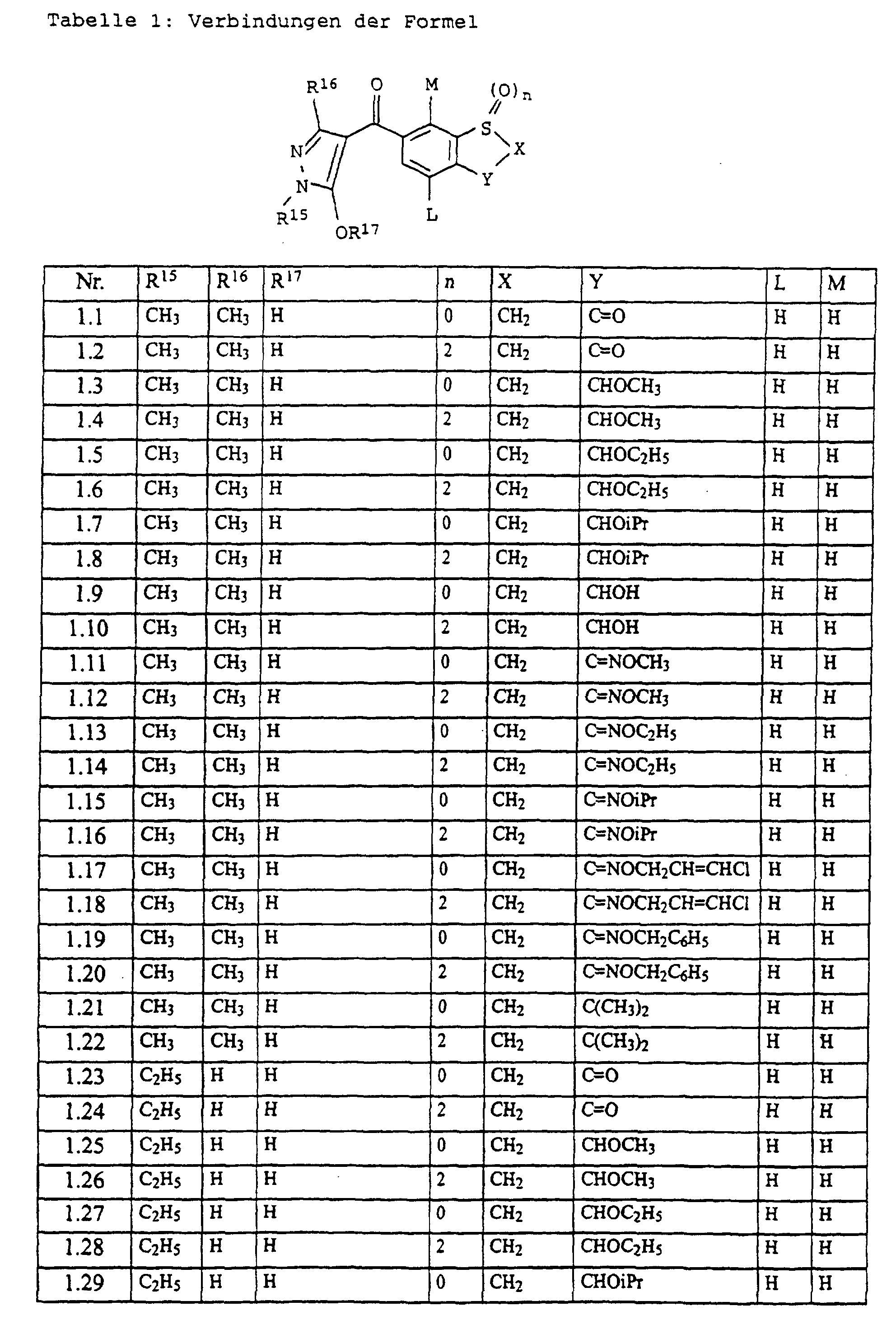

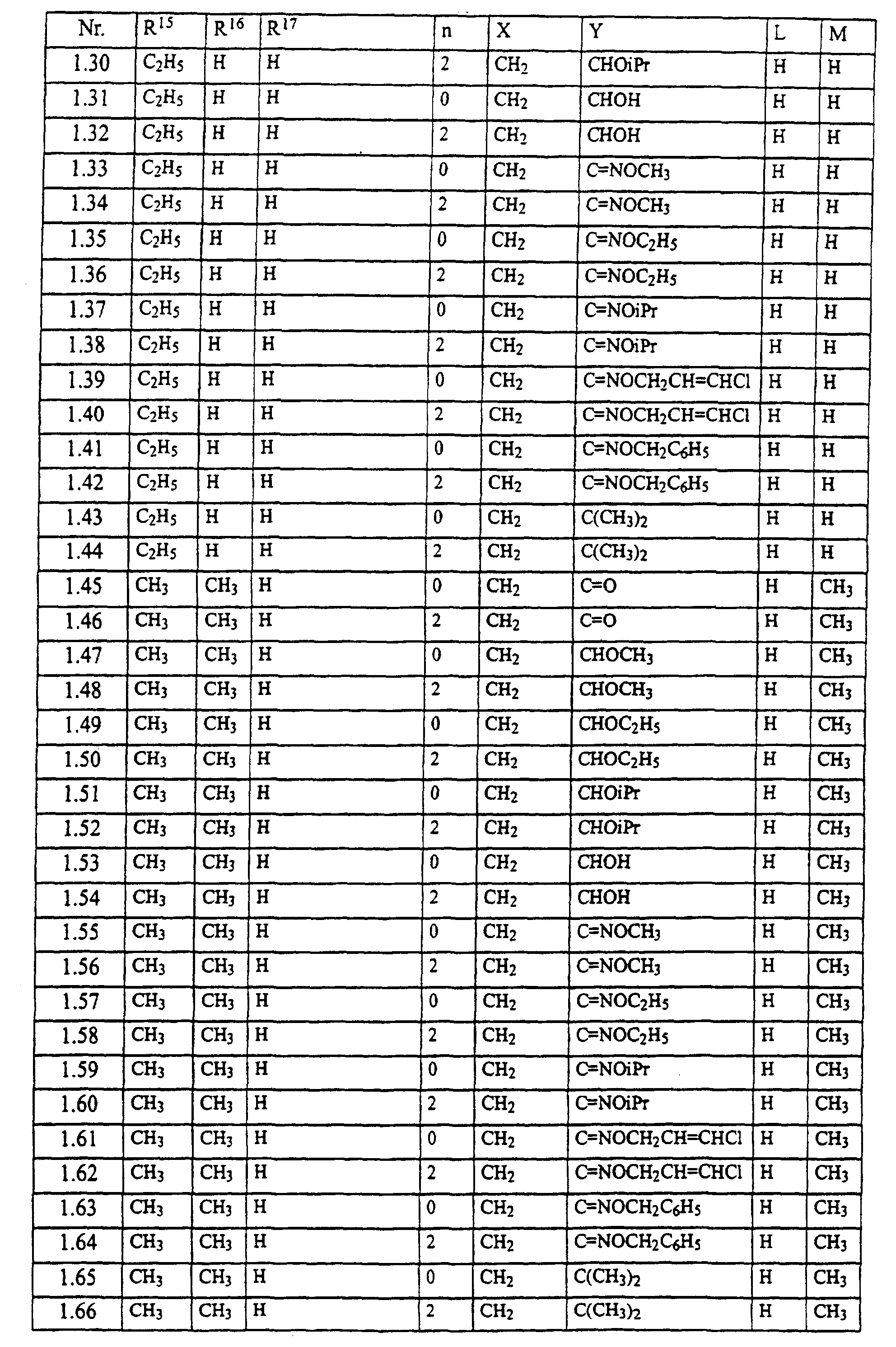

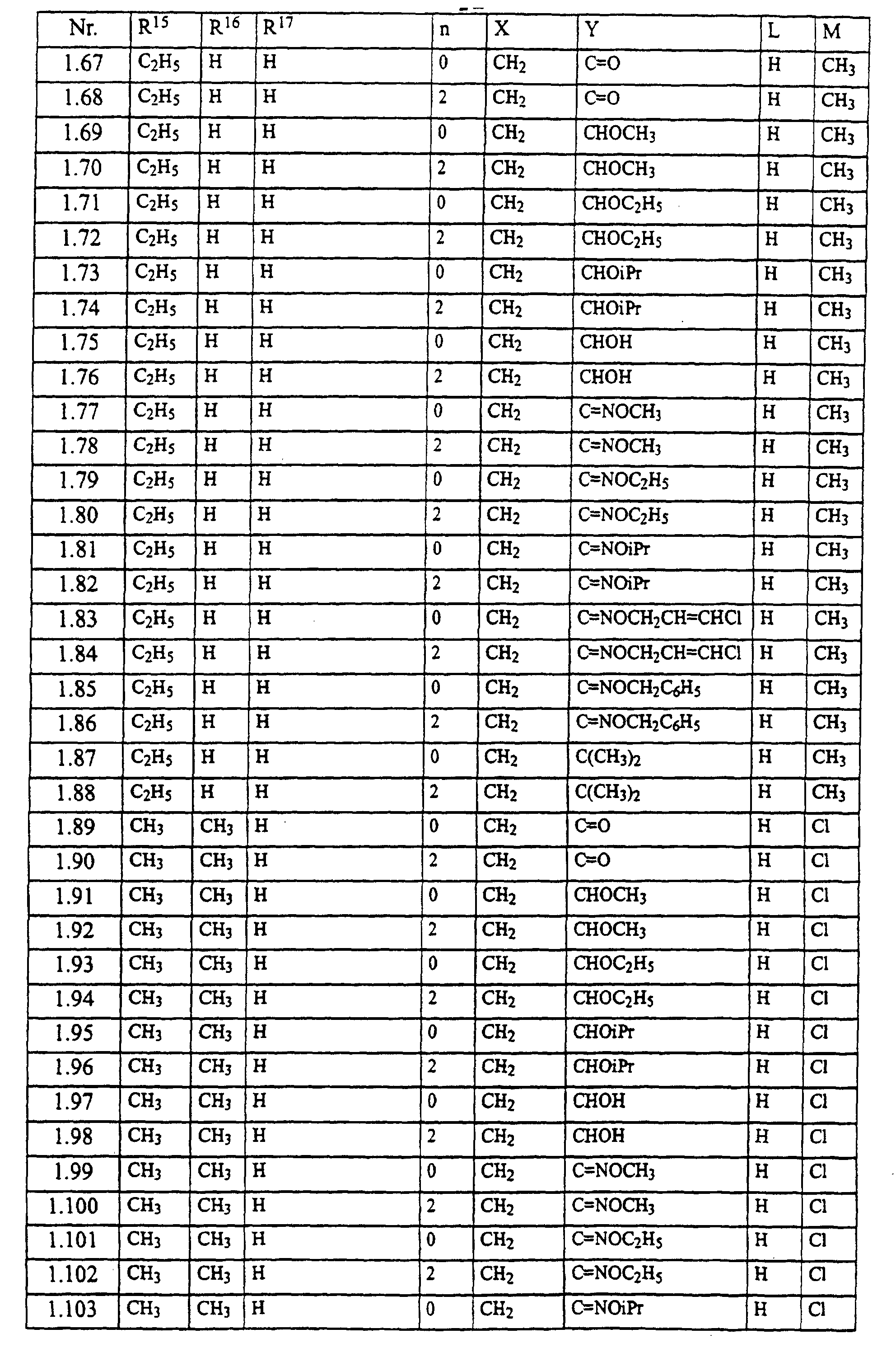

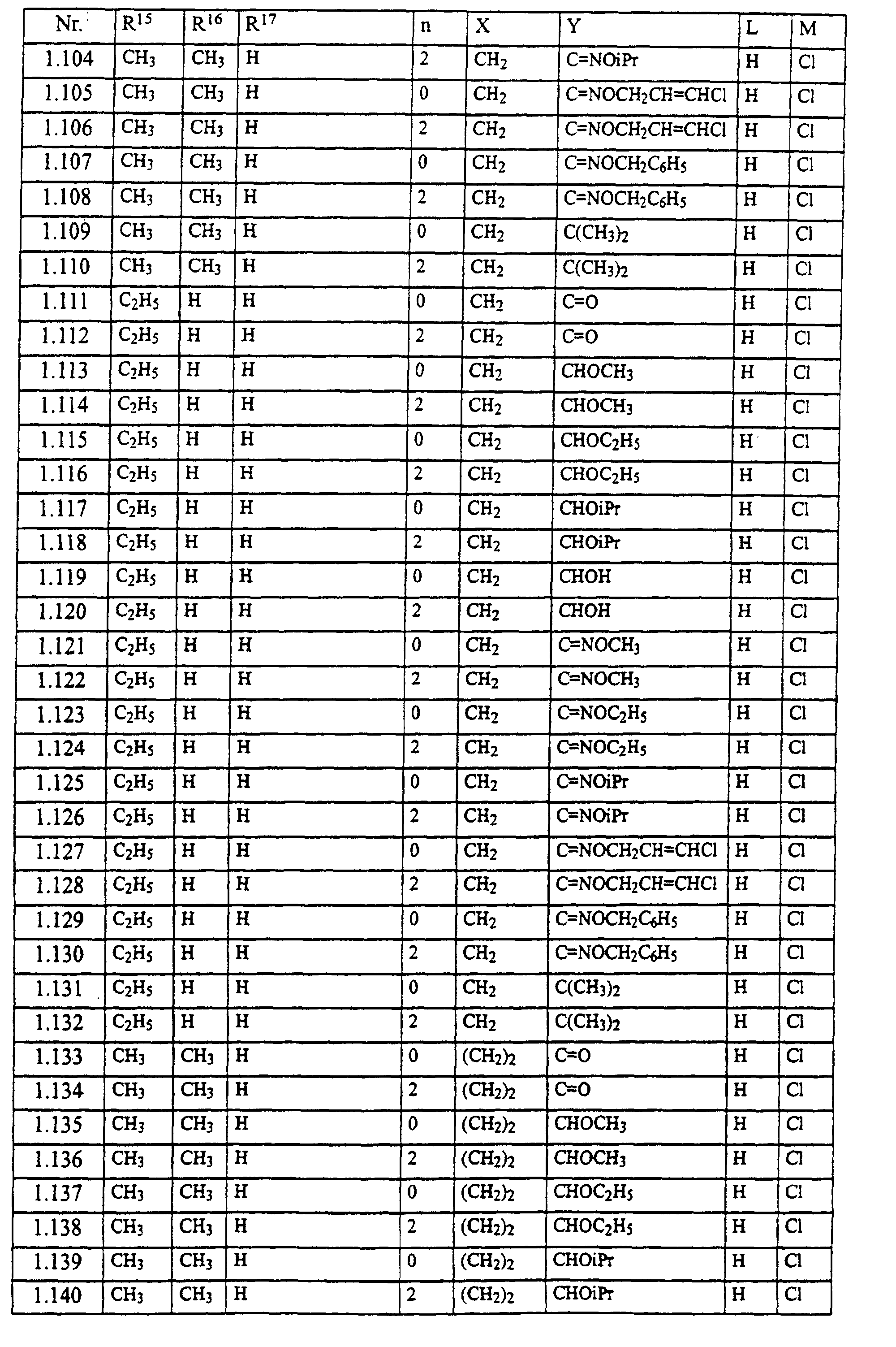

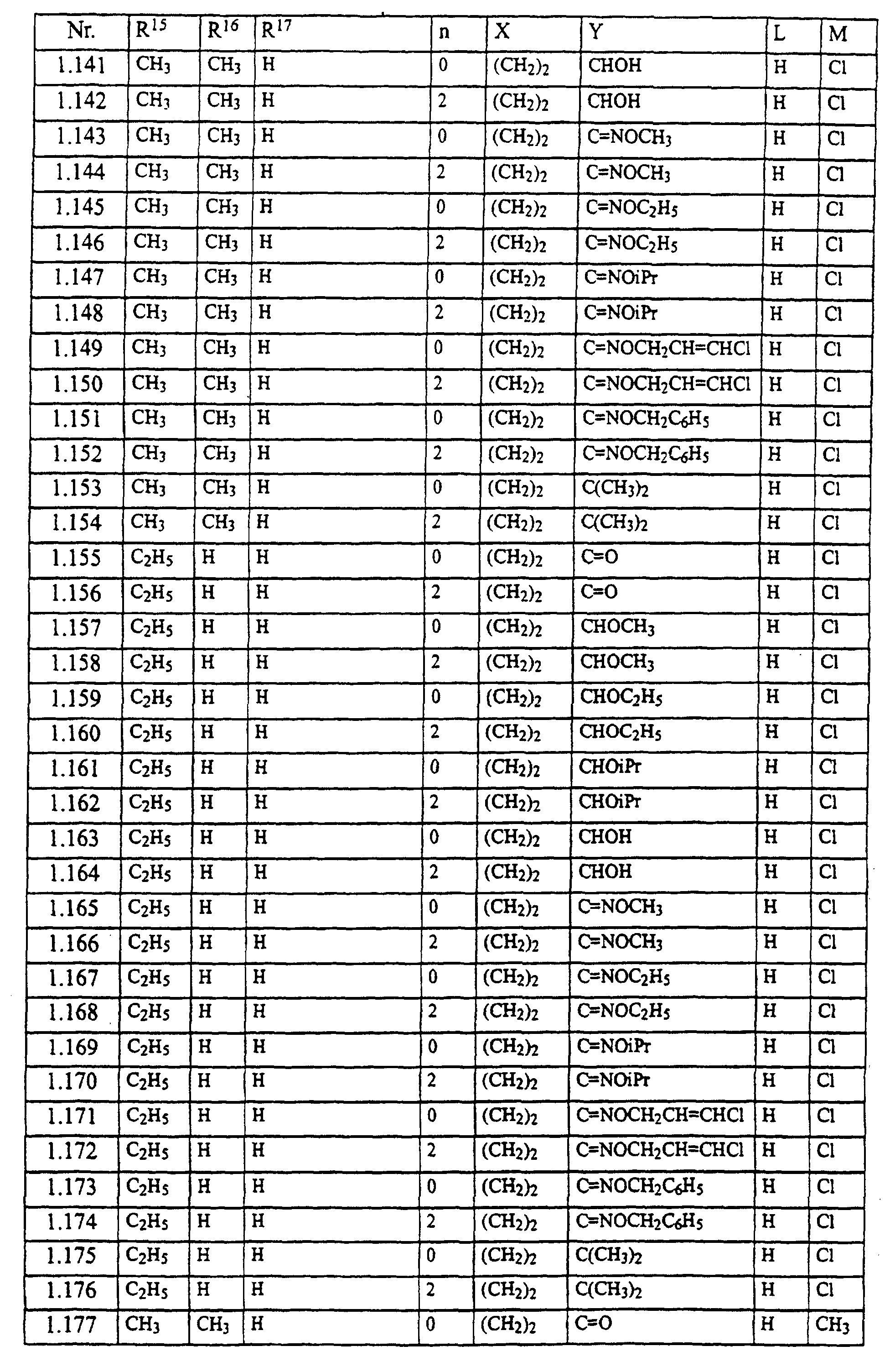

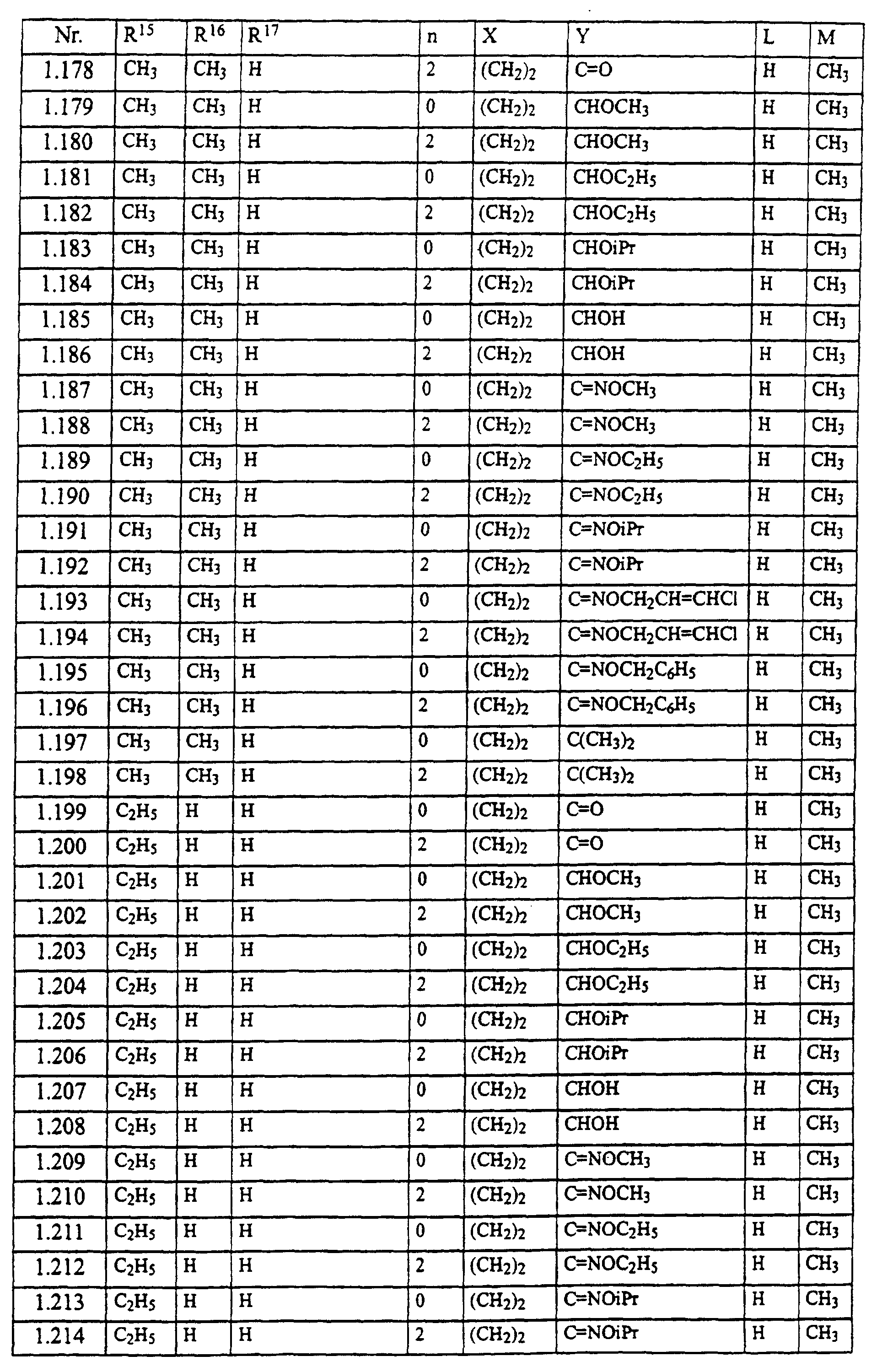

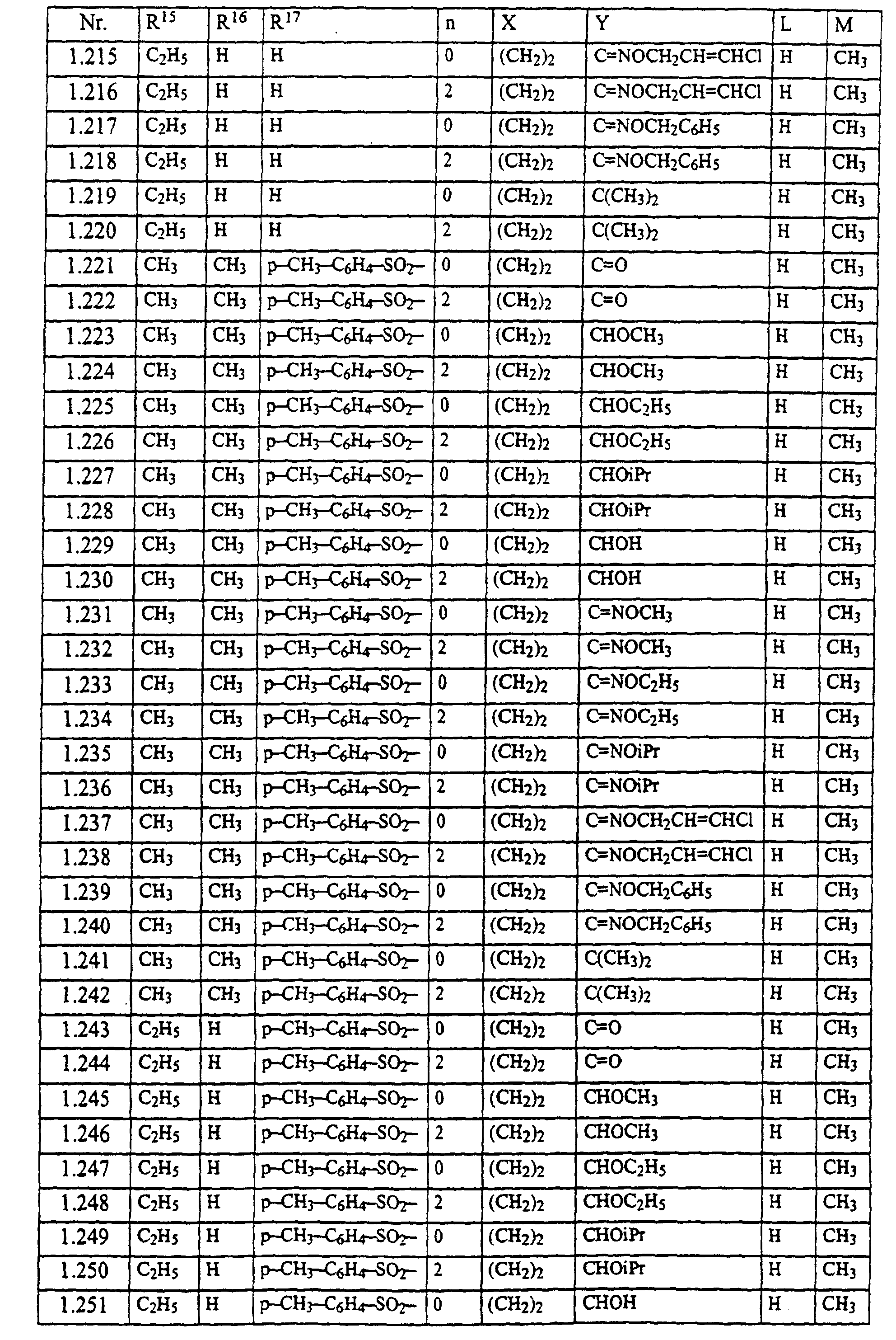

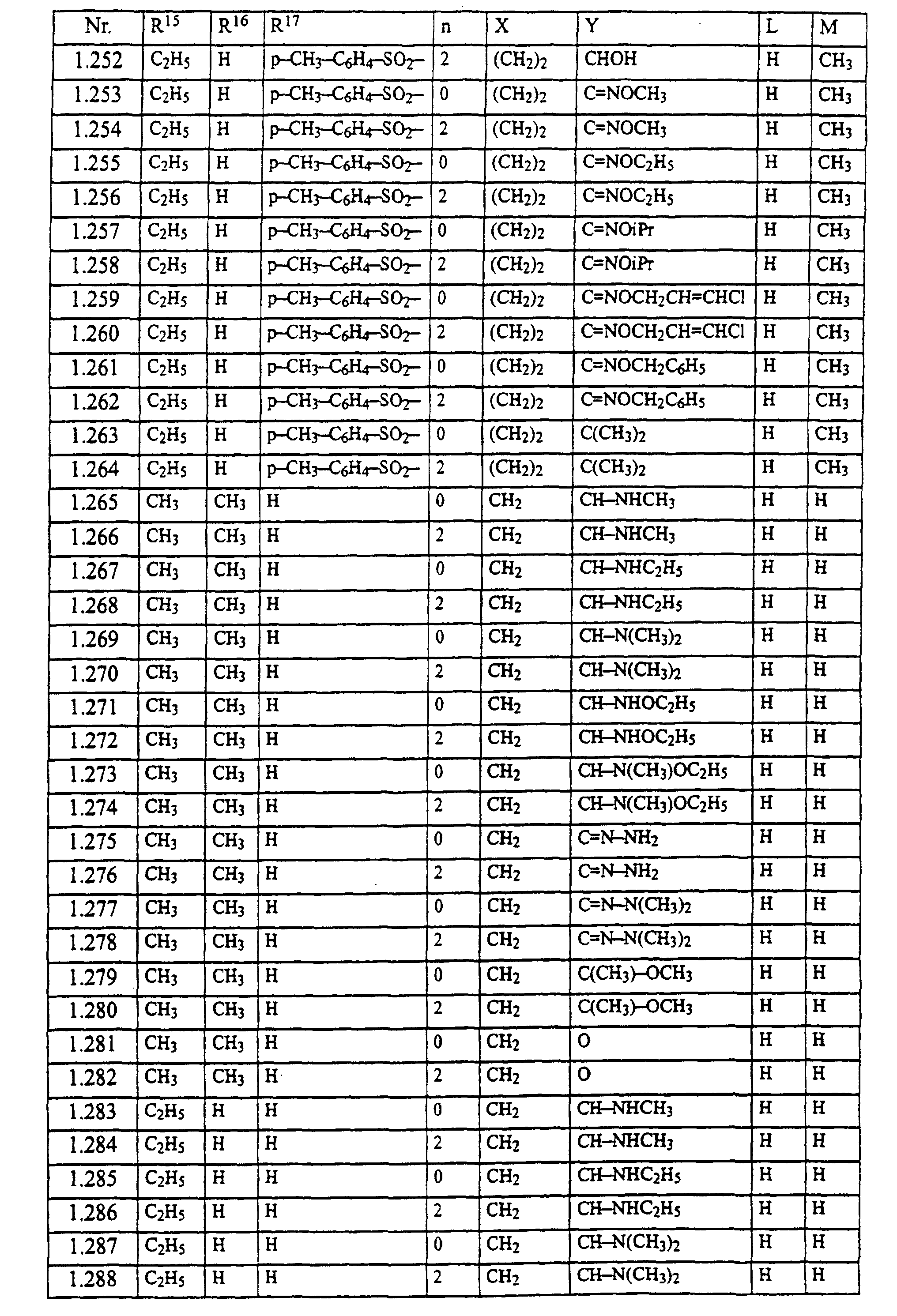

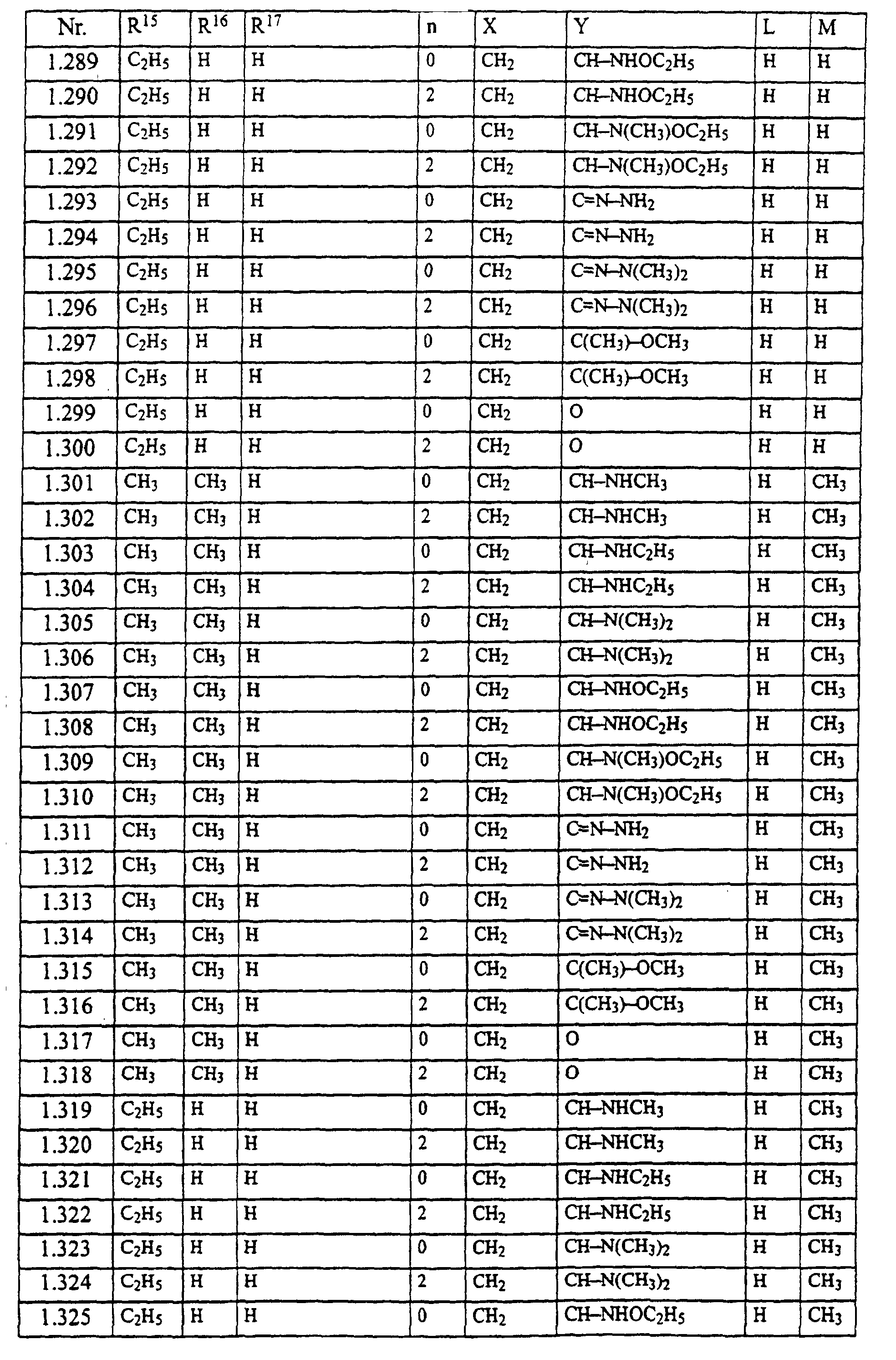

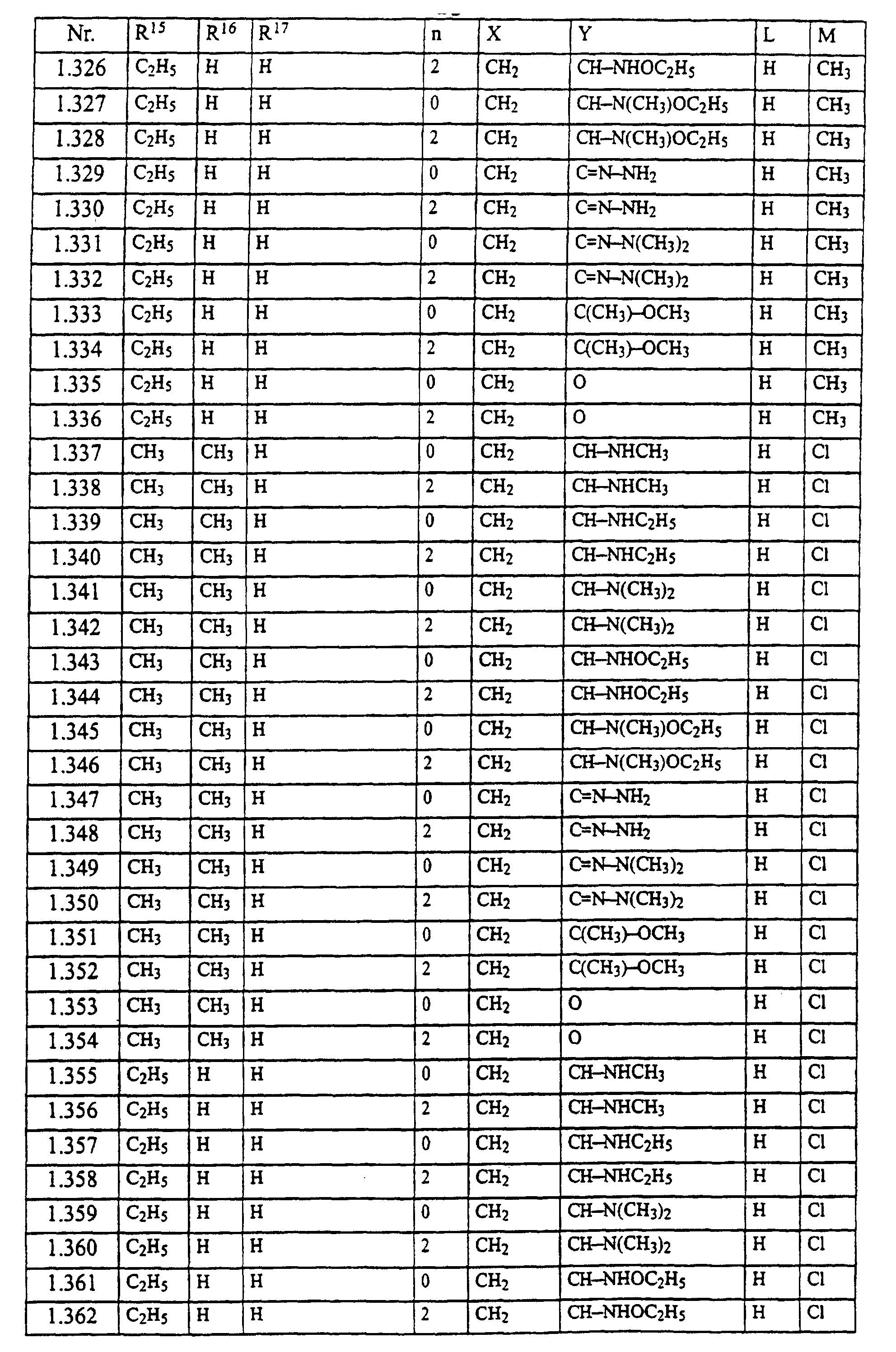

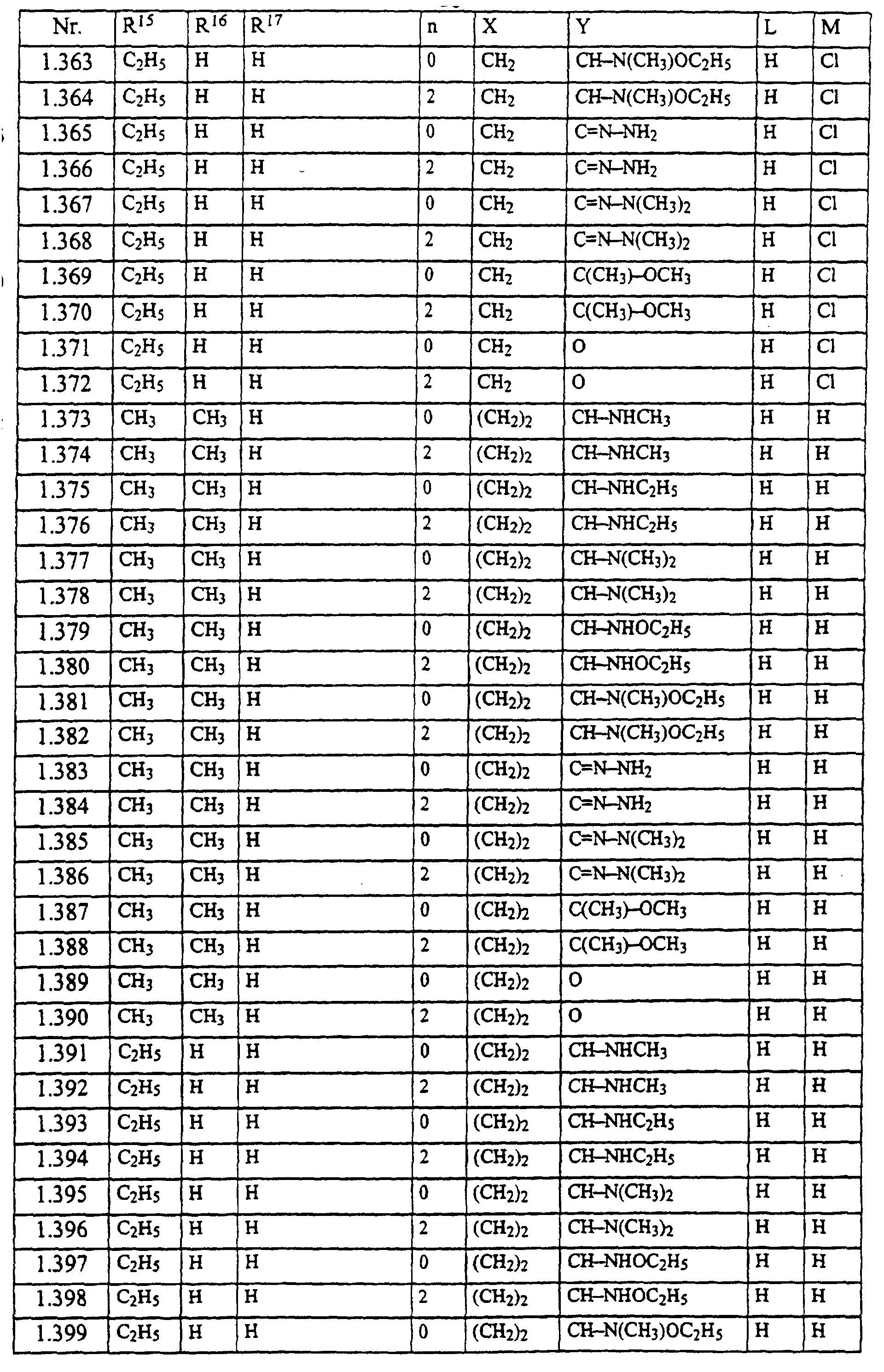

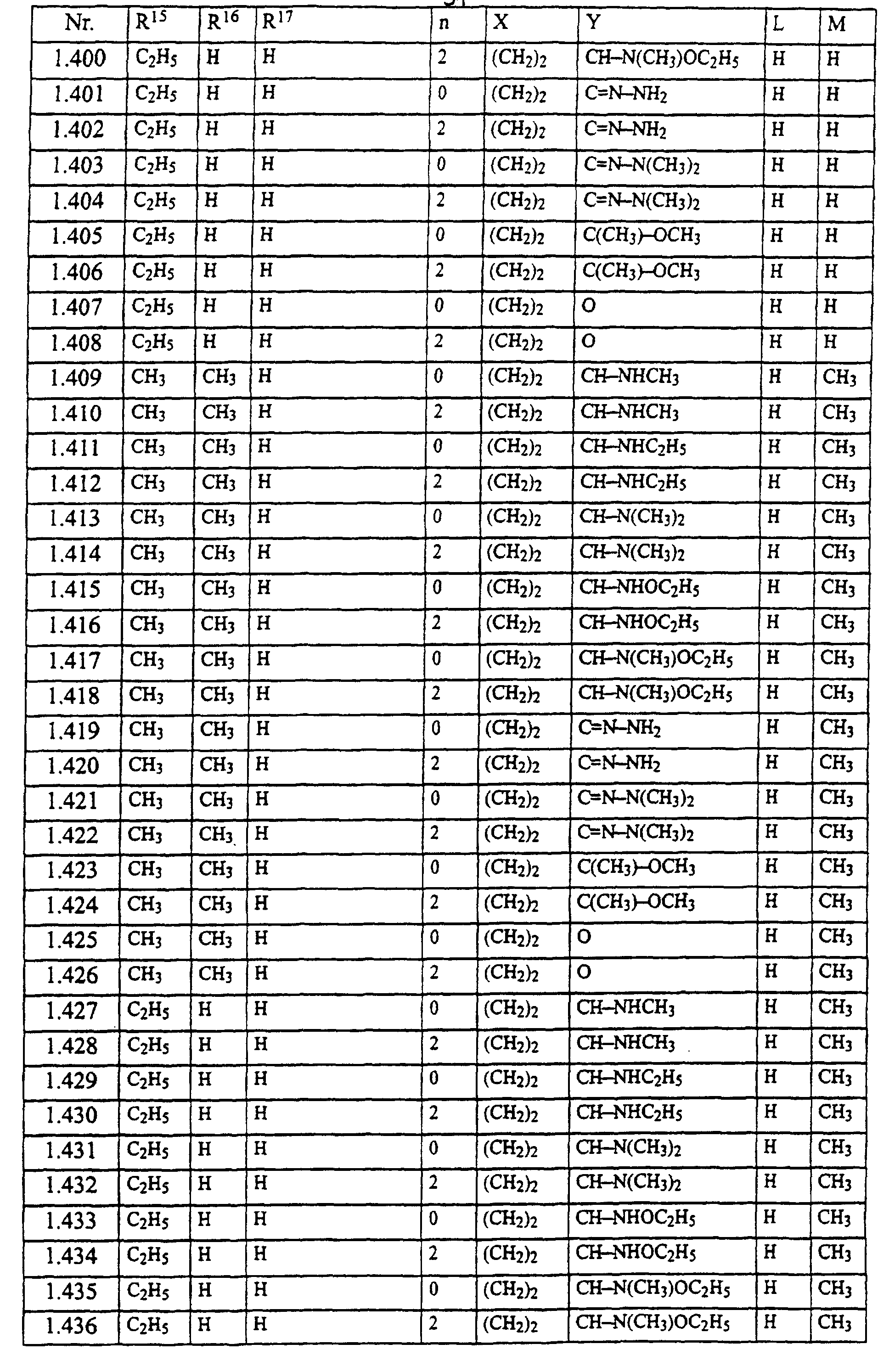

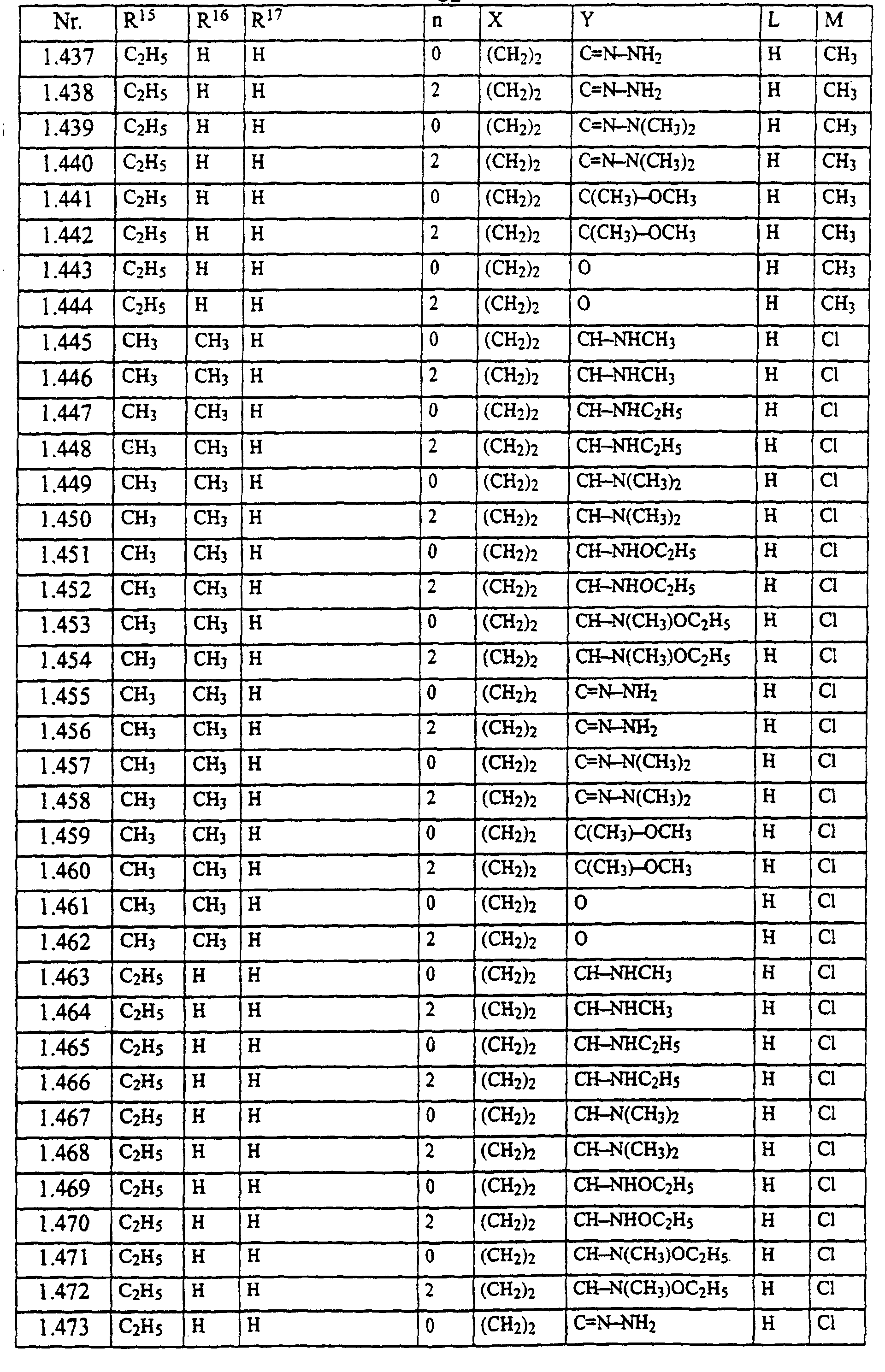

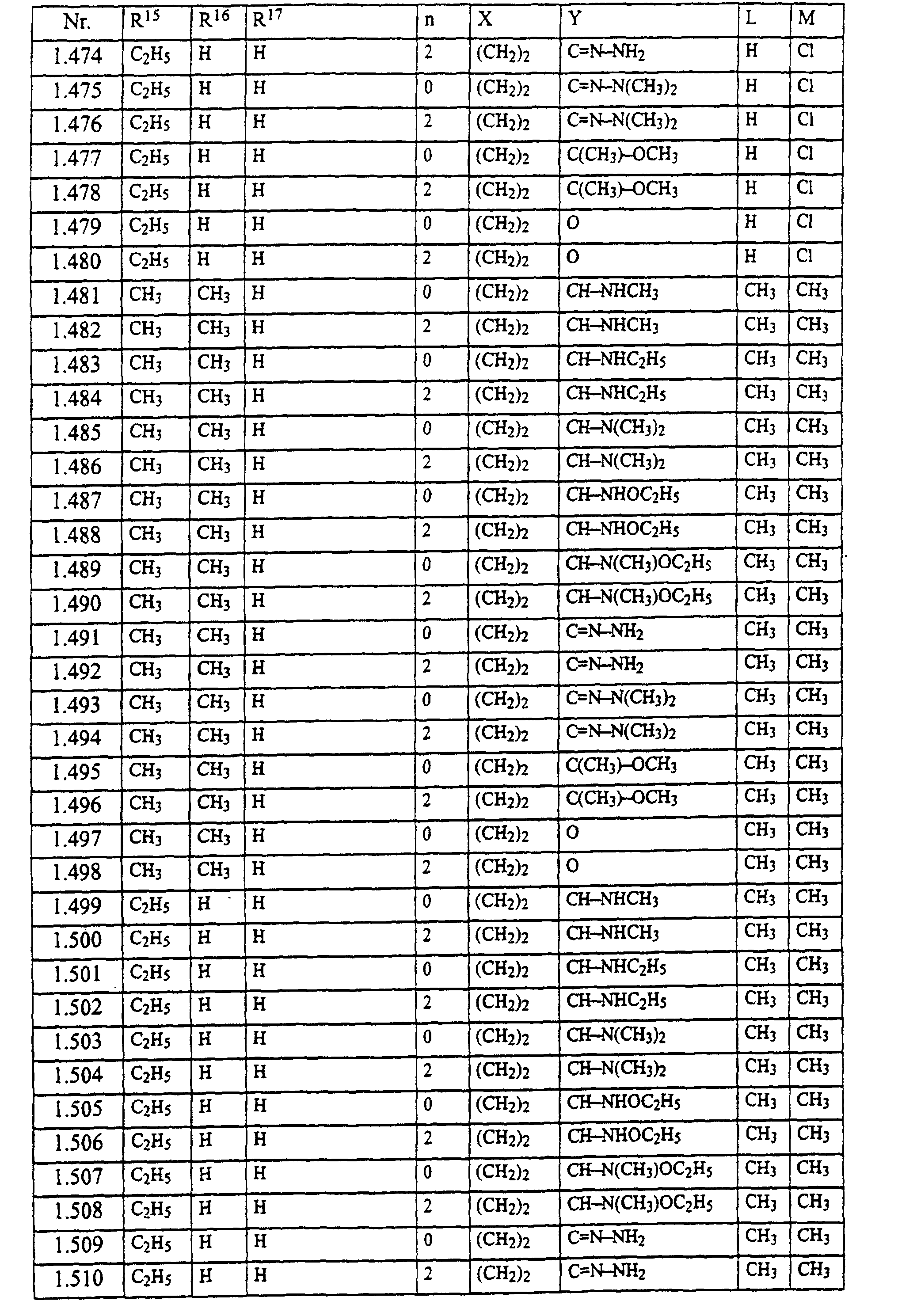

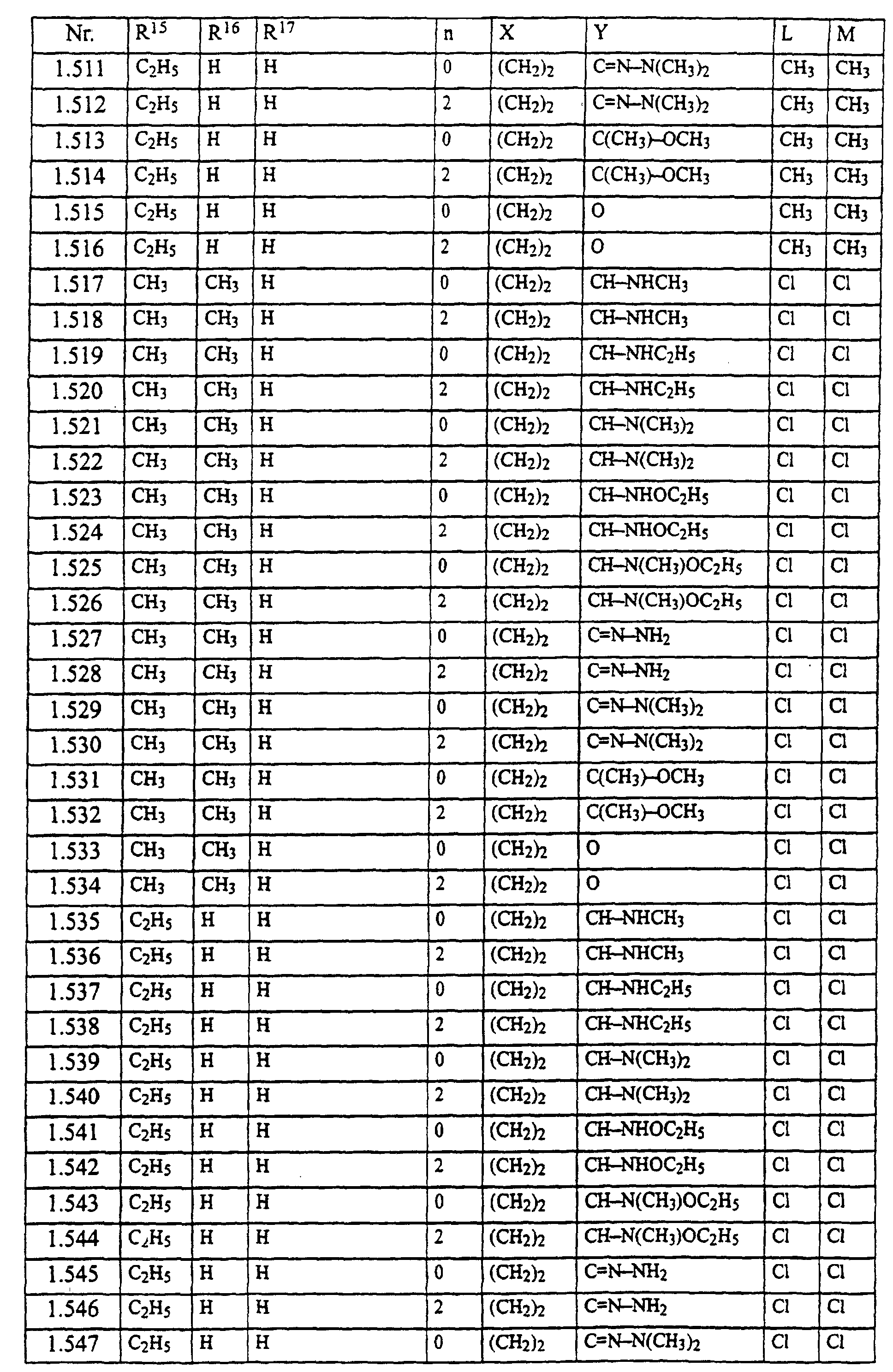

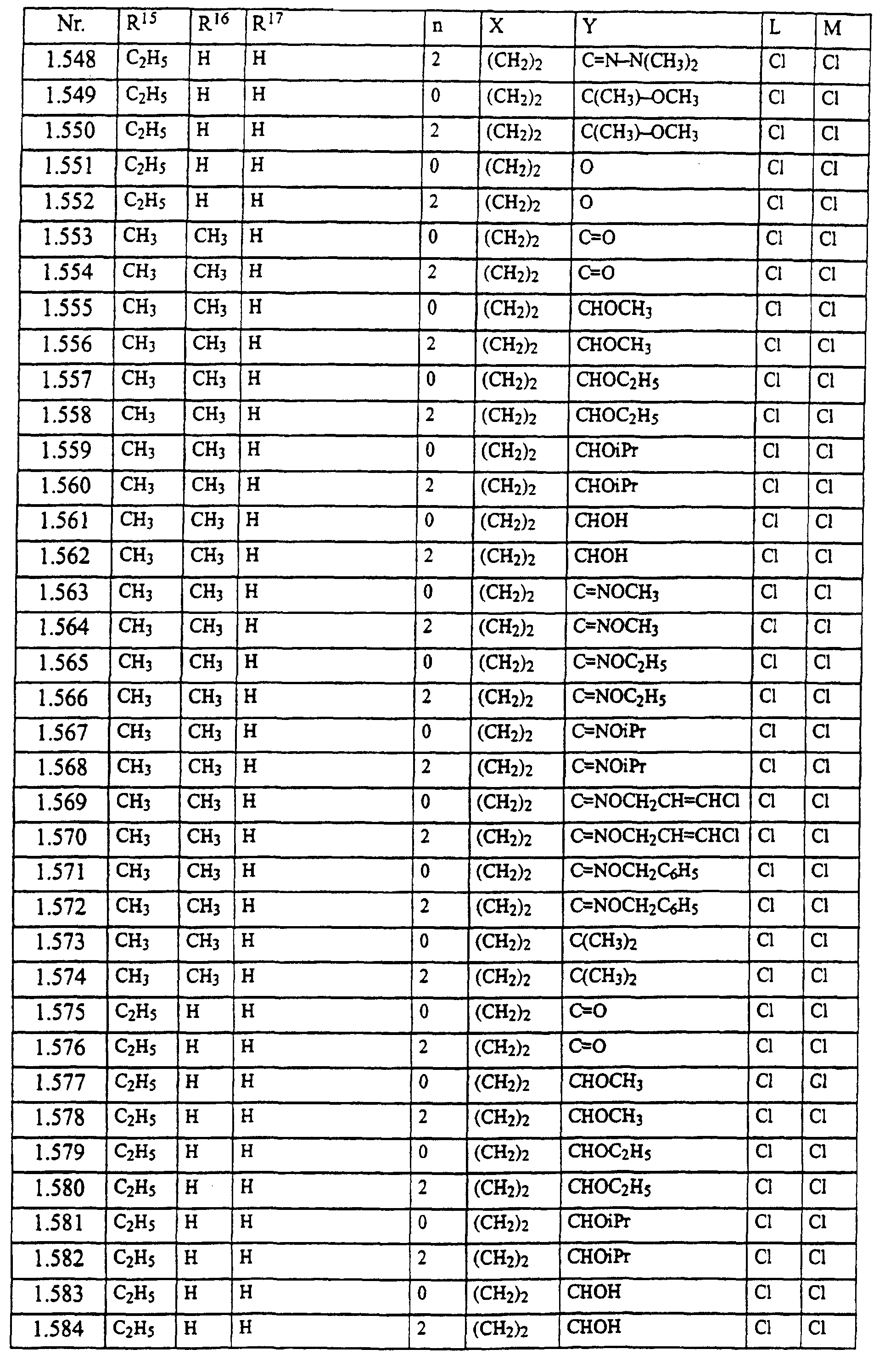

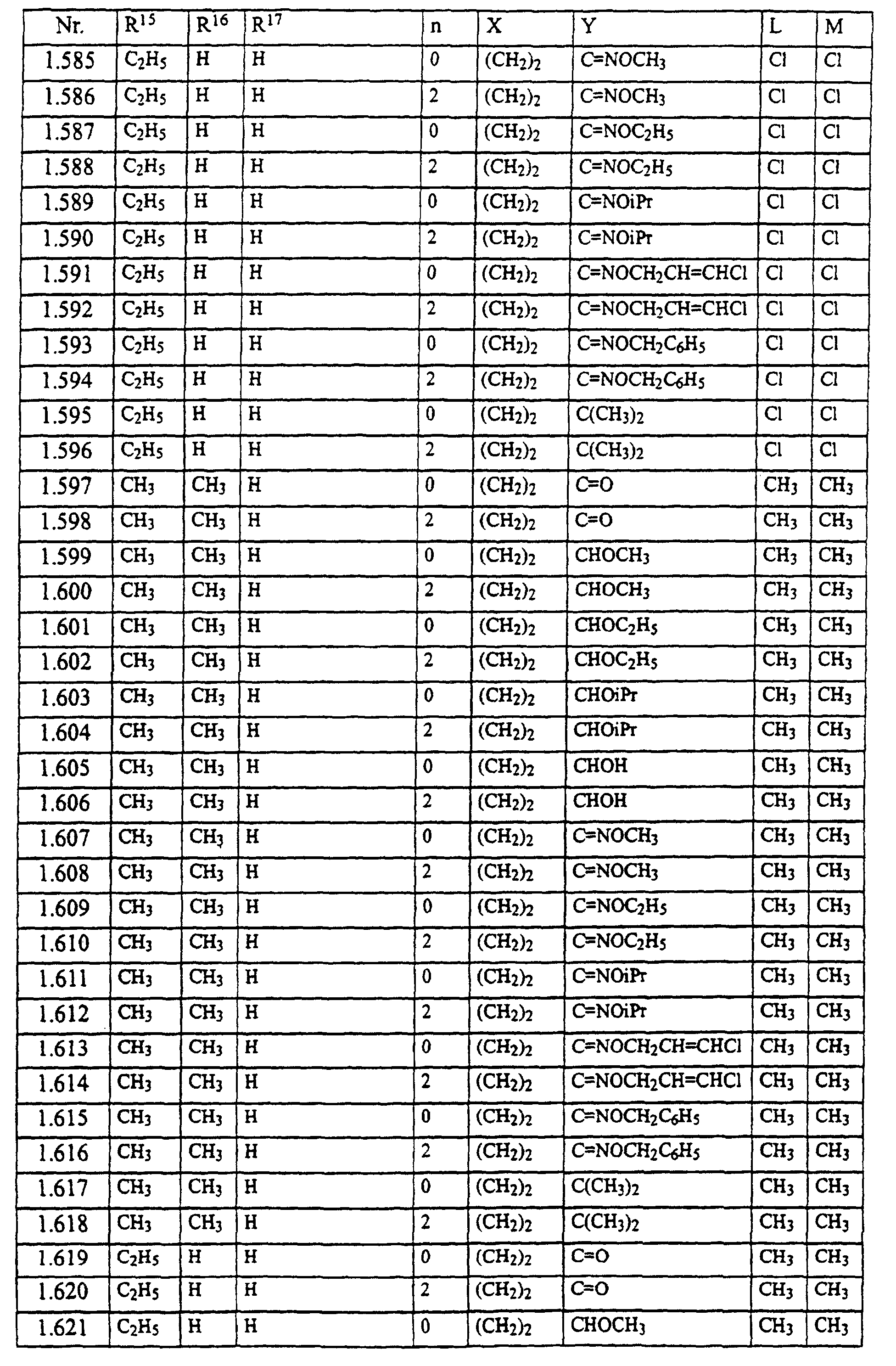

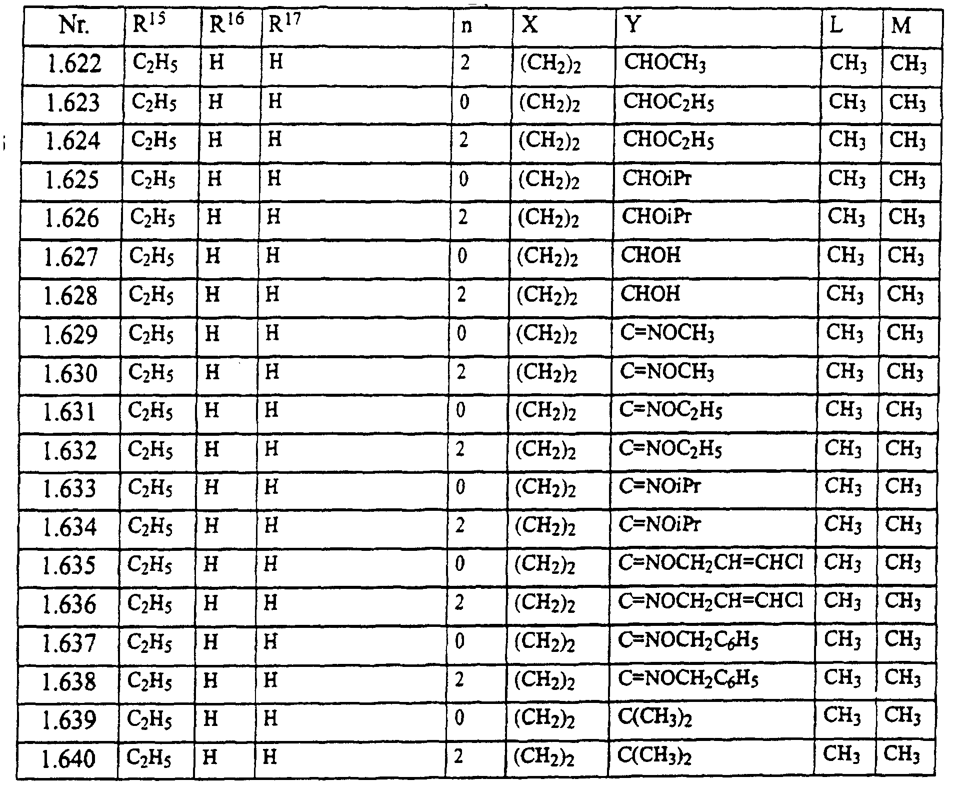

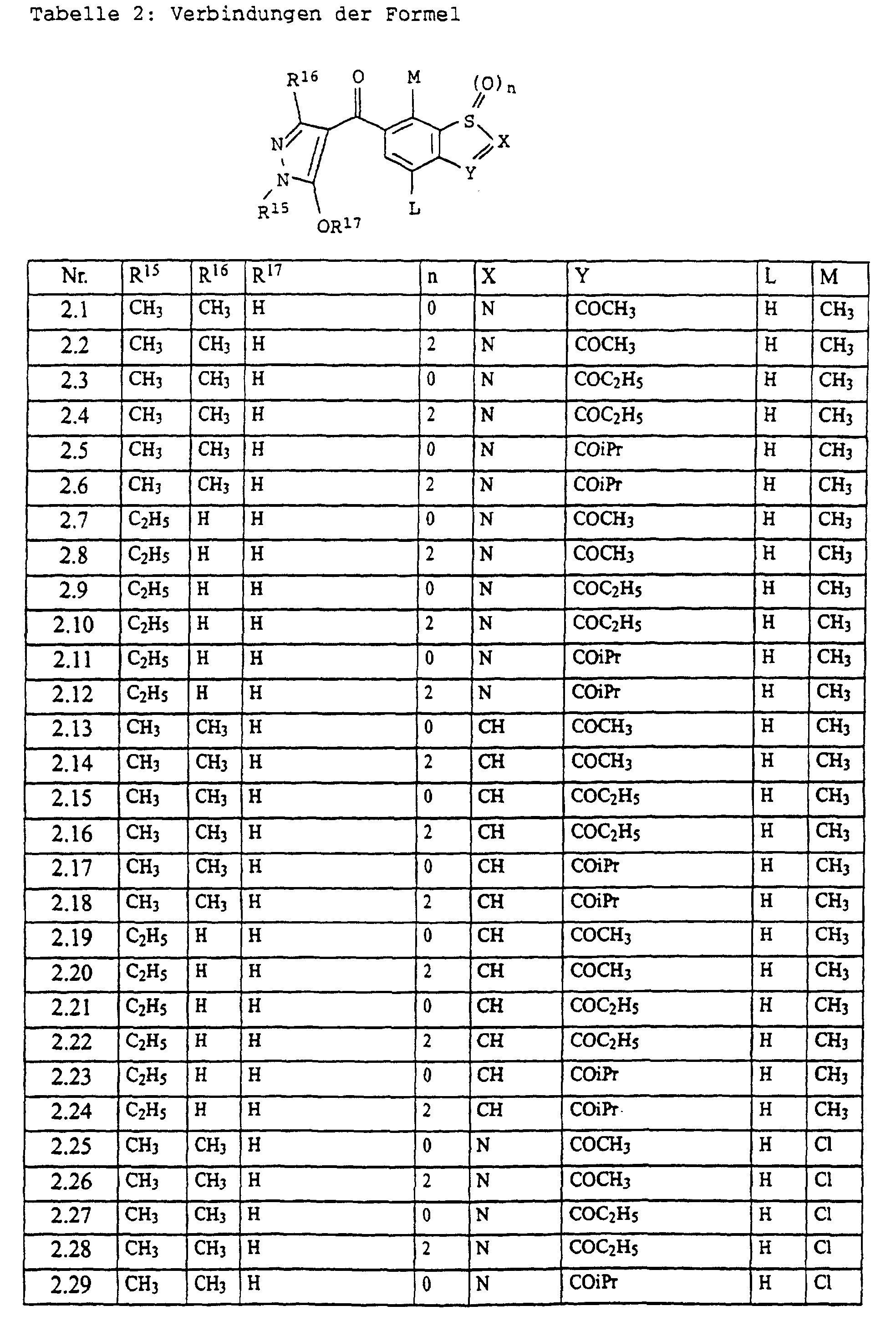

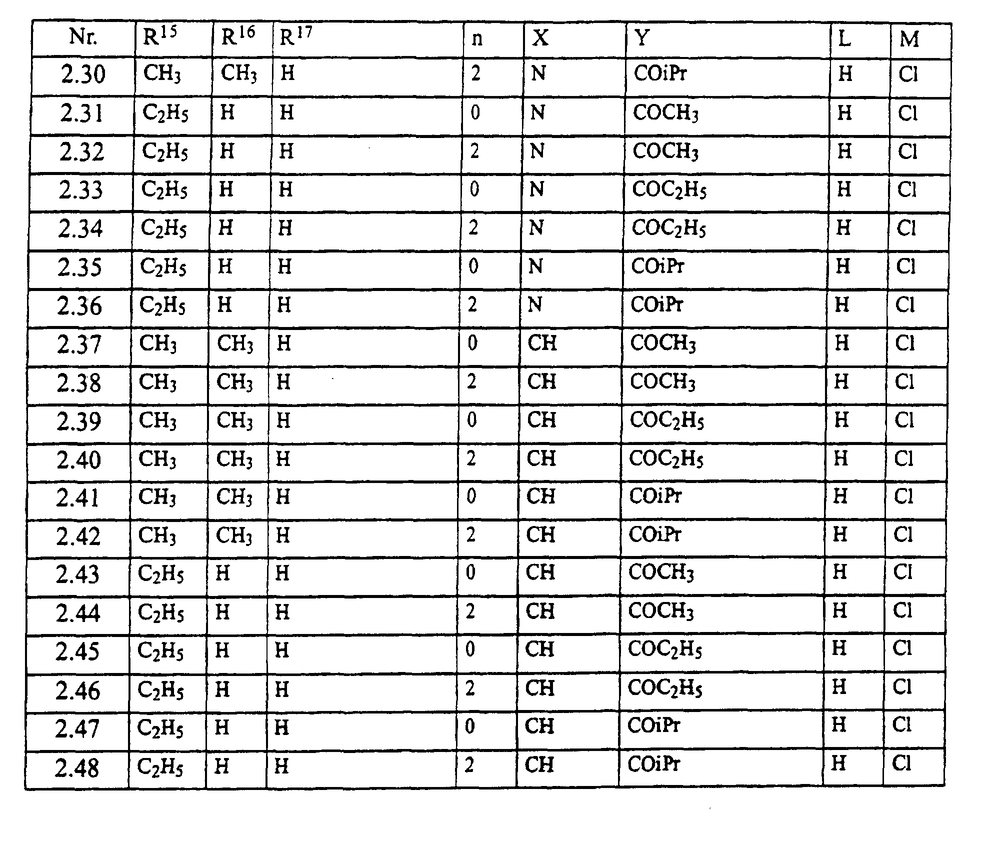

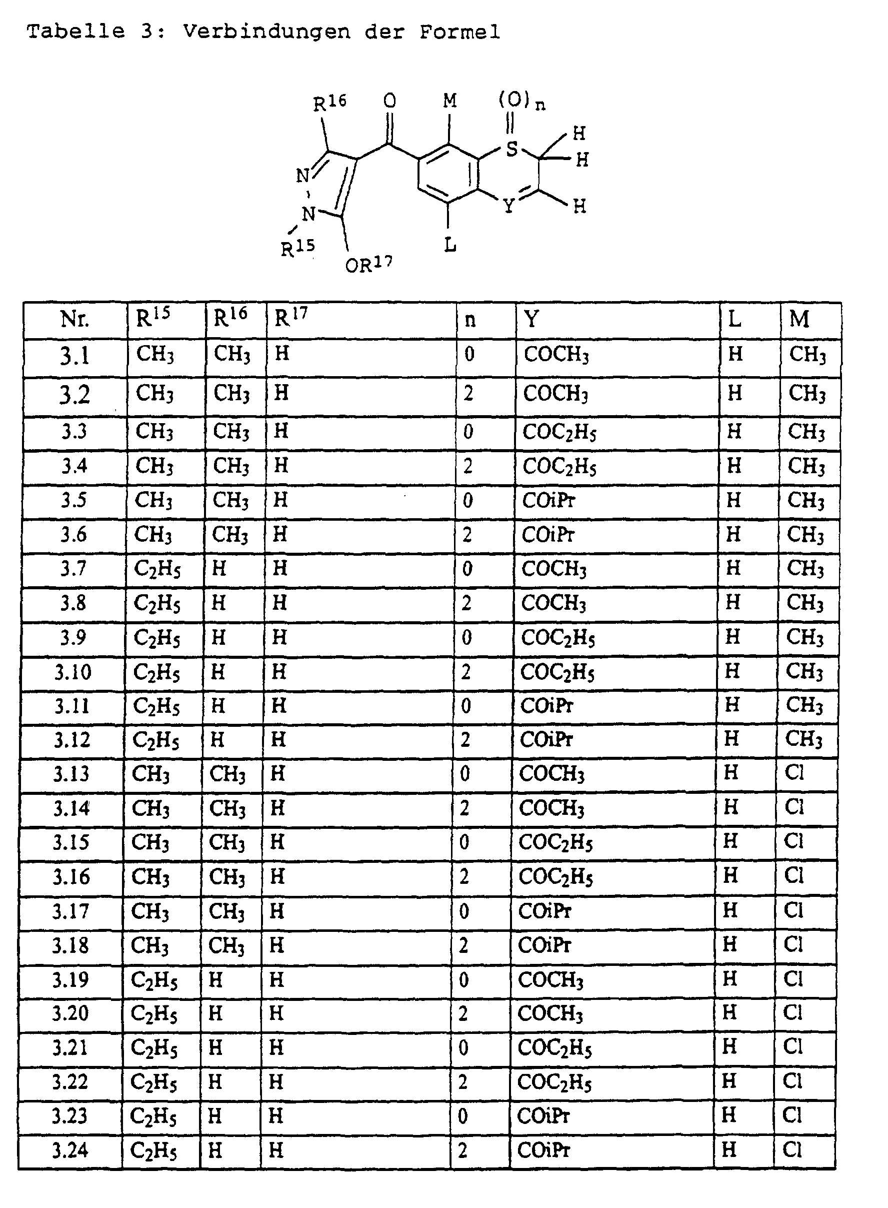

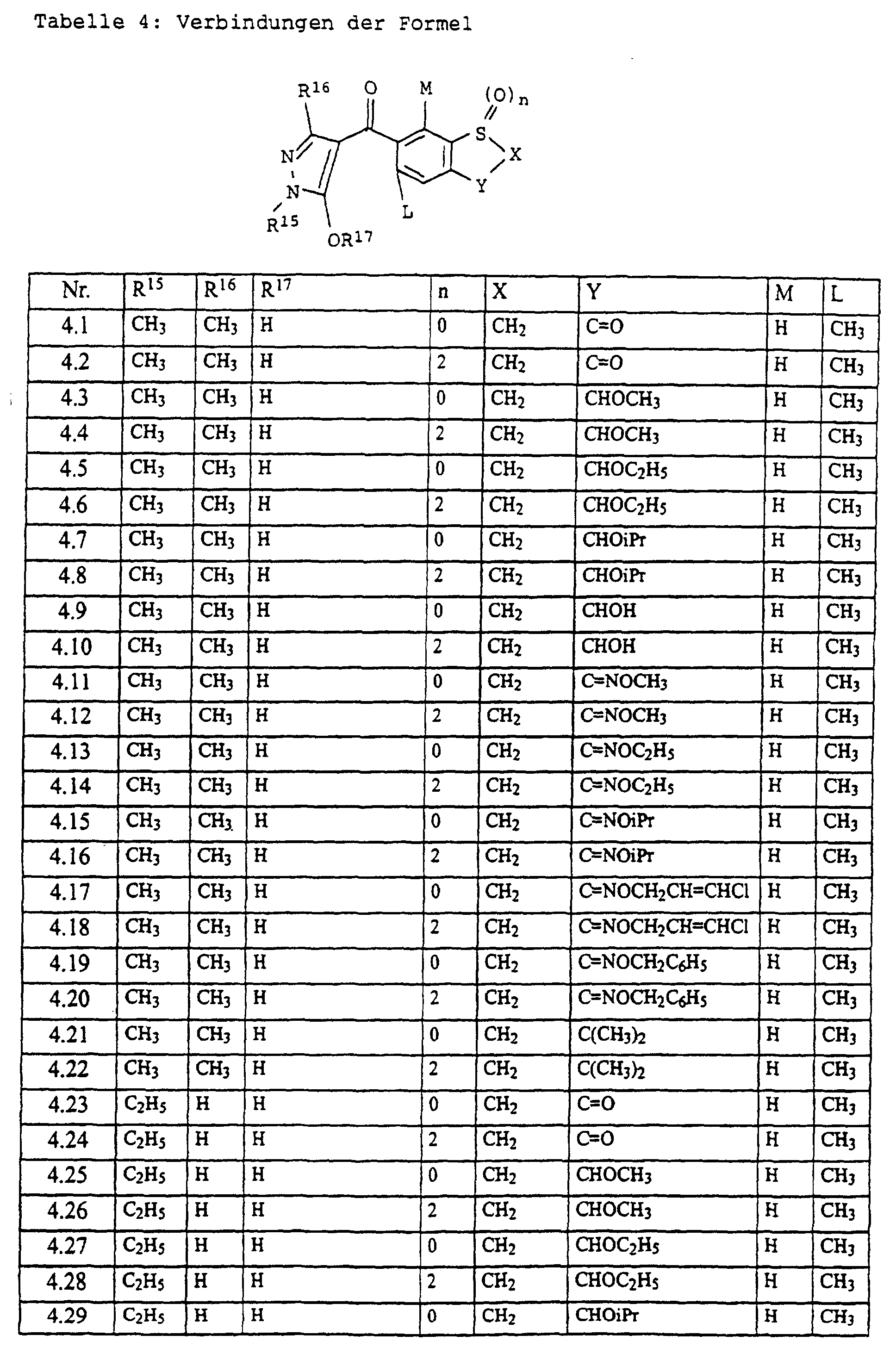

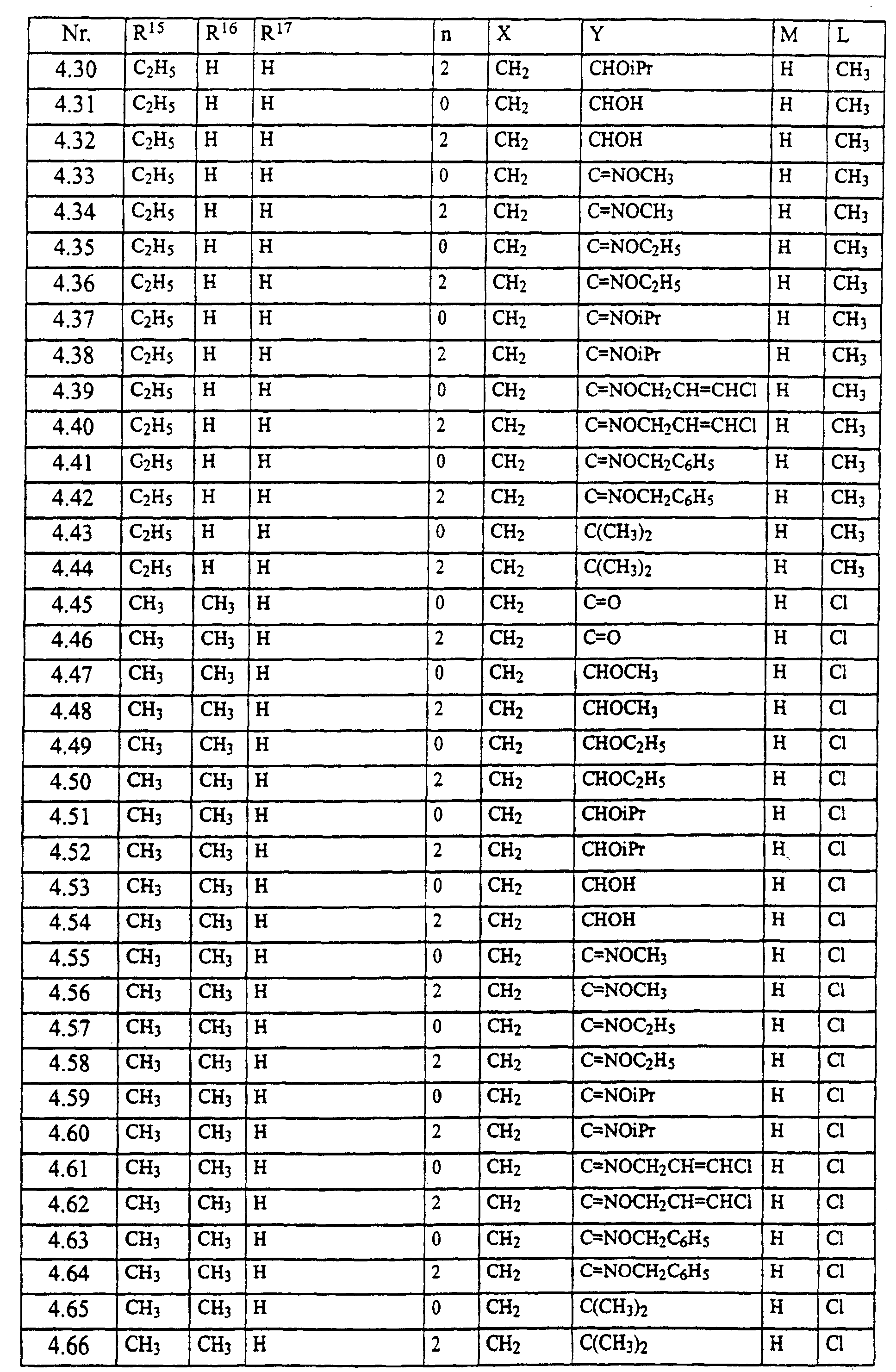

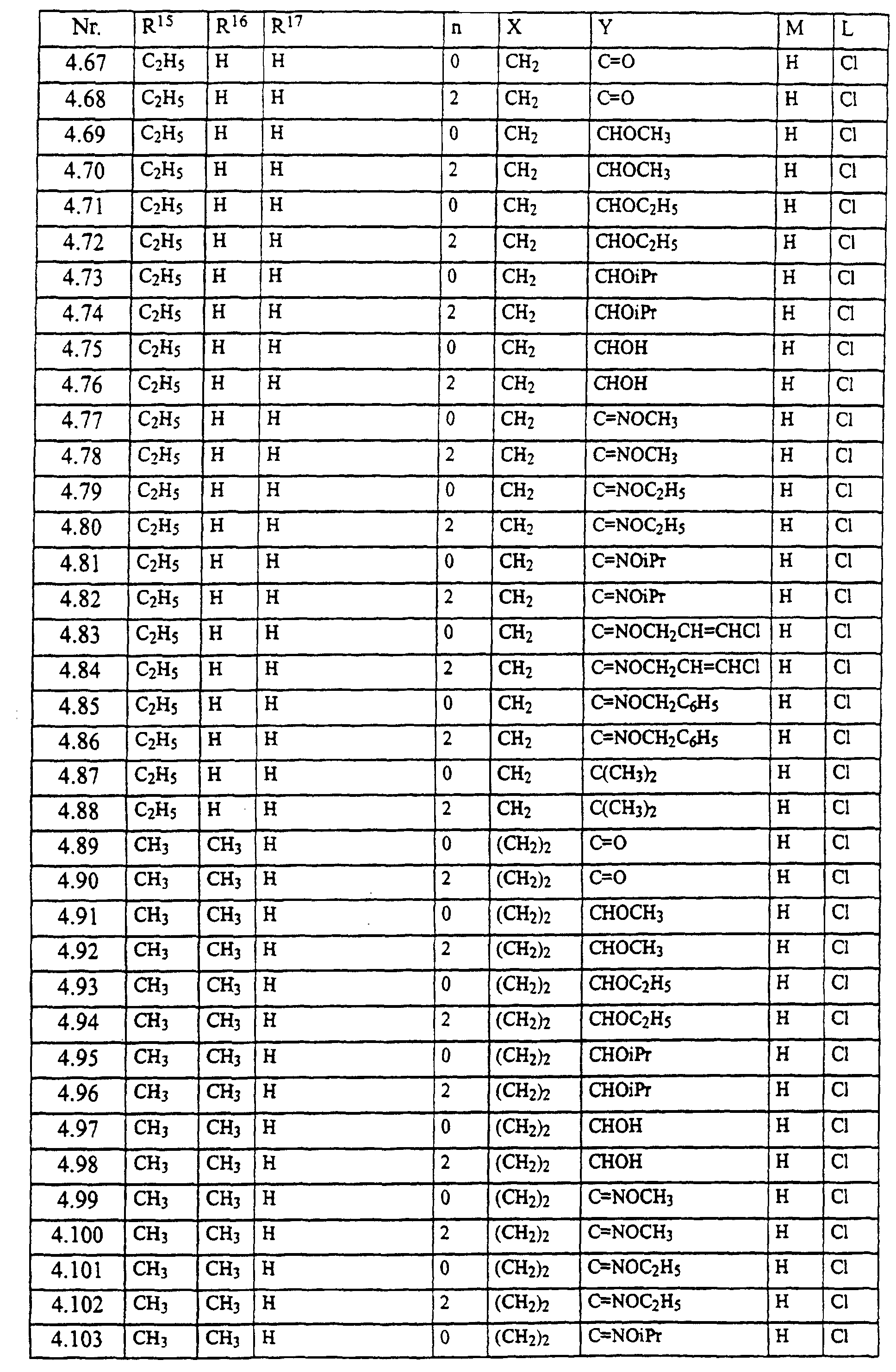

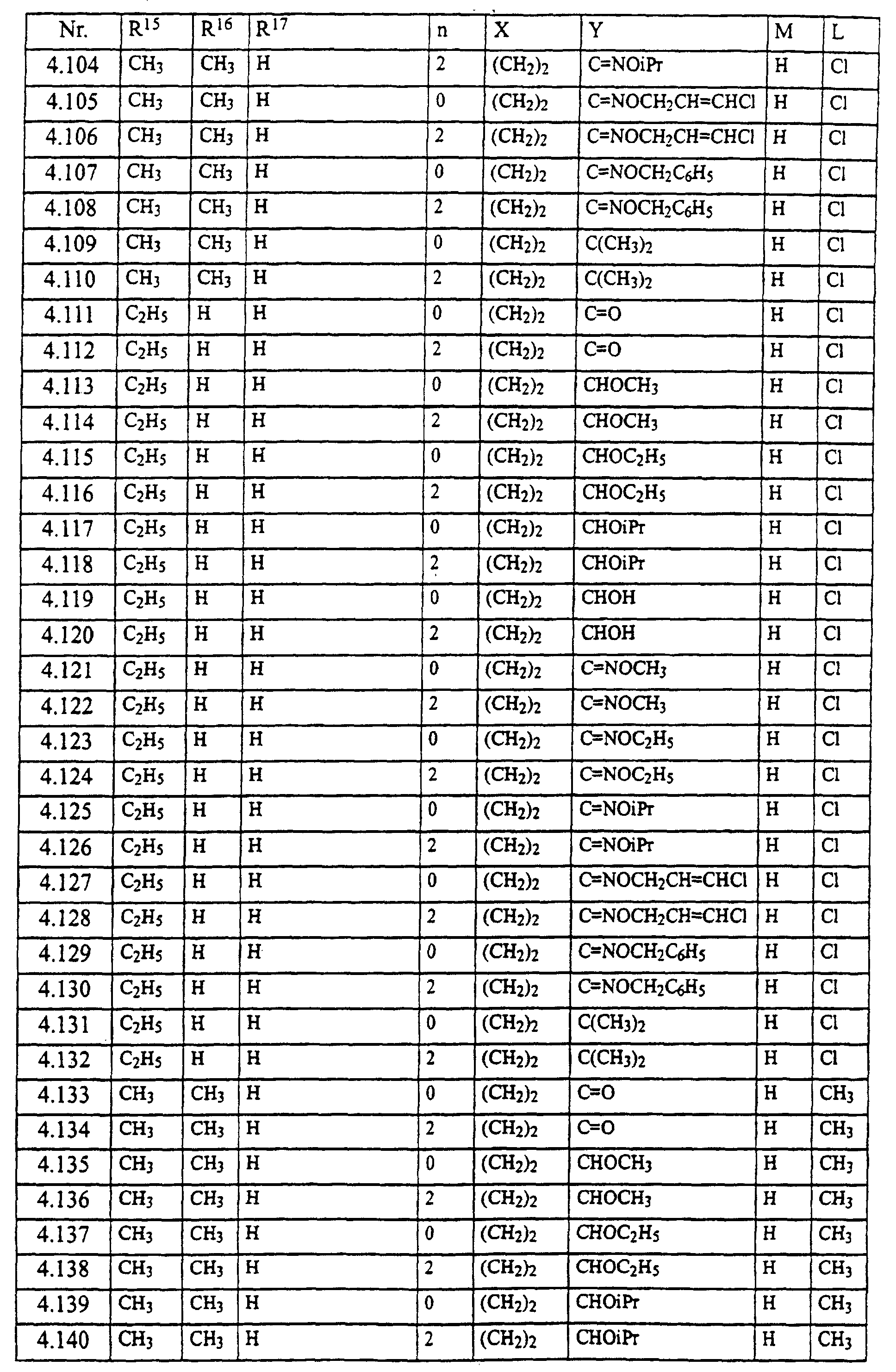

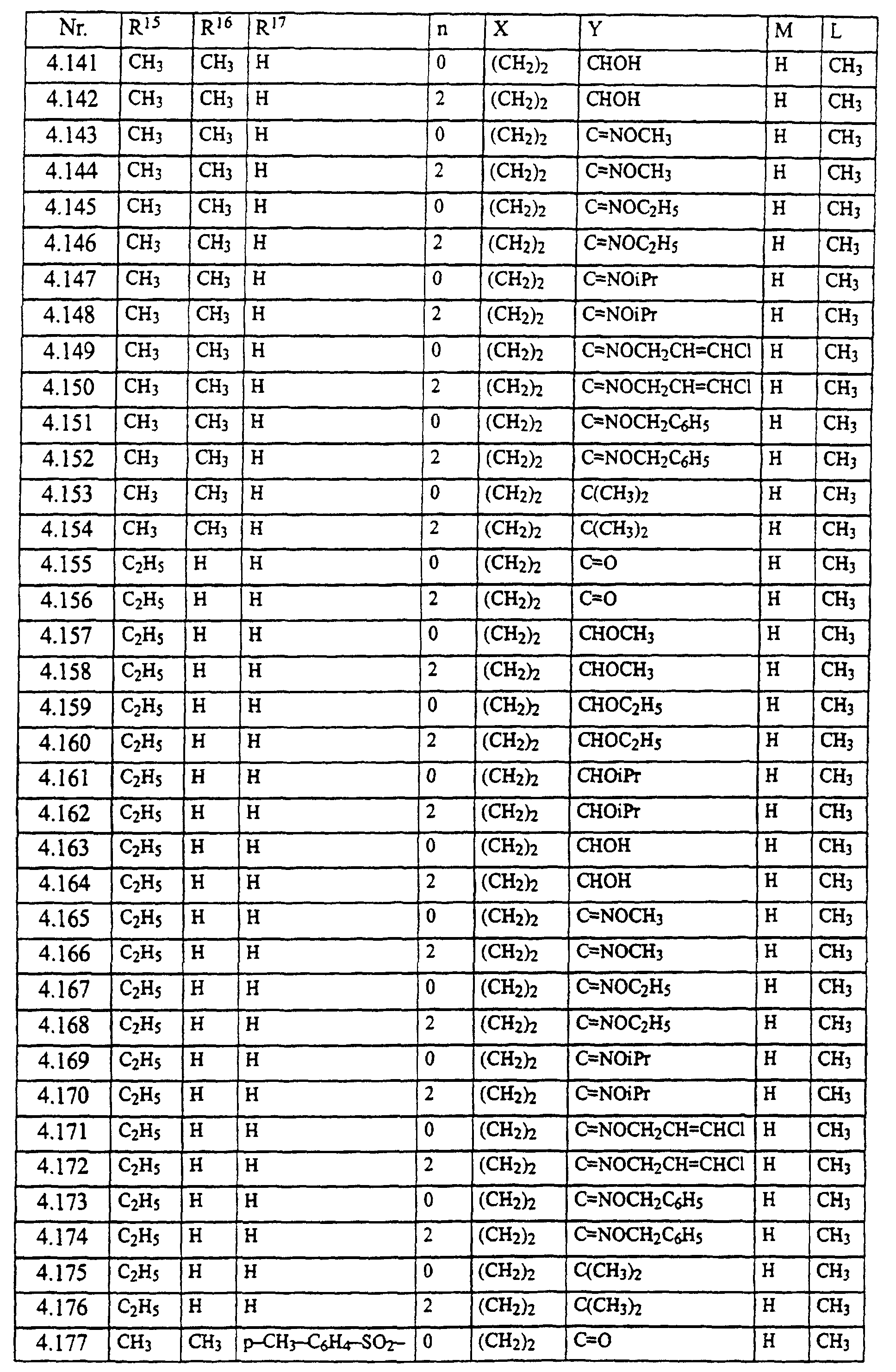

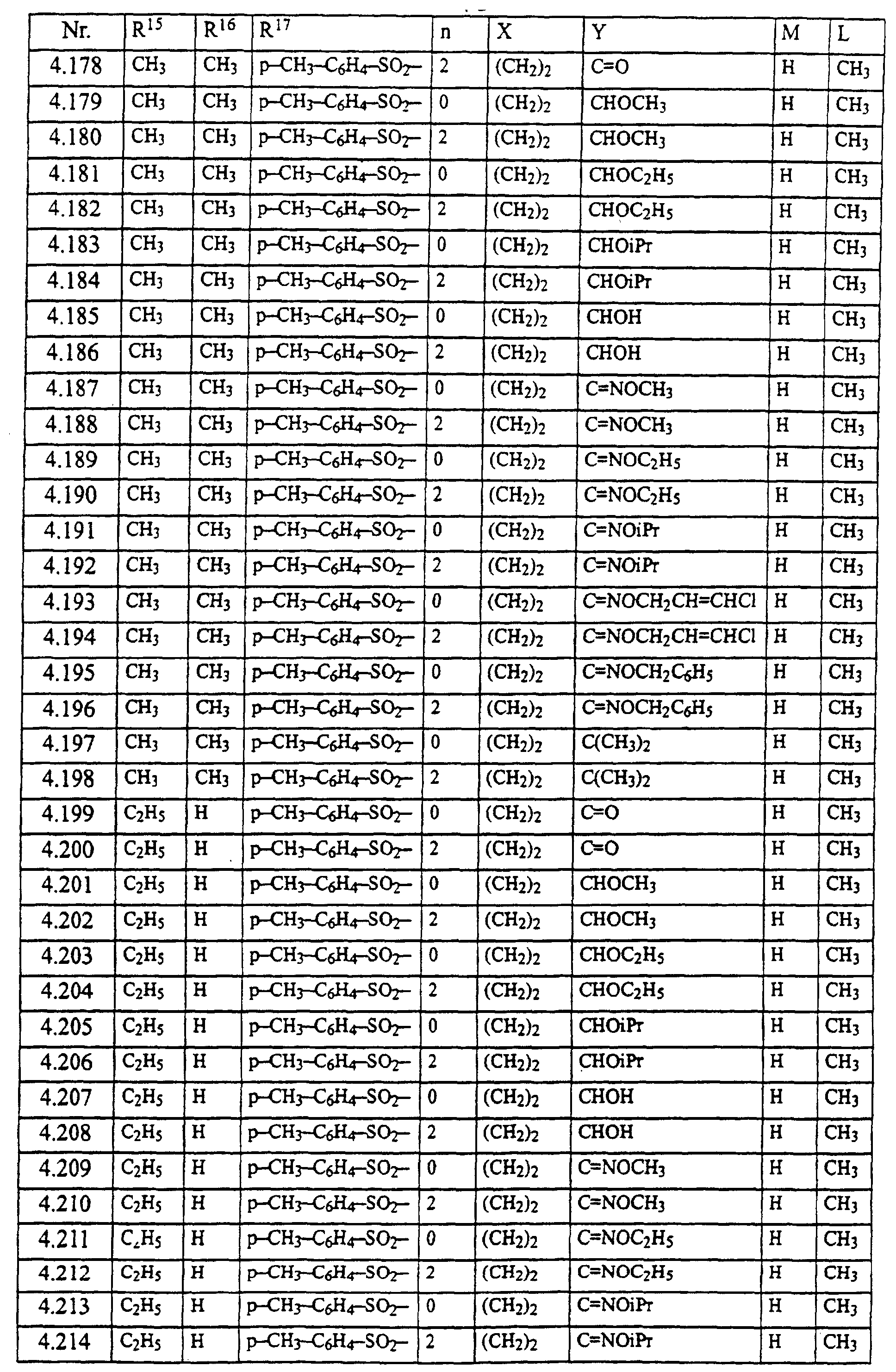

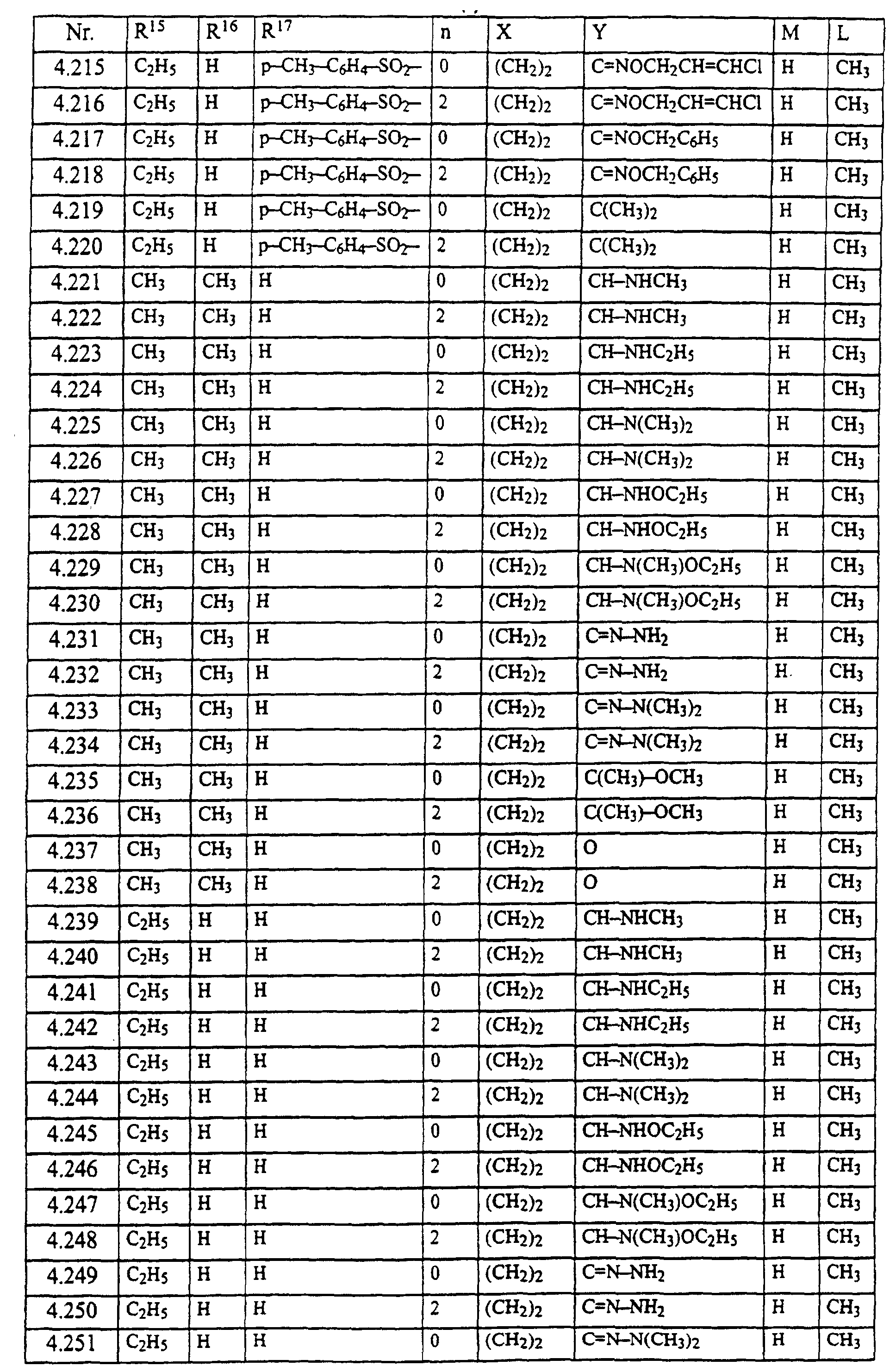

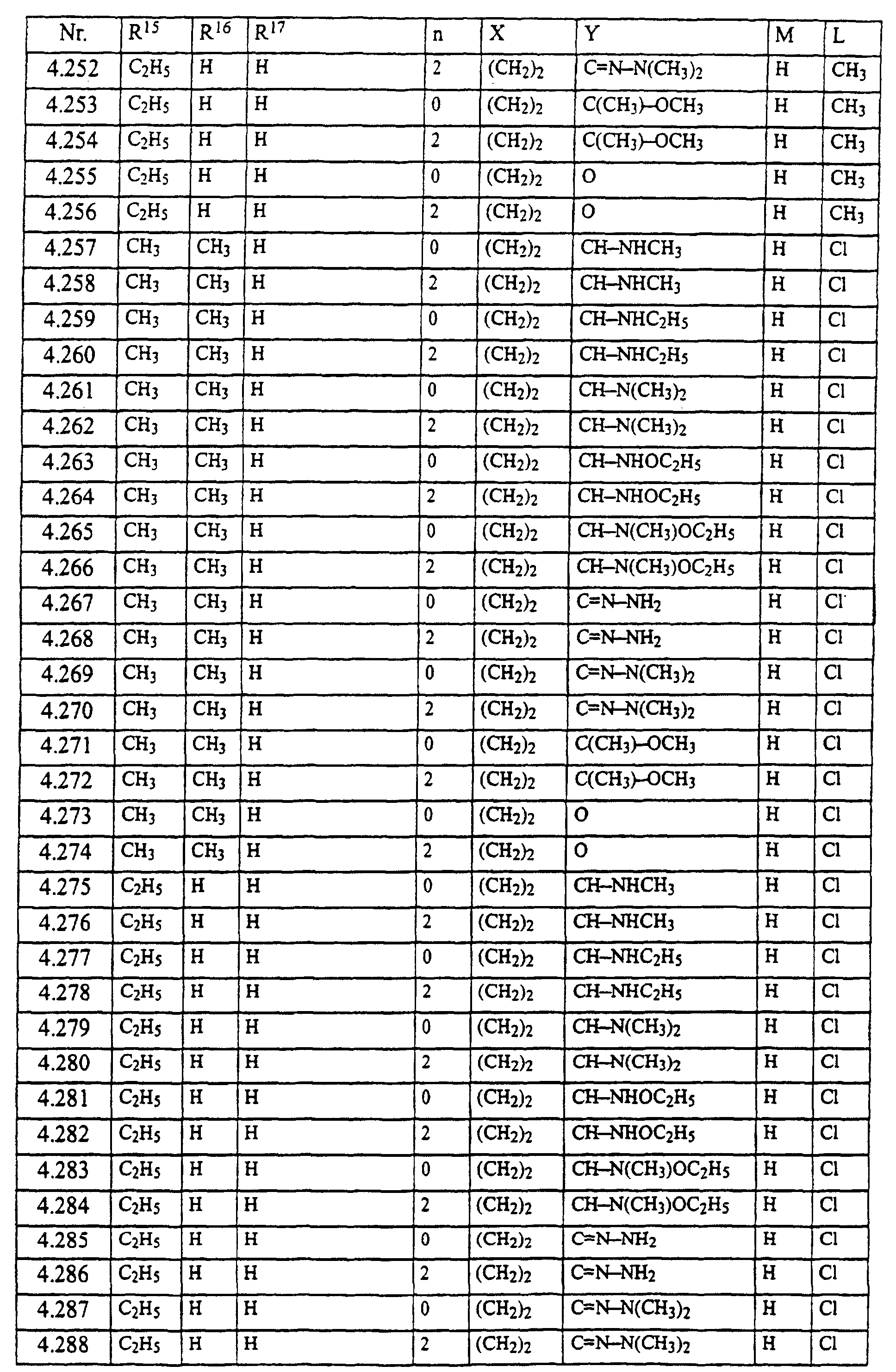

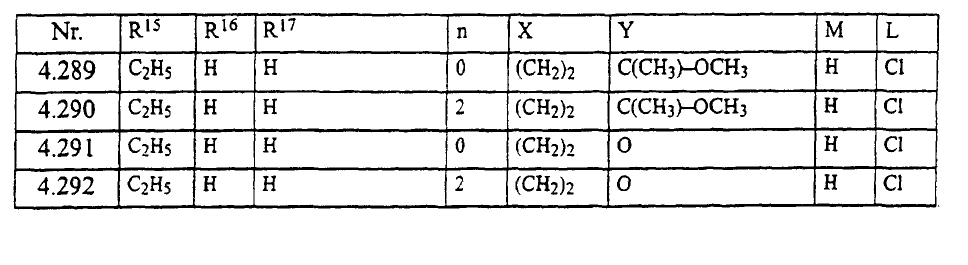

### Herstellungsbeispiele

### A) Herstellung der Ausgangsmaterialien und Zwischenprodukte

1. 3-Thio-2-methylbenzoesäure
   100 g (0.66 mol) 3-Amino-2-methylbenzoesäure werden zusammen mit 270 g Eis und 127 ml konz. HCl vorgelegt. Bei O-10°C werden dann 45.7 g (0.66 mol) Natriumnitrit in 270 ml Wasser zugetropft.
   In einem zweiten Gefäß werden 84.2 g (0.79 mol) Natriumcarbonat und 106 g (0.66 mol) Kaliummethylxanthogenat in 450 ml Wasser gelöst und auf 60-70°C erhitzt. Die Diazoniumlösung wird vorsichtig zugetropft. Es wird 1 Stunde nachgerührt. Anschließend gibt man 106 g (2.65 mol) Natriumhydroxid in 270 ml Wasser hinzu, rührt weitere 2 Stunden, stellt die Lösung mit Salzsäure sauer und saugt den entstehenden Niederschlag ab. Der Feststoff wird mit Wasser gewaschen und getrocknet.
   Ausbeute: 110 g (100 % d.Th.) an 3-Thio-2-methylbenzoesäure; Schmelzpunkt: 155°C; ¹H-NMR (d⁶-DMSO): δ [ppm] = 13.0 (1H,bs), 7.7 (2H,m), 7.3 (1H,tr), 2.4 (3H,s).
2. 3-Thio-2-methylbenzoesäuremethylester
   110 g (0.66 mol) 3-Thio-2-methylbenzoesäure werden in 1.6 1 Methanol, der 5 % Schwefelsäure enthält, gelöst und 5 Stunden unter Rückfluß erhitzt. Anschließend wird der Alkohol abdestilliert, der Rückstand wird in Essigsäureethylester aufgenommen, die organische Phase mit Wasser und mit Natriumcarbonatlösung gewaschen, mit Natriumsulfat getrocknet und einrotiert.
   Ausbeute: 104 g (87 % d.Th.) an 3-Thio-2-methylbenzoesäure-methylester; ¹H-NMR (CDCl₃) : δ [ppm] = 7.6 (1H,d), 7.4 (1H, d) , 7.1 (1H,d), 3.9 (3H,s), 3.4 (1H,s), 2.5 (3H,s).
3. 3-Thio(2'-propionsäure)-2-methylbenzoesäuremethylester 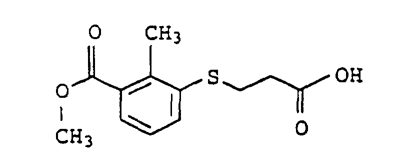
   70 g (0.38 mol) 3-Thio-2-methylbenzoesäuremethylester werden in 400 ml Wasser gelöst und mit 30.8 g (0.77 mol) Natronlauge und 58.8 g (0.45 mol) Brompropionsäure 7 Stunden unter Rückfluß erhitzt. Nach dem Abkühlen wird die wäßrige Phase mit Methyl-tert.-butyl-Ether gewaschen. Anschließend säuert man die wäßrige Phase mit 2N HCl an, saugt den entstandenen Niederschlag ab, wäscht ihn mit Wasser und trocknet das Produkt.
   Ausbeute: 75.5 g (78 % d.Th.) an 3-Thiopropionsäure-2-methylbenzoesäuremethylester; ¹H-NMR (CDCl₃) : δ [ppm] = 7.66 (1H,d), 7.51 (1H,d), 7.20 (1H.tr), 3.96 (3H,s), 3.18 (2H,tr), 2.70 (2H,tr), 2.63 (3H,s).
4. 8-Methylthiochroman-4-on-7-carbonsäuremethylester 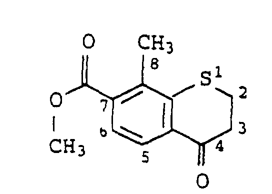
   4 g (15.8 mmol) 3-Thiopropionsäure-2-methylbenzoesäuremethylester werden in 40 g Polyphosphorsäure bei 70°C 15 Minuten gerührt. Danach gibt man die Reaktionslösung auf Eiswasser und saugt den entstandenen Niederschlag ab. Das Produkt wird mit Wasser gewaschen und im Trockenschrank getrocknet. Als Nebenprodukt der Cyclisierung kann 8-Methylthiochromen-4-on-carbonsäuremethylester entstehen, der durch Chromatographie abgetrennt werden kann.
   Ausbeute: 3.1 g (83 % d.Th.) an 8-Methylthiochroman-4-on-7-carbonsäuremethylester; ¹H-NMR (CDCl₃) : δ [ppm] = 8.00 (1H,d), 7.30 (1H,d), 3.94 (3H,s), 3.15 (2H,m), 2.98 (2H,m), 2.50 (3H,s);
   Nebenkomponente 8-Methylthiochromen-4-on-carbonsäuremethylester: ¹H-NMR (CDCl₃): δ [ppm] = 8.4 (1H,d), 7.9 (1H,d), 7.8 (1H,d), 7.0 (1H,d), 4.0 (3H,s), 2.7 (3H,s).
5. 8-Methylthiochroman-4-on-7-carbonsäure
   41.1 g (0.17 mol) 8-Methylthiochroman-4-on-7-carbonsäure-methylester werden in einer Mischung aus 400ml Wasser und Methanol mit 10.3 g (0.26 mol) NaOH bei Rückflußtemperatur hydrolisiert. Anschließend wird das Methanol abdestilliert und der Rückstand mit Wasser verdünnt und mit 2N Salzsäure angesäuert. Das Wertprodukt fällt aus und wird abgesaugt, mit Wasser gewaschen und getrocknet.
   Ausbeute: 34.4 g (89 % d.Th.) an 8-Methylthiochroman-4-on-7-carbonsäure; Schmelzpunkt: 243-246°C.
6. 8-Methyl-1,1-dioxothiochroman-4-on-7-carbonsäure 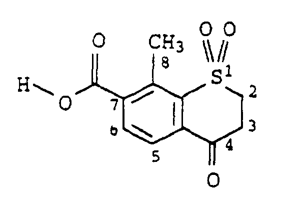
   20 g (0.09 mol) 8-Methylthiochroman-4-on-7-carbonsäure werden in 100 ml Essigsäure gelöst. Man gibt eine Spatelspitze Natriumwolframat hinzu. Bei 50°C werden dann 24.9 g (0.22 mol) 30%ige Wasserstoffperoxidlösung zugetropft. Es wird eine Stunde bei RT nachgerührt. Die Reaktionslösung wird danach auf Wasser gegeben, wobei ein Niederschlag entsteht der abgesaugt wird. Nach dem Waschen des Produktes mit Wasser wird dieses getrocknet.
   Ausbeute: 18.4 g (80 % d.Th.) 8-Methyl-1,1-dioxothiochroman-4-on-7-carbonsäure; Schmelzpunkt: 224-225°C.
7. 4-Hydroxy-8-methylthiochroman-7-carbonsäuremethylester 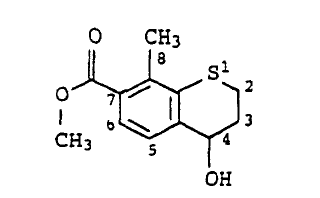
   30 g (0.127 mol) 8-Methylthiochroman-4-on-7-carbonsäure-methylester werden in einer Mischung aus 120 ml Methylenchlorid und 60 ml Methanol gelöst und auf 0-5°C gekühlt. Portionsweise werden dann 2.4 g (0.064 mol) Natriumborhydrid hinzugefügt. Man läßt eine Stunde bei dieser Temperatur nachrühren. Zu der Reaktionslösung werden 200 ml 2N Salzsäure gegeben. Es bilden sich zwei Phasen. Die organische Phase wird abgetrennt getrocknet und das Lösungsmittel abdestilliert.
   Das Rohprodukt wird ohne weitere Reinigung direkt weiter umgesetzt.
   Ausbeute: 27.6 g (91 % d.Th.) an 4-Hydroxy-8-methylthiochroman-7-carbonsäuremethylester.
8. 4-Ethoxy-8-methylthiochroman-7-carbonsäuremethylester
   13.8 g (0.058 mol) 4-Hydroxy-8-methylthiochroman-7-carbonsäuremethylester werden in 60 ml Ethanol unter Zusatz von 1 g Schwefelsäure 4 Stunden unter Siedetemperatur erhitzt. Danach wird das Solvens abdestilliert und der Rückstand mit Wasser aufgenommen. Man extrahiert die wäßrige Phase mit Essigsäureethylester. Die organische Phase wird mit Natriumhydrogencarbonatlösung gewaschen, getrocknet und eingeengt. Das Produkt wird durch Chromatographie gereinigt.
   Ausbeute: 10.1 g (60 % d.Th.) an 4-Ethoxy-8-methylthiochroman-7-carbonsäuremethylester; ¹H-NMR (CDCl₃): δ [ppm] = 7.44 (1H,d), 7.13 (1H,d), 4.40 (1H, m), 3.90 (3H,s), 3.60 (2H,m), 3.38 (1H,dtr), 2.90 (1H,m), 2.50 (3H,s), 2.40 (1H,m), 1.98 (1H,m), 1.10 (3H,tr).
   Die Umsetzung zu 4-Methoxy-8-methylthiochroman-4-on-7-carbonsäuremethylester und 4-Isopropoxy-8-methylthiochroman-4-on-7-carbonsäuremethylester erfolgt analog obiger Vorschrift, wobei im Fall von 4-Methoxy-8-methylthiochroman-4-on-7-carbonsäuremethylester Ethanol durch Methanol und bei 4-Isopropoxy-8-methylthiochroman-4-on-7-carbonsäuremethylester Ethanol durch Isopropanol ersetzt wurde.
9. 4-Ethoxy-8-methylthiochroman-7-carbonsäure
   2.1 g Natronlauge werden in 20 ml Wasser gelöst. Bei ca. 20°C tropft man 4-Ethoxy-8-methylthiochroman-4-on-7-carbonsäuremethylester in 20 ml Methanol gelöst zu. Man erhitzt 2 Stunden unter Rückfluß. Anschließend destilliert man das Lösungsmittel ab und gibt den Rückstand auf 2N Salzsäure. Die wäßrige Phase wird mit Methylenchlorid extrahiert. Die organische Phase wird getrocknet und eingeengt.
   Ausbeute: 9.3 g (100 % d.Th.) an 4-Ethoxy-8-methylthiochroman-7-carbonsäure; Schmelzpunkt: 89-98°C.
   Die Hydrolyse der entsprechenden Ester zu 4-Methoxy-8-methylthiochroman-7-carbonsäure und 4-Isopropoxy-8-methylthiochroman-7-carbonsäure verläuft analog. Gleiches gilt für die Verseifung der entsprechenden unten aufgeführten Benzo[b]thiophenderivate.
10. 8-Methyl-4-ethoxy-1,1-dioxothiochroman-7-carbonsäure
   8.4 g (0.033 mol) 4-Ethoxy-8-methylthiochroman-7-carbonsäure werden in 60 ml Essigsäure vorgelegt. Man gibt eine Spatelspitze Natriumwolframat hinzu. Bei 50°C werden langsam 7.9 g (0.07 mol) 30%ige Wasserstoffperoxidlösung zugetropft. Man rührt 2 Stunden nach. Der Reaktionsansatz wird dann in Wasser gegossen und die wäßrige Phase mit Essigsäureethylester extrahiert. Die organische Phase wird mit Bisulfitlösung gewaschen, dann getrocknet und eingeengt.
   Ausbeute: 9.5 g (100 % d.Th.) an 8-Methyl-4-ethoxy-1,1-dioxothiochroman-7-carbonsäure; Schmelzpunkt: 150°C.
11. 8-Methylthiochroman-4-on-7-carbonsäure-O-ethyloxim 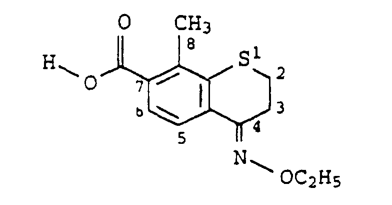
   In 20 ml Methanol werden 0.88 g (9 mmol) Ethylhydroxylamin vorgelegt. Man gibt dann 0.62 g (4.5 mmol) Kaliumcarbonat hinzu. Anschließend werden 2.0 g (9 mmol) 8-Methylthiochroman-4-on-7-carbonsäure zugegeben. Die Reaktion wird 10 Tage bei ca. 20°C gerührt. Aufgearbeitet wird durch Zugabe von Wasser und 2N HCl. Der entstehende Niederschlag wird abgesaugt und getrocknet.
   Ausbeute: 2.2 g (92 % d.Th.) an 8-Methylthiochroman-4-on-7-carbonsäure-O-ethyloxim; Schmelzpunkt: 166°C.
12. 8-Methyl-1,1-dioxothiochroman-4-on-7-carbonsäure-O-ethyloxim
   3.0 g (0.011 mol) 8-Methylthiochroman-4-on-7-carbonsäure-O-ethyloxim werden zusammen mit einer Spatelspitze Natriumwolframat in 30ml Essigsäure vorgelegt. Bei 50°C tropft man 2.8 g (0.024 mol) 30%ige Wasserstoffperoxidlösung zu. Nach einstündigem Rühren wird das Reaktionsgemisch in Eiswasser gegossen, der entstehende Niederschlag wird abgesaugt. Das Produkt wird mit Wasser gewaschen und getrocknet.
   Ausbeute: 2.5 g (74 % d.Th.) 8-Methyl-1,1-dioxothiochroman-4-on-7-carbonsäure-O-ethyloxim; Schmelzpunkt 198°C.
13. 8-Methyl-l-oxothiochroman-4-on-7-carbonsäure
   7.0 g (31.5 mmol) 8-Methylthiochroman-4-on-7-carbonsäure werden zusammen mit einer Spatelspitze Natriumwolframat in 70 ml Essigsäure vorgelegt. Bei 50°C werden 3.6 g (31.5 mmol) 30%ige Wasserstoffperoxidlösung zugetropft. Man rührt 3 Stunden nach. Danach wird die Reaktionslösung in Wasser eingerührt. Man extrahiert mit Essigsäureethylester das Produkt. Die organische Phase wird getrocknet und das Lösungsmittel entfernt. Das Produkt wird durch Chromatographie gereinigt.
   Ausbeute: 5.4 g (72 % d.Th.) 8-Methyl-1-oxothiochroman-4-on-7-carbonsäure; ¹H-NMR (d⁶-DMSO): δ [ppm] = 8.0 (2H,m), 3.5 (3H,m), 2.8 (1H,m), 2.7 (3H,s).
14. 3-Thioessigsäure-2-methylbenzoesäuremethylester 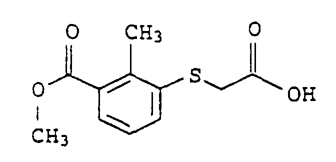
   Zu 1.6 g (0.068 mol) NaH in 40 ml Dimethylformamid werden 12.4 g (0.068 mol) 3-Thio-2-methylbenzoesäuremethylester in 80 ml Dimethylformamid getropft. Es wird 60 min bei ca. 20°C gerührt. Danach gibt man 8 g (0.068 mol) Chloressigsäure zu. Es wird 4 Stunden bei ca. 20°C gerührt.
   Aufgearbeitet wird durch Einrühren des Reaktionsgemisches in salzsaures Eiswasser.
   Der entstehende Niederschlag wird abgesaugt, mit Wasser gewaschen und getrocknet.
   Ausbeute: 14.6 g (89 % d.Th.) 3-Thioessigsäure-2-methylbenzoesäuremethylester; ¹H-NMR (d⁶-DMSO): δ [ppm] = 7.55 (1H,d), 7.45 (1H,d), 7.21 (1H,tr), 3.82 (2H,s), 2.50 (3H,s).
15. 7-Methyl-benzo[b]thiophen-3[2H]-on-6-carbonsäuremethylester 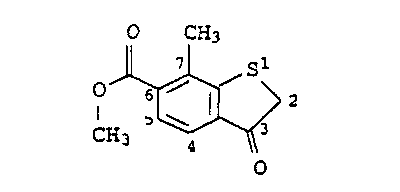
   14.3 g (0.06 mmol) 3-Thioessigsäure-2-methylbenzoesäure werden in 300 ml Methylenchlorid gelöst. 13.1 g (0.11 mmol) Thionylchlorid werden zugetropft. Man erhitzt eine Stunde unter Rückfluß. Danach wird das Lösungsmittel sowie überschüssiges Thionylchlorid abdestilliert. Der Rückstand wird in 100 ml Methylenchlorid aufgenommen und mit 31.8 g (0.24 mmol) Aluminiumtrichlorid versetzt. Die Reaktion wird 1 Stunde bei ca. 20°C gerührt. Anschließend gibt man das Gemisch auf Eiswasser und trennt die organische Phase ab. Nach dem Waschen und Trocknen der organischen Phase wird das Lösungsmittel entfernt. Das Produkt wird ohne Reinigung weiter umgesetzt.
   Ausbeute: 12.9 g (97 % d.Th.) 7-Methyl-benzo[b]thiophen-3[2H]-on-6-carbonsäuremethylester; ¹H-NMR (CDCl₃) : δ [ppm] = 7.65 (2H,m), 3.93 (3H,s) , 3.88 (2H,s), 2.50 (3H,s).
16. 7-Methyl-3-hydroxybenzo[b]thiophen-[2H]-6-carbonsäuremethylester 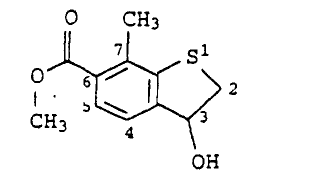
   12.8 g (0.058 mol) 7-Methyl-benzo[b]thiophen-3[2H]-on-6-carbonsäuremethylester werden in 120 ml Methylenchlorid und 60 ml Methanol gelöst und auf 0°C gekühlt. 1.1 g (0.029 mol) Natriumborhydrid wird portionsweise zugegeben. Es wird 3 Stunden gerührt. Durch Zugabe von Wasser wird die Reaktion abgebrochen. Die Phasen werden getrennt und die wäßrige Phase wird mit Methylenchlorid extrahiert. Die vereinigten organischen Phasen werden getrocknet. Das Lösungsmittel wird abdestilliert. Das Rohprodukt wird weiter umgesetzt.
   Ausbeute: 13.2 g (100 % d.Th.) 7-Methyl-3-hydroxybenzo-[b]thiophen-[2H]-6-carbonsäuremethylester; ¹H-NMR (CDCl₃) : δ [ppm] = 7.6 (2H,m), 5.3 (1H,m) 3.9 (3H,s), 3.7 (1H,m), 3.3 (1H,m), 2.4 (3H,s).
17. 7-Methyl-3-methoxybenzo[b]thiophen-[2H]-6-carbonsäuremethylester
   2.4 g (0.059 mol) NaH wird in 50 ml DMF gelöst. 13.2 g 7-Methyl-3-hydroxybenzo[b]thiophen-[2H]-6-carbonsäuremethylester gelöst in 50 ml werden zugetropft.Anschließend wird 2 Stunden bei ca. 20°C gerührt. Danach werden 8.4 g (0.059 mol) Iodmethan zugefügt und weitere 2 Stunden gerührt. Die Reaktionslösung wird auf Eiswasser gegeben und mit Essigsäureethylester extrahiert. Die organische Phase wird getrocknet und anschließend eingeengt. Das Produkt wird durch Chromatographie gereinigt.
   Ausbeute: 3.5 g (25 % d.Th.) 7-Methyl-3-methoxybenzo[b]thiophen-[2H]-6-carbonsäuremethylester; ¹H-NMR (CDCl₃) : δ [ppm] = 7.60 (1H,d), 7.20 (1H,d), 5.04 (1H,m), 3.90 (3H,s), 3.56 (1H,m), 3.40 (3H,s), 3.38 (1H,m), 2.50 (3H,s).

Analog der oben beschriebenen Verseifung der Thiochromanonester erhält man auch die entsprechenden Benzothiophensäuren.

In analoger Weise werden die in den nachfolgenden Tabellen aufgeführten Verbindungen erhalten:

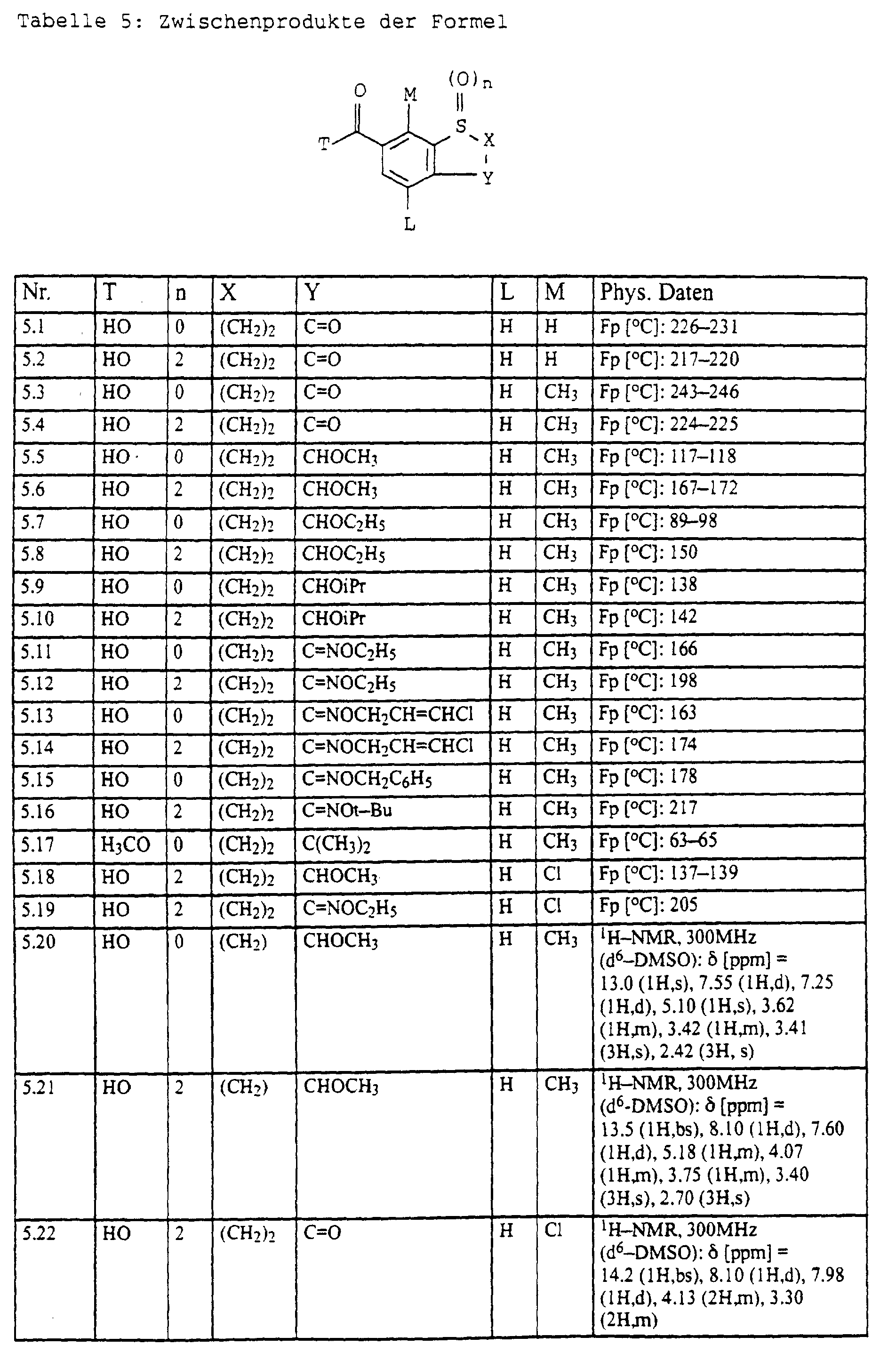

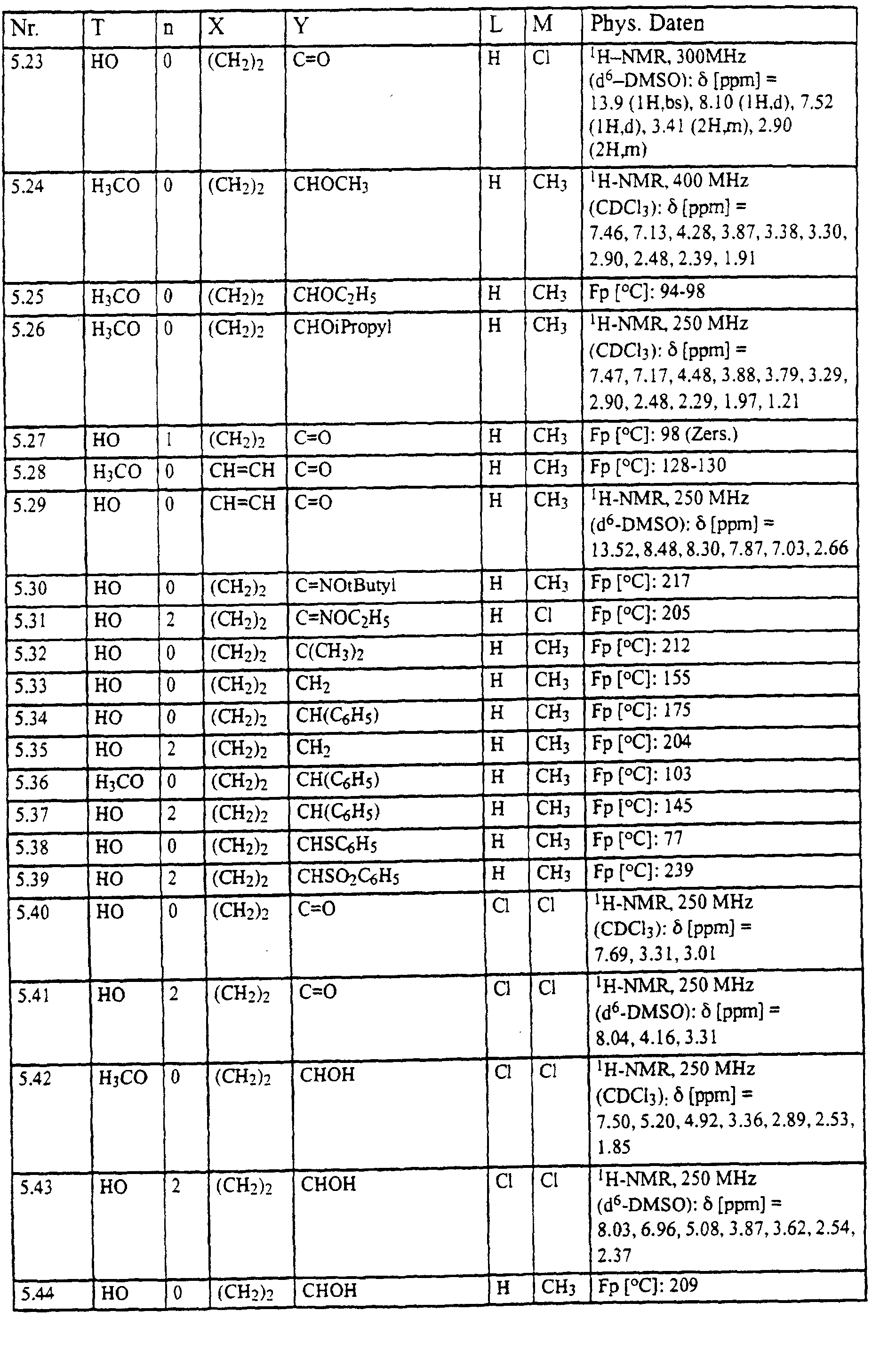

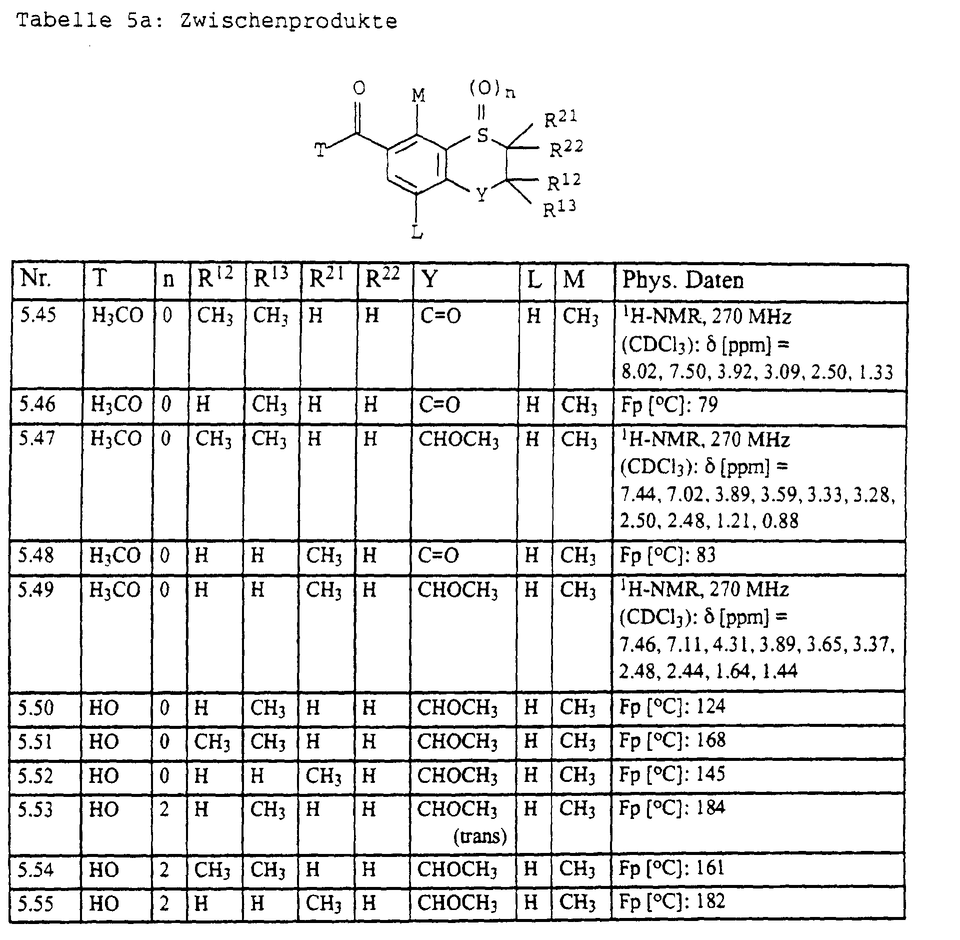

### Herstellung der Endprodukte

1. 1,3-Dimethyl-4-(8-methyl-1,1-dioxothiochroman-4-on-7-carbonyl)-5-hydroxy-pyrazol
   a) 17.4 g (0.0685 mmol) 8-Methyl-1,1.dioxothiochroman-4-on-7-carbonsäure werden in 170 ml Toluol gelöst, mit einem Tropfen Dimethylformamid versetzt und 8.96 g (0.0753 mol) Thionylchlorid zugegeben. Nach 4 Stunden refluxieren wird das Reaktionsgemisch eingeengt. Das Reaktionsprodukt wird direkt weiter umgesetzt.
      Ausbeute: 18.6 g (99 % d.Th.) an 8-Methyl-1,1-dioxothiochroman-4-on-7-carbon-säurechlorid
   b) 0.82 g (7.3 mmol) 1,3-Dimethylpyrazolon werden zusammen mit 0.74 g (7.3 mmol) Triethylamin in 10 ml Acetonitril gelöst. Man tropft dazu 2.0 g (7.3 mmol) Säurechlorid aus a) in 20ml Acetonitril gelöst. Es wird eine Stunde bei Raumtemperatur gerührt. danach gibt man 0.42 g (4.9 mmol) Acetoncyanhydrin und 3.7 g (36.7 mmol) Triethylamin hinzu. Es werden 6 Stunden lang gerührt. Die Aufarbeitung erfolgt durch Zugabe von 2N Salzsäure und Extraktion mit Essigsäureethylester. Die organische Phase wird dann mit Natriumcarbonatlösung gewaschen, die wäßrige Phase wird angesäuert und der entstehende Niederschlag abgesaugt und getrocknet.
      Ausbeute: 0.2 g (8 % d.Th.) 1,3-Dimethyl-4-(8-methyl-1,1-dioxothiochroman-4-on-7-carbonyl)-5-hydroxy-pyrazol; Schmelzpunkt: 83°C.
2. 1,3-Dimethyl-4-(8-methylthiochroman-4-on-7-carbonyl-O-ethyloxim)-5-hydroxy-pyrazol 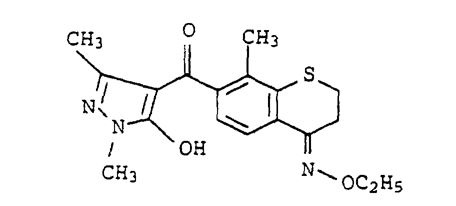
   1 g (3.77 mmol) 8-Methylthiochroman-4-on-7-carbonsäure-O-ethyloxim werden in tert. Amylalkohol zusammen mit 0.46 g (4.07 mmol) 1,3-Dimethylpyrazolon und 0.84 g (4.07 mmol) Dicyclohexylcarbodiimid (DCC) bei RT 2 Stunden gerührt. Anschließend gibt man 0.78 g (5.66 mmol) Kaliumcarbonat hinzu, erhitzt auf 90°C und rührt 5 Stunden bei dieser Temperatur. Aufgearbeitet wird durch Einrühren des Reaktionsansatzes in Wasser und anschließender Extraktion mit Essigsäureethylester. Die organische Phase wird verworfen. Die wäßrige Phase wird mit HCl angesäuert und das Wertprodukt mit Essigester extrahiert. Das Extraktionsmittel wird entfernt. Das Produkt wird durch Chromatographie gereinigt.
   Ausbeute: 0.35 g (26 % d.Th.) an 1,3-Dimethyl-4-(8-methyl-thiochroman-4-on-7-carbonyl-O-ethyloxim)-5-hydroxy-pyrazol; ¹H-NMR (CDCl₃): δ [ppm] = 7.95 (1H,d), 6.99 (1H,d), 4.28 (2H,q), 3.69 (3H,s), 3.10 (2H,m), 2.97 (2H,m), 2.30 (3H,s), 1.75 (3H,s), 1.38 (3H,tr).
3. 1,3-Dimethyl-4-(8-methyl-1,1-dioxothiochroman-4-on-7-carbonyl-O-ethyloxim)-5-hydroxy-pyrazol
   0.35 g (1 mmol) 1,3-Dimethyl-4-(8-methylthiochroman-4-on-7-carbonyl-O-ethyloxim)-5-hydroxy-pyrazol werden zusammen mit 5ml Essigsäure und einer Spatelspitze Natriumwolframat vorgelegt und auf 50°C erwärmt. Man gibt dann 0.24 g (2.1 mmol) 30%ige Wasserstoffperoxidlösung zu und rührt 4 Stunden. Danach wird Wasser zugegeben und mit Essigsäureethylester extrahiert. Die organische Phase wird mit Thiosulfatlösung gewaschen und einrotiert.
   Ausbeute: 0.35 g (89 % d.Th.) an 1,3-Dimethyl-4-(8-methyl-1,1-dioxothiochroman-4-on-7-carbonyl-O-ethyloxim)-5-hydroxy-pyrazol; ¹H-NMR (CDCl₃): δ [ppm] = 9.90 (1H,bs), 8.12 (1H,d), 7.35 (1H,d), 4.32 (2H,q), 3.65 (3H,s), 3.44 (4H,m), 2.70 (3H,s), 1.73 (3H,s), 1.38 (3H,tr).

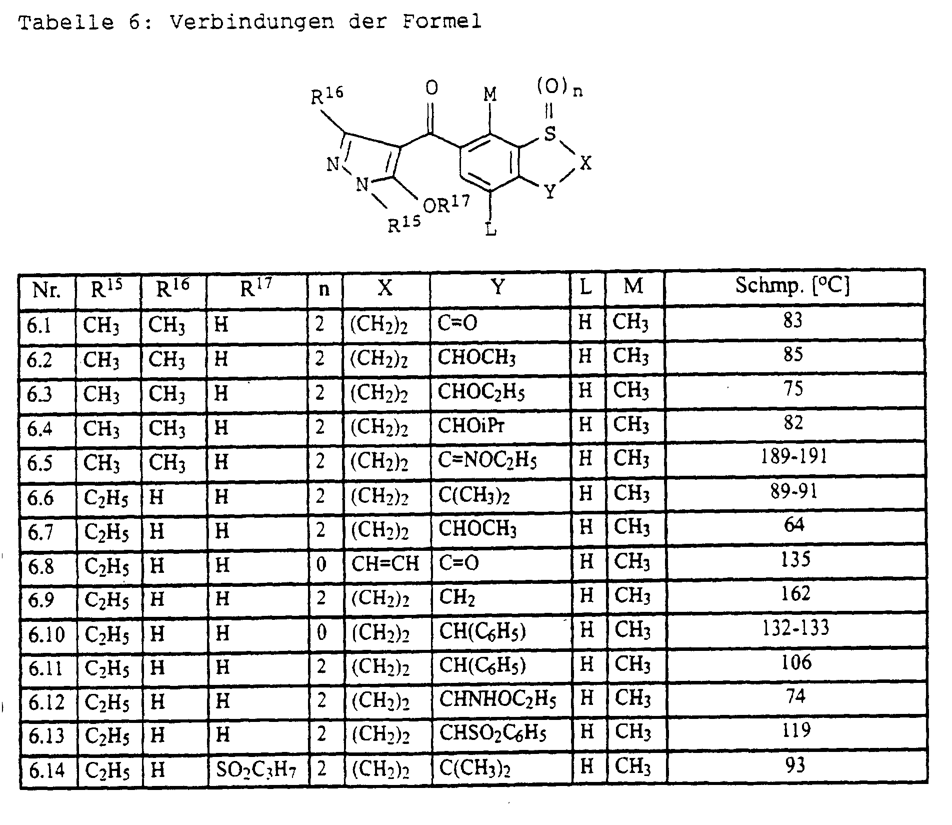

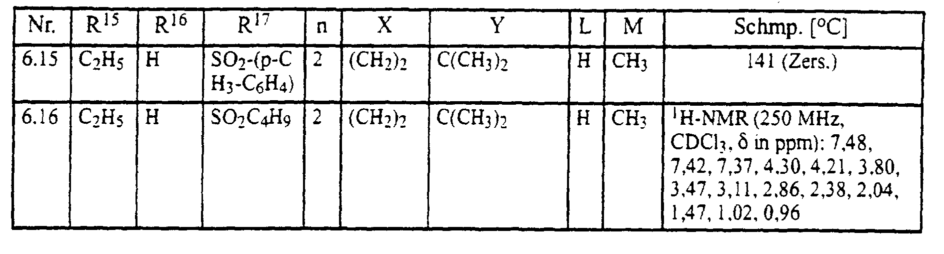

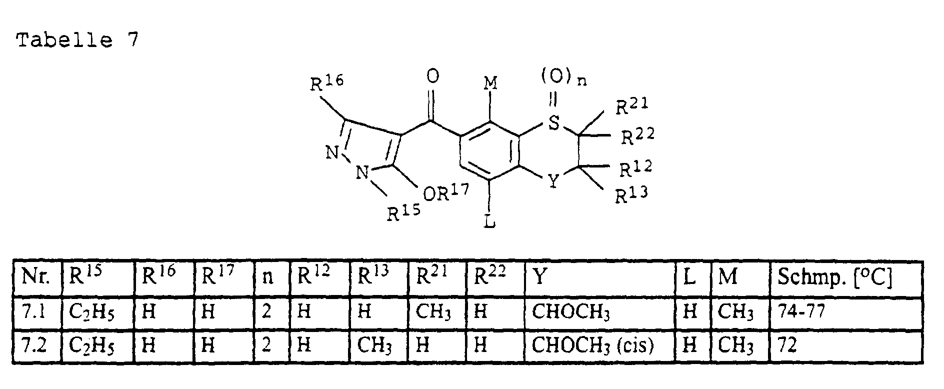

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

Unter Berücksichtigung der Vielseitigkeit der Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden.

Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Sektrum) eingesetzt.

Die erfindungsgemäßen Verbindungen I können beispielsweise wie folgt formuliert werden:
I. 20 Gewichtsteile der Verbindung Nr. 6.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 6.2 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 6.3 werden in einer Mischung gelöst, die aus 25 Gewichtsteilen Cyclohexanon, 65 Gewichtsteilen einer Mineralölfraktion vom Siedepunkt 210 bis 80°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 6.4 werden mit 3 Gewichtsteilen des Natriumsalzes der Diisobutylnaphthalin-a-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
V. 3 Gewichtsteile des Wirkstoffs Nr. 6.5 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 6.8 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 6.9 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 6.10 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl) besteht. Man erhält ein stabiles Emulsionskonzentrat.

Zur Verbreiterung des Wirkungsspektrums und zur Erzielung synergistischer Effekte können die Pyrazol-4-yl-benzolderivate I mit zahlreichen Vertretern anderer herbizider oder wachstums-regulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

### Anwendungsbeispiele

Die herbizide Wirkung der Pyrazol-4-yl-benzolderivate der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0.5 bzw. 0.25 kg/ha a. S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Lateinischer Name | Deutscher Name | Englischer Name |
| Zea mays | Mais | Indian corn |
| Chenopodium album | Weißer Gänsefuß | lambsquarters (goosefoot) |
| Sinapis alba | Weißer Senf | white mustard |
| Solanum nigrum | Schwarzer Nachtschatten | black nightshade |

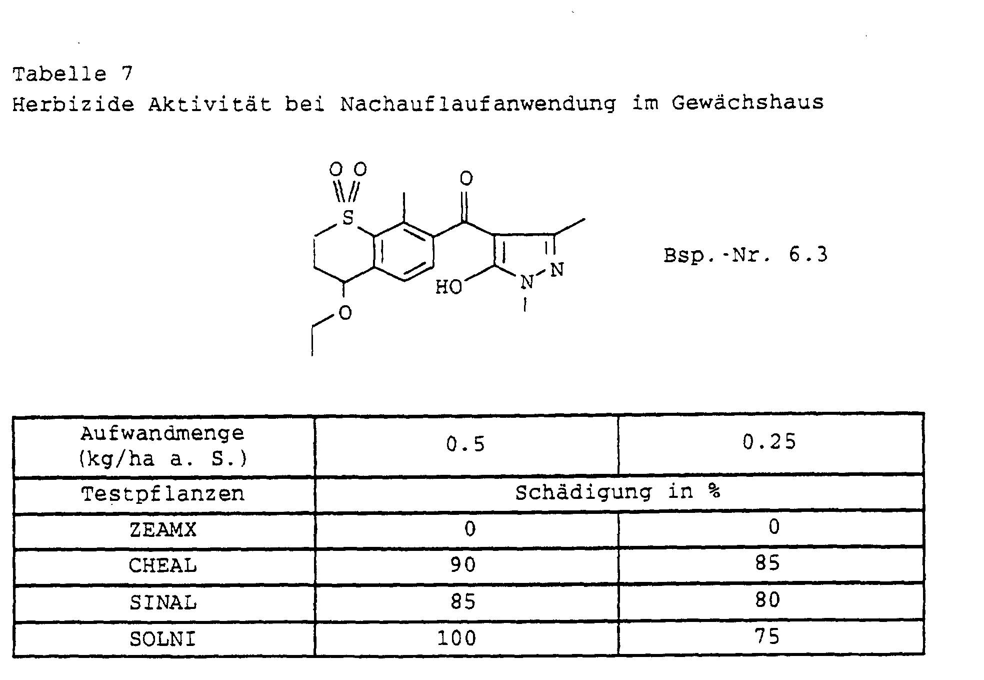

## Patentansprüche

1. Pyrazol-4-yl-benzoylderivate der Formel I 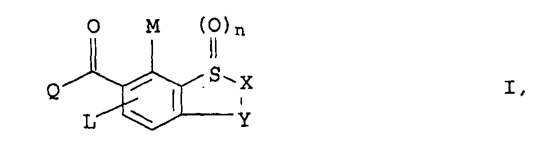 in der Substituenten folgende Bedeutung haben:
L, M Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro, eine Gruppe -(A)ₘ-S(O)ₙR¹ oder eine Gruppe - (A)ₘ-CO-R²;
Y eine Gruppe bestehend aus C=O, C=N-R³, CR⁷-NR⁵R⁶, CR⁷-OR⁸, CR¹⁰R¹¹, CR⁷-SR⁸; ein Heteroatom ausgewählt aus der Gruppe Sauerstoff, Schwefel und Stickstoff;
x eine Kette (-CR¹²R¹³-), (-CR¹²R¹³-CR²¹R²²-), (-CR¹²=CR¹³-), (-CR¹²R¹³-CR¹²=CR¹³-); NR²³;
die Bindung zwischen X und Y kann gesättigt oder ungesättigt sein;
A Sauerstoff oder NR¹⁴;
m null oder eins;
n null, eins oder zwei;
R¹ C₁-C₄-Alkyl, C₁-C₄ Haloalkyl oder NR¹⁴;
R² C₁-C₄ Alkyl, C₁-C₄ Haloalkyl, C₁-C₄-Alkoxy oder NR¹⁴;
R³ Wasserstoff, -NR⁹R⁴; C₁-C₆-Alkyl, C₁-C₆-Haloalkyl, C₁-C₆-Alkoxy, C₁-C₆-Haloalkoxy, C₂-C₆-Alkenyl, C₂-C₆-Haloalkenyl, C₂-C₆-Alkinyl; gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro; gegebenenfalls substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro; gegebenenfalls substituiertes Benzyloxy wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R⁴ Wasserstoff, C₁-C₆-Alkyl, C₁-C₆-Haloalkyl, C₂-C₆-Alkenyl, C₂-C₆-Alkinyl, C=O-NR¹⁴; gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
gegebenenfalls substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R⁹ Wasserstoff, C₁-C₆-Alkyl, C₁-C₆-Haloalkyl, C₂-C₆-Alkenyl, C₂-C₆-Alkinyl, C=O-NR¹⁴; gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
gegebenenfalls substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R⁵, R⁶ unabhängig voneinander Wasserstoff, C₁-C₆-Alkyl, C₂-C₆ Alkenyl, C₁-C₄ Haloalkyl, C₂-C₆ Haloalkenyl, C₁-C₆ Alkoxy, C₁-C₆-Haloalkoxy;
gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
gegebenenfalls substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R⁷ Wasserstoff, C₁-C₆ Alkyl, C₁-C₄-Alkoxy, C₁-C₄-Haloalkyl, C₁-C₄-Haloalkoxy; gegebenenfalls substituiertes Phenyl, wobei die Substituenten bestehen können aus ein bis drei Halogenen, C₁-C₄-Alkyl, C₁-C₄-Alkoxy, C₁-C₄-Haloalkoxy, Nitro; R⁷ und R²¹ oder R⁷ und R²³ oder R⁷ und R¹² können eine Bindung bilden;
R⁸ Wasserstoff, C₁-C₆-Alkyl, C₁-C₄-Halogenalkyl, gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
gegebenenfalls substituiertes Benzyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R¹⁰,R¹¹ unabhängig voneinander Wasserstoff, C₁-C₆-Alkyl; gegebenenfalls substituiertes Phenyl, wobei die Substituenten bestehen können aus ein bis drei Halogenen, C₁-C₄-Alkyl, C₁-C₄-Alkoxy, C₁-C₄-Haloalkoxy, Nitro; R¹⁰ und R¹² oder R¹⁰ und R²¹ oder R¹⁰ und R²³ können eine Bildung bilden;
oder R¹⁰ und R¹¹ können gemeinsam mit dem Kohlenstoff, an den sie gebunden sind, 1,3-Dioxanyl oder 1,3-Dioxolanyl, das mit Wasserstoff oder C₁-C₄-Alkyl substituiert ist, bilden;
R¹²,R¹³ unabhängig voneinander Wasserstoff, C₁-C₆-Alkyl, C₁-C₆-Haloalkyl, C₁-C₆-Alkoxy, C₁-C₆-Haloalkoxy; gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R¹⁴ C₁-C₄-Alkyl;
R²¹ Wasserstoff, C₁-C₆-Alkyl, C₁-C₆-Haloalkyl, C₁-C₆-Alkoxy, C₁-C₆-Haloalkoxy; gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R²² Wasserstoff, C₁-C₆ Alkyl, C₁-C₆ Haloalkyl, C₁-C₆-Alkoxy, C₁-C₆-Haloalkoxy; gegebenenfalls substituiertes Phenyl, wobei die Substituenten bestehen können aus C₁-C₄-Alkyl, C₁-C₄-Alkoxy, C₁-C₄-Haloalkoxy, C₁-C₄-Haloalkyl, Halogen, Cyano, Nitro;
R²³ Wasserstoff, C₁-C₆-Alkyl, C₂-C₆-Alkenyl, C₁-C₆-Alkoxy; gegebenenfalls mit C₁-C₄-Alkyl, C₁-C₄-Alkoxy, C₁-C₄-Haloalkoxy, C₁-C₄-Haloalkyl, Halogen, Cyano, Nitro substituiertes Phenyl oder Benzyl;
Q ein in Stellung 4 verknüpfter Pyrazolring der Formel II 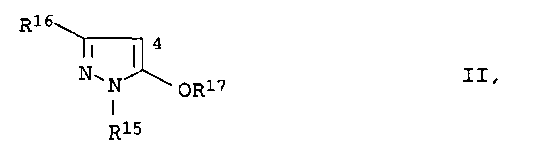
in welcher
R¹⁵ C₁-C₄-Alkyl,
R¹⁶ Wasserstoff, C₁-C₄-Alkyl oder C₁-C₄-Halogenalkyl und
R¹⁷ Wasserstoff, C₁-C₄-Alkylsulfonyl, Phenylsulfonyl oder Alkylphenylsulfonyl bedeuten,
wobei für den Fall, daß Y = C=O ist, X ungleich NR²³ ist,
sowie landwirtschaftlich brauchbare Salze.

2. Pyrazol-4-yl-benzoylderivate der Formel Ia 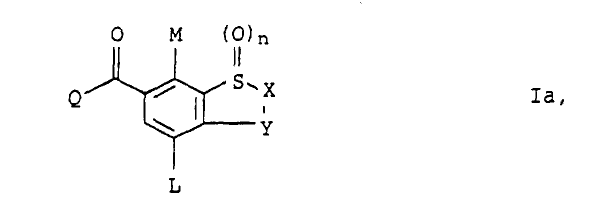 in der L für Wasserstoff, C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkoxy, C₁-C₄-Halogenalkylthio, C₁-C₄-Alkylsulfonyl, Halogen, Nitro oder Cyano steht und Q, X, n und Y die in Anspruch 1 angegebenen Bedeutungen haben, wobei für den Fall, daß Y = C=O ist, X ungleich NR²³ ist.

3. Pyrazol-4-yl-benzoylderivate der Formel Ib 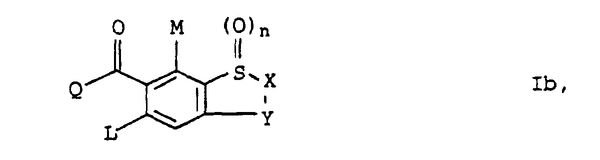 in der L für C₁-C₆ Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und M für Wasserstoff, C₁-C₆ Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und Q, X, n und Y die in Anspruch 1 angegebenen Bedeutungen haben, wobei für den Fall, daß Y = C=O ist, X ungleich NR²³ ist.

4. Pyrazol-4-yl-benzoylderivate der Formel I gemäß Anspruch 1 in der die Reste L bzw. M für Wasserstoff, Methyl, Methoxy, Chlor, Cyano, Nitro, Trifluormethyl stehen.

5. Pyrazol-4-yl-benzoylderivate der Formel Ic 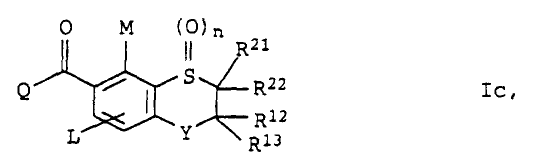 in der L für Wasserstoff, C₁-C₆-Alkyl, C₂-C₆-Alkenyl, C₂-C₆-Alkinyl, C₁-C₄-Alkoxy, C₁-C₄-Haloganalkyl, C₁-C₄-Halogenalkoxy, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und Q, n, Y sowie R²², R²¹, R¹² und R¹³ die in Anspruch 1 angegebenen Bedeutungen haben.

6. Pyrazol-4-yl-benzoylderivate der Formel Id 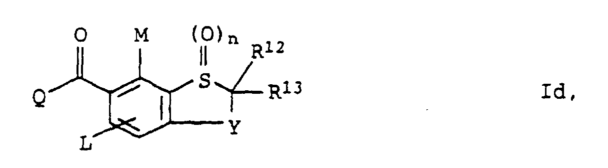 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Nitro oder Cyano und Q, n, Y sowie R¹² und R¹³ die in Anspruch 1 angegebenen Bedeutungen haben.

7. Pyrazol-4-yl-benzoylderivate der Formel Ie 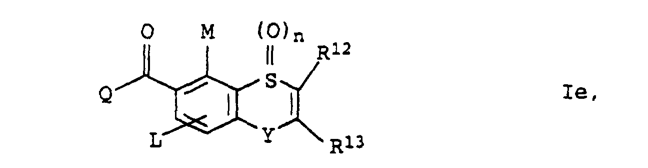 in der L für Wasserstoff, C₁-C₆ Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen Nitro oder Cyano und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen Nitro oder Cyano und Q, n, Y sowie R¹² und R¹³ die in Anspruch 1 angegebenen Bedeutungen haben.

8. Pyrazol-4-yl-benzoylderivate der Formel I gemäß Anspruch 1 in der n für eins oder zwei steht und Y für CR⁷-OR⁸, wobei R⁷ und R⁸ die in Anspruch 1 angegebene Bedeutung haben.

9. Verfahren zur Herstellung der Verbindung der Formel I gemäß Anspruch 1, **dadurch gekennzeichnet, daß** man die Pyrazole der Formel IIa 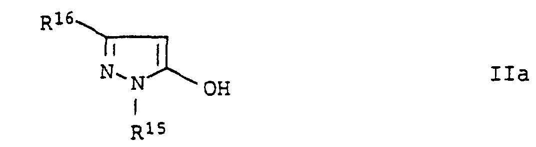 mit einem Säurechlorid der Formel IIIa oder einer Säure IIIb 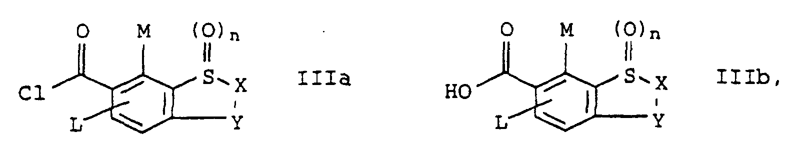 wobei R¹⁵, R¹⁶, L, M, X, n und Y die in Anspruch 1 genannte Bedeutung haben, acyliert und das Acylierungsprodukt in Gegenwart eines Katalysators zu den Verbindungen I umlagert.

10. Herbizides Mittel, enthaltend mindestens ein Pyrazol-4-yl-benzoylderivat der Formel I gemäß Anspruch I und übliche inerte Zusatzstoffe.

11. Verfahren zur Bekämpfung unerwünschten Pflanzenwuchses, **dadurch gekennzeichnet, daß** man eine herbizid wirksame Menge eines Pyrazol-4-yl-benzoylderivates der Formel 1 gemäß Anspruch 1 auf die Pflanzen oder deren Lebensraum einwirken läßt.

## Claims

1. A pyrazol-4-yl-benzoyl derivative of the formula I, 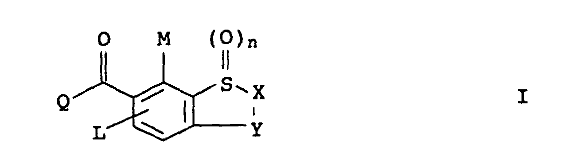 where the substituents have the following meanings:
L,M are hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen, cyano, nitro, a group -(A)ₘ-S(O)ₙR¹ or a group-(A)ₘ-CO-R²;
Y is a group consisting of C=O, C=N-R³, CR⁷-NR⁵R⁶, CR⁷-OR⁸, CR¹⁰R¹¹, CR⁷-SR⁸; a hetero atom selected from the group consisting of oxygen, sulfur and nitrogen;
X is a chain (-CR¹²R²³-), (-CR¹²R²³-CR²¹R²²-), (-CR¹²=CR¹³-), (-CR¹²R¹³-CR¹²=CR¹³- ) ; _{NR}²³;
the bond between X and Y can be saturated or unsaturated;
A is oxygen or NR¹⁴;
m is zero or one;
n is zero, one or two;
R¹ is C₁-C₄-alkyl, C₁-C₄-haloalkyl or NR¹⁴;
R² is C₁-C₄-alkyl, C₁-C₄-haloalkyl, C₁-C₄-alkoxy or NR¹⁴;
R³ is hydrogen, -NR⁹R⁴; C₁-C₆-alkyl, C₁-C₆-haloalkyl, C₁-C₆-alkoxy, C₁-C₆-haloalkoxy, C₂-C₆-alkenyl, C₂-C₆-haloalkenyl, C₂-C₆-alkynyl; unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro; unsubstituted or substituted benzyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
unsubstituted or substituted benzyloxy, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R⁴ is hydrogen, C₁-C₆-alkyl, C₁-C₆-haloalkyl, C₂-C₆-alkenyl, C₂-C₆-alkynyl, C=O-NR¹⁴; unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
unsubstituted or substituted benzyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R⁹ is hydrogen, C₁-C₆-alkyl, C₁-C₆-haloalkyl, C₂-C₆-alkenyl, C₂-C₆-alkynyl, C=O-NR¹⁴; unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
unsubstituted or substituted benzyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R⁵,R⁶ independently of one another are hydrogen, C₁-C₆-alkyl, C₂-C₆-alkenyl, C₁-C₄-haloalkyl, C₂-C₆-haloalkenyl, C₁-C₆-alkoxy, C₁-C₆-haloalkoxy; unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
unsubstituted or substituted benzyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R⁷ is hydrogen, C₁-C₆-alkyl, C₁-C₄-alkoxy, C₁-C₄-haloalkyl, C₁-C₄-haloalkoxy; unsubstituted or substituted phenyl, it being possible for the substituents to be from the series consisting of one to three halogens, C₁-C₄-alkyl, C₁-C₄-alkoxy, C₁-C₄-haloalkoxy, nitro; R⁷ and R²¹ or R⁷ and R²³ or R⁷ and R¹² can form a bond;
R⁸ is hydrogen, C₁-C₆-alkyl, C₁-C₄-haloalkyl, unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
unsubstituted or substituted benzyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R¹⁰,R¹¹ independently of one another are hydrogen, C₁-C₆-alkyl; unsubstituted or substituted phenyl, it being possible for the substituents to be from the series consisting of one to three halogens, C₁-C₄-alkyl, C₁-C₄-alkoxy, C₁-C₄-haloalkoxy, nitro; R¹⁰ and R¹² or R¹⁰ and R²¹ or R¹⁰ and R²³ can form a bond;
or R¹⁰ and R¹¹ can form, together with the carbon to which they are bonded, 1,3-dioxanyl or 1,3-dioxolanyl which is substituted by hydrogen or C₁-C₄-alkyl;
R¹²,R¹³ independently of one another are hydrogen, C₁-C₆-alkyl, C₁-C₆-haloalkyl, C₁-C₆-alkoxy, C₁-C₆-haloalkoxy; unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R¹⁴ is C₁-C₄-alkyl;
R²¹ is hydrogen, C₁-C₆-alkyl, C₁-C₆-haloalkyl, C₁-C₆-alkoxy, C₁-C₆-haloalkoxy; unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R²² is hydrogen, C₁-C₆-alkyl, C₁-C₆ haloalkyl, C₁-C₆-alkoxy, C₁-C₆-haloalkoxy; unsubstituted or substituted phenyl, it being possible for the substituents to be from the series consisting of C₁-C₄-alkyl, C₁-C₄-alkoxy, C₁-C₄-haloalkoxy, C₁-C₄-haloalkyl, halogen, cyano, nitro;
R²³ is hydrogen, C₁-C₆-alkyl, C₂-C₆-alkenyl, C₁-C₆-alkoxy; phenyl or benzyl, each of which is unsubstituted or substituted by C₁-C₄-alkyl, C₁-C₄-alkoxy, C₁-C₄-haloalkoxy, C₁-C₄-haloalkyl, halogen, cyano, nitro;
Q is a pyrazole ring, linked in the 4-position, of the formula II 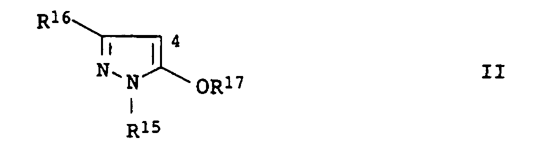
where
R¹⁵ is C₁-C₄-alkyl,
R¹⁶ is hydrogen, C₁-C₄-alkyl or C₁-C₄-haloalkyl and
R¹⁷ is hydrogen, C₁-C₄-alkylsulfonyl, phenylsulfonyl or alkylphenylsulfonyl,
where, in the event that Y = C=O, X is other than NR²³,
or an agriculturally useful salt thereof.

2. A pyrazol-4-yl-benzoyl derivative of the formula Ia, 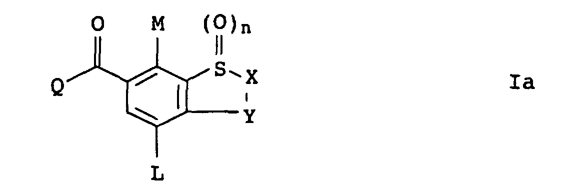 where L is hydrogen, C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-haloalkoxy, C₁-C₄-haloalkylthio, C₁-C₄-alkylsulfonyl, halogen, nitro or cyano and M is hydrogen, C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-haloalkoxy, C₁-C₄-haloalkoxy, C₁-C₄-haloalkylthio, C₁-C₄-alkylsulfonyl, halogen, nitro or cyano and Q, X, n and Y have the meanings given in claim 1, where, in the event that Y = C=O, X is other than NR²³.

3. A pyrazol-4-ylbenzoyl derivative of the formula Ib, 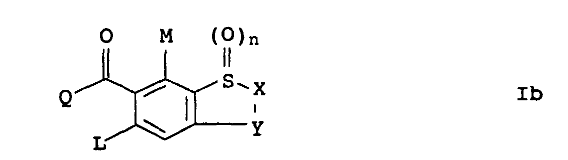 where L is C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and Q, X, n and Y have the meanings given in claim 1, where, in the event that Y = C=O, X is other than NR²³.

4. A pyrazol-4-ylbenzoyl derivative of the formula I as claimed in claim 1 where the radicals L and M are hydrogen, methyl, methoxy, chlorine, cyano, nitro or trifluoromethyl.

5. A pyrazol-4-ylbenzoyl derivative of the formula Ic, 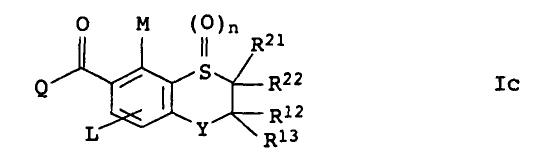 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and Q, n, Y and R²², R²¹, R¹² and R¹³ have the meanings given in claim 1.

6. A pyrazol-4-ylbenzoyl derivative of the formula Id, 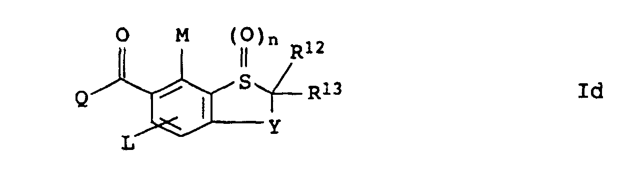 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and Q, n, Y and R¹² and R¹³ have the meanings given in claim 1.

7. A pyrazol-4-ylbenzoyl derivative of the formula Ie 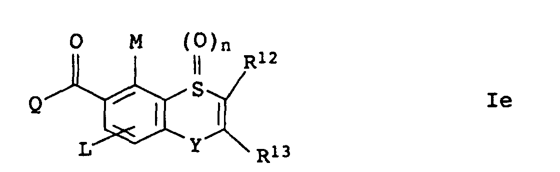 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, nitro or cyano and Q, n, Y and R¹² and R¹³ have the meanings given in claim 1.

8. A pyrazol-4-ylbenzoyl derivative of the formula I as claimed in claim 1 where n is one or two and Y is CR⁷-OR⁸, where R⁷ and R⁸ have the meanings given in claim 1.

9. A process for the preparation of the compound of the formula I as claimed in claim 1, which comprises acylating the pyrazoles of the formula IIa 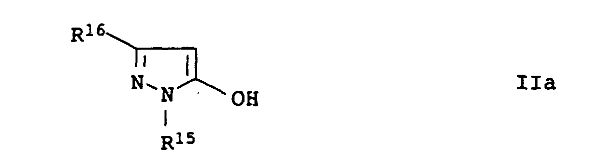 with an acid chloride of the formula IIIa or an acid IIIb 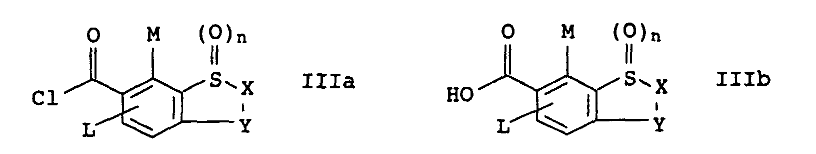 where R¹⁵, R¹⁶, L, M, X, n and Y have the meanings given in claim 1 and subjecting the acylation product to a rearrangement reaction in the presence of a catalyst to give the compounds I.

10. A herbicidal composition comprising at least one pyrazol-4-ylbenzoyl derivative of the formula I as claimed in claim 1 and customary inert additives.

11. A method of controlling undesirable vegetation, which comprises allowing a herbicidally active amount of a pyrazol-4-ylbenzoyl derivative of the formula 1 as claimed in claim 1 to act on the plants or their environment.

## Revendications

1. Dérivés de pyrazol-4-yl-benzoyle de formule I 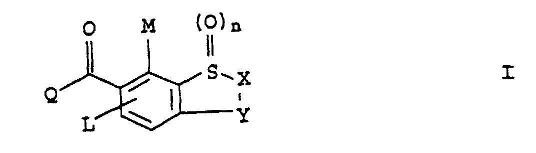 dans laquelle les substituants ont la signification suivante:
L, M hydrogène, alkyle en C₁-C₆, alcényle en C₂-C₆. alcynyle en C_{2_}C₆, alcoxy en C₁-C₄, tandis que ces groupes peuvent éventuellement être substitués par un à cinq atomes d'halogène ou alcoxy en C₁-C₄; halogéno, cyano, nitro, un groupe - (A)ₘ-S(O)ₙR¹ ou un groupe - (A)ₘ-CO-R²;
Y un groupe consistant en C=O, C=N-R³, CR⁷-NR⁵R⁶, CR⁷-OR⁸, CR¹⁰R¹¹, CR⁷-SR⁸; un hétéroatome choisi dans le groupe de l'oxygène, le soufre et l'azote;
x une chaîne (-CR¹²R¹³-), (-CR¹²R¹³-CR²¹R²²-), (-CR¹²=CR¹³-), (-CR¹²R¹³-CR¹²=CR¹³-) ; NR²³;
la liaison entre X et Y pouvant être saturée ou insaturée;
A oxygène ou NR¹⁴;
m zéro ou un;
n zéro, un ou deux;
R¹ alkyle en C₁-C₄, halogénoalkyle en C₁-C₄ ou NR¹⁴;
R2 alkyle en C₁-C₄, halogénoalkyle en C₁-C₄, alcoxy en C₁-C₄ ou NR¹⁴;
R3 hydrogène, -NR⁹R⁴; alkyle en C₁-C₆, halogénoalkyle en C₁-C₆, alcoxy en C₁-C₆, halogénoalcoxy en C₁-C₆, alcényle en C₂-C₆, halogénoalcényle en C₁-C₆, alcynyle en C₂-C₆; phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro; benzyle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro; benzyloxy éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R⁴ hydrogène, alkyle en C₁-C₆, halogénoalkyle en C₁-C₆, alcényle en C₂-C₆, alcynyle en C₂-C₆, C=O-NR¹⁴; phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro; benzyle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R⁹ hydrogène, alkyle en C₁-C₆ halogénoalkyle en C₁-C₆, alcényle en C₂-C₆, alcynyle en C₂-C₆, C=O-NR¹⁴; phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro; benzyle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R⁵,R⁶ indépendamment l'un de l'autre, hydrogène, alkyle en C₁-C₆, alcényle en C₂-C₆, halogénoalkyle en C₁-C₄, halogénoalcényle en C₂-C₆, alcoxy en C₁-C₆, halogénoalcoxy en C₁-C₆; phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro; benzyle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R⁷ hydrogène, alkyle en C₁-C₆, alcoxy en C₁-C₄, halogénoalkyle en C₁-C₄, halogénoalcoxy en C₁-C₄; phényle éventuellement substitué, tandis que les substituants peuvent consister en un à trois halogéno, alkyle en C₁-C₄, alcoxy en C₁-C₄, halogénoalcoxy en C₁-C₄, nitro; R⁷ et R²¹ ou R⁷ et R²³ ou R⁷ et R¹² peuvent former une liaison;
R⁸ hydrogène, alkyle en C₁-C₆, halogénoalkyle en C₁-C₄, phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro; benzyle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R¹⁰, R¹¹ indépendamment l'un de l'autre, hydrogène, alkyle en C₁-C₆; phényle éventuellement substitué, tandis que les substituants peuvent consister en un à trois halogéno, alkyle en C₁-C₄, alcoxy en C₁-C₄, halogénoalcoxy en C₁-C₄, nitro; R¹⁰ et R²¹ ou R¹⁰ et R²³ peuvent former une liaison; ou R¹⁰ et R¹¹ peuvent, conjointement avec l'atome de carbone auquel ils sont liés, former un 1,3-dioxanyle ou un 1,3-dioxolanyle, qui est substitué par l'hydrogène ou un alkyle en C₁-C₄;
R¹²,R¹³ indépendamment l'un de l'autre, hydrogène, alkyle en C₁-C₆, halogénoalkyle en C₁-C₆, alcoxy en C₁-C₆, halogénoalcoxy en C₁-C₆; phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R¹⁴ alkyle en C₁-C₄;
R²¹ hydrogène, alkyle en C₁-C₆, halogénoalkyle en C₁-C₆, alcoxy en C₁-C₆, halogénoalcoxy en C₁-C₆; phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R²² hydrogène, alkyle en C₁-C₆, halogénoalkyle en C₁-C₆, alcoxy en C₁-C₆, halogénoalcoxy en C₁-C₆; phényle éventuellement substitué, tandis que les substituants peuvent consister en alkyle en C₁-C₄, alcoxy en C₁-C₄, halogénoalcoxy en C₁-C₄, halogénoalkyle en C₁-C₄, halogéno, cyano, nitro;
R²³ hydrogène, alkyle en C₁-C₆, alcényle en C₂-C₆, alcoxy en C₁-C₆; phényle ou benzyle éventuellement substitué par alkyle en C₁-C₄, alcoxy en C₁-C₄, halogénoalcoxy en C₁-C₄, halogénoalkyle en C₁-C₄, halogéno,cyano, nitro;
Q un cycle pyrazole relié sur la position 4, de formule II 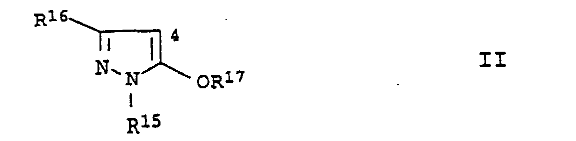
dans laquelle
R¹⁵ désigne un alkyle en C₁-C₄,
R¹⁶ hydrogène, alkyle en C₁-C₄ ou halogénoalkyle en C₁-C₄, et
R¹⁷ hydrogène, alkylsulfonyle en C₁-C₄, phénylsulfonyle ou alkylphénylsulfonyle,
tandis que dans le cas où Y = C=O, X est différent de NR²³,
ainsi que les sels utilisables en agriculture.

2. Dérivés de pyrazol-4-yl-benzoyle de formule Ia 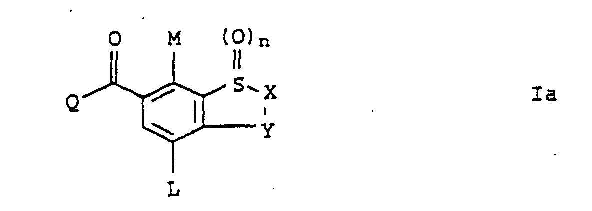 dans laquelle L désigne hydrogène, alkyle en C₁-C₆, alcényle en C₂-C₆, alcynyle en C₂-C₆, alcoxy en C₁-C₄, alkylthio en C₁-C₄, halogénoalkyle en C₁-C₄, halogénoalcoxy en C₁-C₄, halogénoalkylthio en C₁-C₄, alkylsulfonyle en C₁-C₄, halogéno, nitro ou cyano et M désigne hydrogène, alkyle en C₁-C₆, alcényle en C₂-C₆, alcynyle en C₂-C₆, alcoxy en C₁-C₄, alkylthio en C₁-C₄, halogénoalkyle en C₁-C₄, halogénoalcoxy en C₁-C₄, halogénoalcoxy en C₁-C₄, halogénoalkylthio en C₁-C₄, alkylsulfonyle en C₁-C₄, halogéno, nitro ou cyano, et Q, X, n et Y ont les significations indiquées dans la revendication 1, tandis que dans le cas où Y = C=O, X est différent de NR²³.

3. Dérivés de pyrazol-4-yl-benzoyle de formule Ib 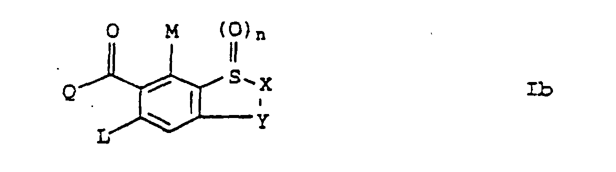 dans laquelle L désigne alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano et M désigne hydrogène, alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano, et Q, X, n et Y ont les significations indiquées dans la revendication 1, tandis que dans le cas où Y = C=O, X est différent de NR²³.

4. Dérivés de pyrazol-4-yl-benzoyle de formule I selon la revendication 1, dans laquelle les restes L et M respectivement représentent hydrogène, méthyle, méthoxy, chloro, cyano, nitro, trifluorométhyle.

5. Dérivés de pyrazol-4-yl-benzoyle de formule Ic 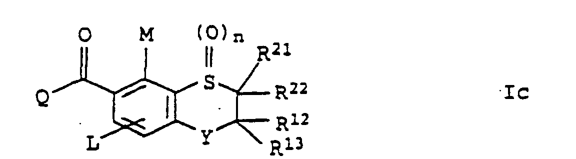 dans laquelle L désigne hydrogène, alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano et M désigne hydrogène, alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano, et Q, n et Y, ainsi que R²², R²¹, R¹² et R¹³ ont les significations indiquées dans la revendication 1.

6. Dérivés de pyrazol-4-yl-benzoyle de formule Id 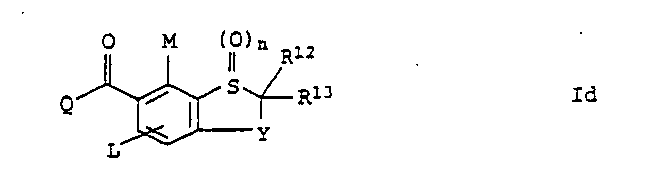 dans laquelle L désigne hydrogène, alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano et M désigne hydrogène, alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano, et Q, n et Y, ainsi que R¹² et R¹³ ont les significations indiquées dans la revendication 1.

7. Dérivés de pyrazol-4-yl-benzoyle de formule Ie 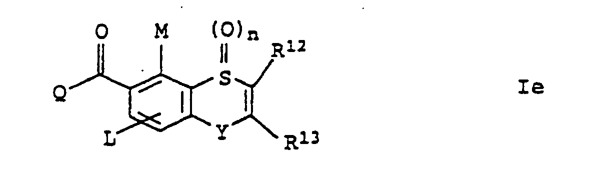 dans laquelle L désigne hydrogène, alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano et M désigne hydrogène, alkyle en C₁-C₆, alcényle en C₂-C₆, alcynyle en C₂-C₆, alcoxy en C₁-C₄, halogénoalkyle en C₁-C₄, halogénoalcoxy en C₁-C₄, halogéno, nitro ou cyano, et Q, n et Y, ainsi que R¹² et R¹³ ont les significations indiquées dans la revendication 1.

8. Dérivés de pyrazol-4-yl-benzoyle de formule I selon la revendication 1, dans laquelle n vaut un ou deux et Y désigne CR⁷-OR⁸, tandis que R⁷ et R⁸ ont la signification indiquée dans la revendication 1.

9. Procédé pour la préparation du composé de formule I selon la revendication 1, **caractérisé par le fait qu'**on acyle les pyrazoles de formule IIa 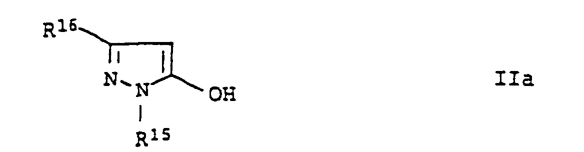 avec un chlorure d'acide de formule IIIa ou un acide IIb 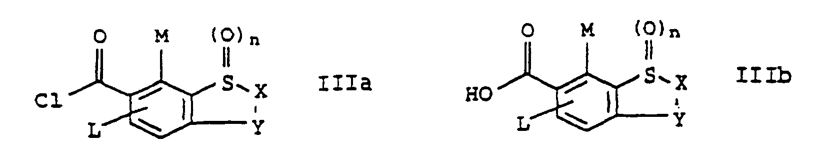 où R¹⁵, R¹⁶, L, M, X, n et Y ont la signification indiquée dans la revendication 1, et on réarrange le produit d'acylation en présence d'un catalyseur pour donner les composés I.

10. Produit herbicide, contenant au moins un dérivé de pyrazol-4-yl-benzoyle de formule I selon la revendication 1 et des additifs inertes classiques.

11. Procédé pour la lutte contre la croissance indésirable des plantes, **caractérisé par le fait qu'**on fait agir une quantité à efficacité herbicide d'un dérivé de pyrazol-4-yl-benzoyle de formule I selon la revendication 1 sur les plantes ou leur biotope.
